# EUROPEAN PATENT SPECIFICATION

(11) **EP 3 678 700 B1**
(45) Date of publication and mention of the grant of the patent: **19.06.2024**
(21) Application number: 18854741.8
(22) Date of filing: 31.08.2018
(51) Int. Cl.: A61K 39/395, A61K 38/03, A61K 47/22, A61K 47/18, A61P 35/00, C07K 16/28

(54) **COMPOUNDS FOR REDUCING THE VISCOSITY OF BIOLOGICAL FORMULATIONS**
VERBINDUNGEN ZUR VERRINGERUNG DER VISKOSITÄT VON BIOLOGISCHEN FORMULIERUNGEN
COMPOSÉS POUR LA RÉDUCTION DE LA VISCOSITÉ DE FORMULATIONS BIOLOGIQUES

(30) Priority: 05.09.2017 US 201762554134 P
(43) Date of publication of application: 15.07.2020
(73) Proprietor: Merck Sharp & Dohme LLC, Rahway, New Jersey 07065 (US)
(72) Inventor: CHU, Lin, Scotch Plains New Jersey 07076 (US); TOUSSAINT, Nathalie, Y., Rahway New Jersey 07065-0907 (US); XIAO, Dong, Kenilworth New Jersey 07033 (US); VACHAL, Petr, Kenilworth New Jersey 07033 (US); KASHI, Ramesh, S., Warren New Jersey 07059 (US); BAK, Annette, 43330 Partille (SE)
(74) Representative: Merck Sharp & Dohme LLC
(86) International application number: PCT/US2018/048995
(87) International publication number: WO 2019/050780

(56) References cited:
- EP-A2- 3 043 775
- EP-B1- 3 043 775
- US-A1- 2012 231 972
- US-B2- 8 933 075
- US-B2- 9 278 131
- DATABASE CAPLUS [Online] Chemical Abstracts; 1 January 1976 (1976-01-01), Pfeilsticker Konrad ET AL: "Studies on the behavior of ethylene oxide in food fumigation, part IX.", XP055786686, retrieved from STN Database accession no. 1976:163013 CAPLUS
- MANZINI B ET AL: "Polymer-supported syntheses of oxo-crown ethers and derivatives containing @a-amino-acid residues", REACTIVE AND FUNCTIONAL POLYMERS, ELSEVIER, AMSTERDAM, NL, vol. 68, no. 9, 1 September 2008 (2008-09-01), pages 1297-1306, XP023979605, ISSN: 1381-5148, DOI: 10.1016/J.REACTFUNCTPOLYM.2008.06.003 [retrieved on 2008-06-10]
- JING ZHANG ET AL: "Synthesis and characterization of heterotelechelic poly(ethylene glycol)s with amino acid at one end and hydroxyl group at another end", JOURNAL OF APPLIED POLYMER SCIENCE, vol. 110, no. 4, 15 November 2008 (2008-11-15), pages 2432-2439, XP055217315, ISSN: 0021-8995, DOI: 10.1002/app.27735
- WANG BING ET AL: "Amino acid endcapped poly(p-dioxanone): synthesis and crystallization", JOURNAL OF POLYMER RESEARCH, vol. 20, no. 4, 16 March 2013 (2013-03-16) , XP055786794, NL ISSN: 1022-9760, DOI: 10.1007/s10965-013-0116-6 Retrieved from the Internet: URL:http://link.springer.com/article/10.10 07/s10965-013-0116-6/fulltext.html>
- QING GUANGYAN ET AL: "Chiral Effect at Protein/Graphene Interface: A Bioinspired Perspective To Understand Amyloid Formation", JOURNAL OF THE AMERICAN CHEMICAL SOCIETY, vol. 136, no. 30, 18 July 2014 (2014-07-18), pages 10736-10742, XP055786800, US ISSN: 0002-7863, DOI: 10.1021/ja5049626 Retrieved from the Internet: URL:https://pubs.acs.org/doi/pdf/10.1021/j a5049626>
- HERMANS JOS ET AL: "Physicochemical Parameters Affecting the Electrospray Ionization Efficiency of Amino Acids after Acylation", ANALYTICAL CHEMISTRY, vol. 89, no. 17, 16 August 2017 (2017-08-16), pages 9159-9166, XP055786803, ISSN: 0003-2700, DOI: 10.1021/acs.analchem.7b01899 Retrieved from the Internet: URL:http://pubs.acs.org/doi/pdf/10.1021/ac s.analchem.7b01899>
- ZHENG GUO ET AL: "Structure-Activity Relationship for Hydrophobic Salts as Viscosity-Lowering Excipients for Concentrated Solutions of Monoclonal Antibodies", PHARMACEUTICAL RESEARCH, KLUWER ACADEMIC PUBLISHERS-PLENUM PUBLISHERS, NL, vol. 29, no. 11, 13 June 2012 (2012-06-13) , pages 3102-3109, XP035126580, ISSN: 1573-904X, DOI: 10.1007/S11095-012-0802-9
- DATABASE PURCHEM substance 13 January 2016 (2016-01-13), XP055581628, retrieved from NCBI Database accession no. SID281990137

## Description

### FIELD OF THE INVENTION

This invention relates to novel PEGylated amino acids of Formula (I) and uses thereof. The compounds are useful as excipients to reduce the viscosity of formulations comprising high concentrations of biological therapeutics.

### CROSS-REFERENCE TO RELATED APPLICATIONS

This application claims the benefit of U.S. Provisional Application No. 62/554,134, filed on September 5, 2017.

### REFERENCE TO SEQUENCE LISTING SUBMITTED ELECTRONICALLY

The sequence listing of the present application is submitted electronically via EFS-Web as an ASCII formatted sequence listing with a file name "24494WOPCT-SEQLIST-07AUG2018.TXT", creation date of August 7, 2018, and a size of 33.1 Kb. This sequence listing submitted via EFS-Web is part of the specification.

### BACKGROUND OF THE INVENTION

Advances in biotechnology have allowed the launch of numerous therapeutic recombinant protein and monoclonal antibody products. Commercially viable biological products often require the development of liquid formulations containing a high concentration (50 mg/mL or more) of active biological ingredient, especially when subcutaneous administration is the preferred delivery route. However, formulation of these products remains a challenge for several reasons, including providing sufficient stability, overcoming potential aggregate formation, and overcoming the high viscosity associated with high concentrations of protein or antibody. *See* Shire et al., Challenges in the Development of High Protein Concentration Formulations. J. Pharm. Sci. 93(6): 1390-1402 (2004). Highly viscous liquid formulations are particularly problematic due to difficulties in manufacturing and delivery.

Several methods of controlling formulation viscosity through modification of formulation components have been proposed. For review, see Jezek et al. Viscosity of concentrated therapeutic protein compositions, Advanced Drug Delivery Reviews, 63 :1101-1117 (2011); Tomar et al., Molecular basis of high viscosity in concentrated antibody solutions; Strategies for high concentration drug product development. MABS 8(2): 216-228 (2116). For example, EP 2 116 265 proposes modifying the pH of the formulation outside of the physiological pH range as a means to reduce viscosity or including >100 mM salt in the formulation. Liu et al. (US 2007/0053900) propose the use of histidine and/or arginine-HCl, in combination with other formulation components, to control the viscosity of formulations comprising a high concentration of antibody. Kaisheva et al. (US 2003/0138417) disclose the use of a tonicity modifier selected from various salts or amino acids, especially proline, in combination with a succinate or histidine buffer and polysorbate in high-concentration antibody formulations. Bowen et al. (WO 2011/139718) propose the addition of specific compounds to biological formulations to reduce the viscosity, including certain charged amino acids. Soane et al. (US 9,605,051) disclose caffeine as a viscosity-reducing excipient for use with high concentration therapeutic antibody formulations. Also US 9278131 B2 and Zheng Guo et al. in PHARMACEUTICAL RESEARCH, pages 3102-3109, vol. 29(11) 2012 disclose viscosity-lowering excipients for antibody solutions. Additional methods of controlling solution viscosity of biological formulations through formulation components are disclosed in Larson et al., WO 2015/038818, Dauty et al., and US Appln. Publication No. 2014/0044708.

Despite the proposed formulation components discussed above for reducing viscosity of biological formulations, there exists a need for formulation excipients and compositions that are capable of controlling viscosity of solutions comprising a high concentration of protein or antibody.

### SUMMARY OF THE INVENTION

The present invention is directed to PEGylated amino acid compounds of Formula I, which are useful as excipients in pharmaceutical formulations comprising active biological ingredients. Compounds with similar structural elements are disclosed in the prior art in chemical Abstracts RN 58970-48-2 "N-[2-(2-hydroxyethoxy)ethyl]-L-Tyrosine"; in MANZINI et al. "Polymer supported syntheses of oxo-crown ethers and derivatives containing amino-acid residues". REACTIVE AND FUNCTIONAL POLYMERS, 68(9) 2008; in JING ZHANG et al. "Synthesis and characterization of heterotelechelic polyethylene glycols with amino acid at one end and hydroxyl group at another end", J. APPLIED POLYMER SCIENCE, 110(4) 2008; in WANG BING et al. "Amino acid endcapped poly(p-dioxanone): synthesis and crystallization", J. POLYMER RESEARCH, 20(4) 2013; in QING GUANGYAN et al. "Chiral Effect at Protein/Graphene Interface: A Bioinspired Perspective To Understand Amyloid Formation", J. AMERICAN CHEMICAL SOCIETY, 136(30) 2014; in HERMANS JOS et al. "Physicochemical Parameters Affecting the Electrospray Ionization Efficiency of Amino Acids after Acylation", ANALYTICAL CHEMISTRY, 89(17) 2017. More particularly, the present invention includes compounds of Formula I: 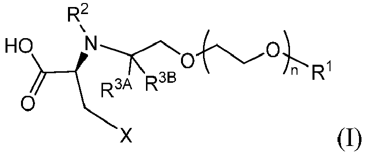 or a pharmaceutically acceptable salt thereof, wherein:
X is 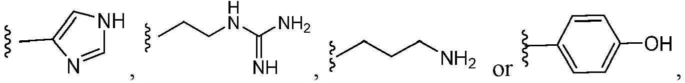
R¹ is H or methyl;
R² is 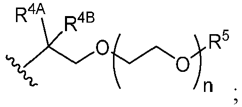
R^{3A} and R^{3B} are each H or together form oxo;
R^{4A} and R^{4B} are each H or together form oxo;
R⁵ is H or methyl; and
each occurrence of n is independently 1 to 5;
and wherein 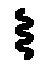 indicates the point of attachment to the rest of the compound.

Compounds of Formula I are useful for lowering the viscosity of pharmaceutical formulations comprising active biological ingredients; i.e. antibodies or antigen binding fragments thereof or therapeutic proteins. Accordingly, in certain embodiments, the present invention provides compositions comprising a compound of Formula I, or a pharmaceutically acceptable salt thereof, an active biological ingredient (ABI), and a pharmaceutically acceptable carrier, In specific embodiments, the pharmaceutical compositions of the invention optionally comprise one or more additional excipients. In some embodiments, the ABI is present in the composition in a high concentration, e.g. from about 50 mg/mL to about 250 mg/mL. In specific embodiments, the ABI is an anti-human PD-1 antibody or antigen binding fragment thereof that specifically binds to human PD-1. The invention further includes methods for treating a pathological disease or condition by administration of a compound of Formula I, or a pharmaceutically acceptable salt thereof, to a patient in need thereof, or by administration of a composition comprising a compound of Formula I or its salt and a pharmaceutically acceptable carrier.

Embodiments, sub-embodiments, aspects and features of the present invention are either further described herein or will be apparent from the ensuing description, examples and appended claims.

### DETAILED DESCRIPTION OF THE INVENTION

This invention relates to novel PEGylated amino acids of Formula (I) and uses thereof. The compounds are useful as excipients to reduce the viscosity of formulations comprising high concentrations of biological therapeutics, as discussed more fully, *infra.*

### I. Definitions and Abbreviations

As used throughout the specification and appended claims, the following abbreviations apply:
- ABI: active biological ingredient
- API: active pharmaceutical ingredient
- (Boc)₂O: di-*tert*-butyl dicarbonate
- CDR: complementarity determining region in the immunoglobulin variable regions, defined using the Kabat numbering system, unless otherwise indicated
- CELITE: diatomaceous earth
- CHO: Chinese hamster ovary
- CI: confidence interval
- CTLA4: cytotoxic T lymphocyte associated antigen 4
- DCM: dichloromethane
- DIEA: N,N-diisopropylethylamine
- DMF: N,N-dimethylformamide
- D₂O: deuterated water
- DTPA: diethylenetriaminepentaacetic acid
- EC50: concentration resulting in 50% efficacy or binding
- ELISA: enzyme-linked immunosorbent assay
- EtOAc: ethyl acetate
- EtOH: ethanol
- FFPE: formalin-fixed, paraffin-embedded
- FR: framework region
- HATU: (1-[Bis(dimethylamino)methylene]-1H-1,2,3-triazolo[4,5-b]pyridinium 3-oxid hexafluorophosphate)
- HRP: horseradish peroxidase
- HNSCC: head and neck squamous cell carcinoma
- IC50: concentration resulting in 50% inhibition
- IgG: immunoglobulin G
- IHC: immunohistochemistry or immunohistochemical
- LC-MS: liquid chromatography-mass spectrometry (also abbreviated LCMS)
- mAb: monoclonal antibody
- Me: methyl
- MeOH: methanol
- MES: 2-(N-morpholino)ethanesulfonic acid
- NCBI: National Center for Biotechnology Information
- NMR: nuclear magnetic resonance
- NSCLC: non-small cell lung cancer
- PCR: polymerase chain reaction
- PD-1: programmed death 1 (a.k.a. programmed cell death-1 and programmed death receptor 1)
- PD-L1: programmed cell death 1 ligand 1
- PD-L2: programmed cell death 1 ligand 2
- PEG: polyethylene glycol
- PS80: polysorbate 80
- TEA: triethylamine (also abbreviated Et₃N)
- TFA: trifluoroacetic acid
- TLC: thin layer chromatography
- TNBC: triple negative breast cancer
- V_{H}: immunoglobulin heavy chain variable region
- VK: immunoglobulin kappa light chain variable region
- V_{L}: immunoglobulin light chain variable region
- v/v: volume per volume
- WFI: water for injection
- w/v: weight per volume

So that the invention may be more readily understood, certain technical and scientific terms are specifically defined below. Unless specifically defined elsewhere in this document, all other technical and scientific terms used herein have the meaning commonly understood by one of ordinary skill in the art to which this invention belongs.

As used throughout the specification and in the appended claims, the singular forms "a," "an," and "the" include the plural reference unless the context clearly dictates otherwise.

Reference to "or" indicates either or both possibilities unless the context clearly dictates one of the indicated possibilities. In some cases, "and/or" was employed to highlight either or both possibilities.

An "active biological ingredient" (or "ABI"), as used herein, refers to an active ingredient of a pharmaceutical formulation that is either an antibody or antigen-binding fragment thereof, or a therapeutic protein or peptide. An ABI is the component of a biological pharmaceutical formulation that is useful for inducing a desired positive therapeutic effect when administered to a patient, e.g. treating or preventing a disease or condition, which may include halting or delaying the progression of a disease or pathological condition, reducing the severity or duration of the clinical symptoms of the disease, prolonging the survival of a patient relative to the expected survival in a similar untreated patient, and inducing complete or partial remission of the disease or condition. An "active pharmaceutical ingredient" (or "API") refers to any active ingredient in a pharmaceutical formulation that is useful for treating or preventing a pathological disease or condition, including but not limited to, antibodies and antigen-binding fragments thereof, proteins, and small molecules.

"Antibody B," as used herein, is a high affinity, humanized, IgG1anti-IL23p19 monoclonal antibody.

"Treat" or "treating" means to administer a composition of the invention to a patient in order to induce a positive therapeutic effect. The terms do not necessarily indicate a total elimination of all disease or disorder symptoms. "Treating" a cancer or immune condition refers to administration of a composition of the invention to a patient having an immune condition or cancerous condition, or diagnosed with or predisposed to a cancer or a pathogenic infection (e.g. viral, bacterial, fungal), to achieve at least one positive therapeutic effect, such as for example, reduced number of cancer cells, reduced tumor size, reduced rate of cancer cell infiltration into peripheral organs, or reduced rate of tumor metastasis or tumor growth. "Treatment" may include one or more of the following: inducing/increasing an antitumor immune response, stimulating an immune response to a pathogen, toxin, and/or self-antigen, stimulating an immune response to a viral infection, decreasing the number of one or more tumor markers, inhibiting the growth or survival of tumor cells, eliminating or reducing the size of one or more cancerous lesions or tumors, decreasing the level of one or more tumor markers, ameliorating, reducing the severity or duration of the cancer, prolonging the survival of a patient relative to the expected survival in a similar untreated patient.

"Immune condition" or "immune disorder" encompasses, e.g., pathological inflammation, an inflammatory disorder, and an autoimmune disorder or disease. "Immune condition" also refers to infections, persistent infections, and proliferative conditions, such as cancer, tumors, and angiogenesis, including infections, tumors, and cancers that resist eradication by the immune system. "Cancerous condition" includes, e.g., cancer, cancer cells, tumors, angiogenesis, and precancerous conditions such as dysplasia.

Positive therapeutic effects in cancer can be measured in a number of ways (See, W. A. Weber, J. Nucl. Med. 50:1S-10S (2009)). For example, with respect to tumor growth inhibition, according to NCI standards, a T/C ≦42% is the minimum level of anti-tumor activity. A T/C < 10% is considered a high anti-tumor activity level, with T/C (%) = Median tumor volume of the treated/Median tumor volume of the control × 100. In some embodiments, the treatment achieved by administration of a formulation of the invention is any of progression free survival (PFS), disease free survival (DFS) or overall survival (OS). PFS, also referred to as "Time to Tumor Progression" indicates the length of time during and after treatment that the cancer does not grow, and includes the amount of time patients have experienced a complete response or a partial response, as well as the amount of time patients have experienced stable disease. DFS refers to the length of time during and after treatment that the patient remains free of disease. OS refers to a prolongation in life expectancy as compared to naive or untreated individuals or patients. While an embodiment of the formulations, treatment methods, and uses of the present invention may not be effective in achieving a positive therapeutic effect in every patient, it should do so in a statistically significant number of subjects as determined by any statistical test known in the art such as the Student's t-test, the chi²-test, the U-test according to Mann and Whitney, the Kruskal-Wallis test (H-test), Jonckheere-Terpstra-test and the Wilcoxon-test.

The term "patient" (alternatively referred to as "subject" or "individual" herein) refers to a mammal (e.g., rat, mouse, dog, cat, rabbit) capable of being treated with the formulations of the invention, most preferably a human. The term "patient" may also include non-human animals including livestock animals and domestic animals including, but not limited to, cattle, horses, sheep, swine, goats, rabbits, cats, dogs, and other mammals in need of treatment. In some embodiments, the patient is an adult patient. In other embodiments, the patient is a pediatric patient. A patient "in need of treatment" is an individual diagnosed with, suspected of having, or predisposed to a disease or disorder in which a composition of the invention is intended to treat, or a patient for whom prevention of a disorder is desired.

The term "antibody" refers to any form of antibody that exhibits the desired biological activity. Thus, it is used in the broadest sense and specifically covers, but is not limited to, monoclonal antibodies (including full length monoclonal antibodies), polyclonal antibodies, humanized, fully human antibodies, and chimeric antibodies. "Parental antibodies" are antibodies obtained by exposure of an immune system to an antigen prior to modification of the antibodies for an intended use, such as humanization of an antibody for use as a human therapeutic antibody.

In general, the basic antibody structural unit comprises a tetramer. Each tetramer includes two identical pairs of polypeptide chains, each pair having one "light" (about 25 kDa) and one "heavy" chain (about 50-70 kDa). The amino-terminal portion of each chain includes a variable region of about 100 to 110 or more amino acids primarily responsible for antigen recognition. The variable regions of each light/heavy chain pair form the antibody binding site. Thus, in general, an intact antibody has two binding sites. The carboxy-terminal portion of the heavy chain may define a constant region primarily responsible for effector function. Typically, human light chains are classified as kappa and lambda light chains. Furthermore, human heavy chains are typically classified as mu, delta, gamma, alpha, or epsilon, and define the antibody's isotype as IgM, IgD, IgG, IgA, and IgE, respectively. Within light and heavy chains, the variable and constant regions are joined by a "J" region of about 12 or more amino acids, with the heavy chain also including a "D" region of about 10 more amino acids. See generally, Fundamental Immunology Ch. 7 (Paul, W., ed., 2nd ed. Raven Press, N.Y. (1989).

Typically, the variable domains of both the heavy and light chains comprise three hypervariable regions, also called complementarity determining regions (CDRs), which are located within relatively conserved framework regions (FR). The CDRs are usually aligned by the framework regions, enabling binding to a specific epitope. In general, from N-terminal to C-terminal, both light and heavy chains variable domains comprise FR1, CDR1, FR2 , CDR2, FR3, CDR3 and FR4. The assignment of amino acids to each domain is, generally, in accordance with the definitions of Sequences of Proteins of Immunological Interest, Kabat, et al.; National Institutes of Health, Bethesda, Md. ; 5th ed.; NIH Publ. No. 91-3242 (1991); Kabat (1978) Adv. Prot. Chem. 32:1-75; Kabat, et al., (1977) J. Biol. Chem. 252:6609-6616; Chothia, et al., (1987) J Mol. Biol. 196:901-917 or Chothia, et al., (1989) Nature 342:878-883.

An antibody that "specifically binds to" a specified target protein is an antibody that exhibits preferential binding to that target as compared to other proteins, but this specificity does not require absolute binding specificity. An antibody is considered "specific" for its intended target if its binding is determinative of the presence of the target protein in a sample, e.g. without producing undesired results such as false positives. Antibodies, or binding fragments thereof, useful in the present invention will bind to the target protein with an affinity that is at least two fold greater, preferably at least ten times greater, more preferably at least 20-times greater, and most preferably at least 100-times greater than the affinity with non-target proteins. As used herein, an antibody is said to bind specifically to a polypeptide comprising a given amino acid sequence, e.g. the amino acid sequence of a mature human PD-1 molecule, if it binds to polypeptides comprising that sequence but does not bind to proteins lacking that sequence.

"Chimeric antibody" refers to an antibody in which a portion of the heavy and/or light chain is identical with or homologous to corresponding sequences in an antibody derived from a particular species (e.g., human) or belonging to a particular antibody class or subclass, while the remainder of the chain(s) is identical with or homologous to corresponding sequences in an antibody derived from another species (e.g., mouse) or belonging to another antibody class or subclass, as well as fragments of such antibodies, so long as they exhibit the desired biological activity.

The term "pharmaceutically effective amount" or "therapeutically effective amount" means an amount whereby sufficient therapeutic composition or formulation is introduced to a patient to treat a disease or condition. One skilled in the art recognizes that this level may vary according the patient's characteristics such as age, weight, etc. The term "effective amount," when used with a compound of the invention (i.e. a compound of Formula I), means the amount of compound sufficient to decrease viscosity of a formulation relative to the same formulation without the compound. An "effective amount" of an active pharmaceutical ingredient or active biological ingredient means an amount sufficient to elicit the response being sought in a cell, tissue, system, animal or human. In one embodiment, the effective amount is a "therapeutically effective amount" for the alleviation of the symptoms of the disease or condition being treated. In another embodiment, the effective amount is a "prophylactically effective amount" for prophylaxis of the symptoms of the disease or condition being prevented. When the active compound (*i.e.,* active ingredient) is administered as the salt, references to the amount of active ingredient are to the free acid or free base form of the compound. An "effective amount," when used to modify a compound of the invention, i.e. a compound of Formula I, means that sufficient amount of the compound is present in a formulation to perform the function as desired, e.g. lowering or maintaining the viscosity of a solution within an acceptable range.

The term "about", when modifying the quantity (e.g., mM, or M) of a substance or composition, the percentage (v/v or w/v) of a formulation component, the pH of a solution/formulation, or the value of a parameter characterizing a step in a method, or the like refers to variation in the numerical quantity that can occur, for example, through typical measuring, handling and sampling procedures involved in the preparation, characterization and/or use of the substance or composition; through inadvertent error in these procedures; through differences in the manufacture, source, or purity of the ingredients employed to make or use the compositions or carry out the procedures; and the like. In certain embodiments, "about" can mean a variation of ± 0.1, 0.2, 0.3, 0.4, 0.5, 1.0, 2.0, 3.0, 4.0, or 5.0 of the appropriate unit. In certain embodiments, "about" can mean a variation of ± 0.1%, 0.5%, 1%, 2%, 3%, 4%, 5%, or 10%.

The terms "cancer", "cancerous", or "malignant" refer to or describe the physiological condition in mammals that is typically characterized by unregulated cell growth. Examples of cancer include but are not limited to, carcinoma, lymphoma, leukemia, blastoma, and sarcoma. More particular examples of such cancers include squamous cell carcinoma, myeloma, small-cell lung cancer, non-small cell lung cancer, glioma, Hodgkin's lymphoma, non-Hodgkin's lymphoma, gastrointestinal (tract) cancer, renal cancer, ovarian cancer, liver cancer, lymphoblastic leukemia, lymphocytic leukemia, colorectal cancer, endometrial cancer, kidney cancer, prostate cancer, thyroid cancer, melanoma, chondrosarcoma, neuroblastoma, pancreatic cancer, glioblastoma multiforme, cervical cancer, brain cancer, stomach cancer, bladder cancer, hepatoma, breast cancer, colon carcinoma, and head and neck cancer.

"Chothia" means an antibody numbering system described in Al-Lazikani et al., JMB 273:927-948 (1997).

"Kabat" as used herein means an immunoglobulin alignment and numbering system pioneered by Elvin A. Kabat ((1991) Sequences of Proteins of Immunological Interest, 5th Ed. Public Health Service, National Institutes of Health, Bethesda, Md.).

The terms "PD-1 binding fragment," "antigen binding fragment thereof," "binding fragment thereof' or "fragment thereof' encompass a fragment or a derivative of an antibody that still substantially retains its biological activity of binding to antigen (human PD-1) and inhibiting its activity (e.g., blocking the binding of PD-1 to PDL1 and PDL2). Therefore, the term "antibody fragment" or PD-1 binding fragment refers to a portion of a full length antibody, generally the antigen binding or variable region thereof. Examples of antibody fragments include Fab, Fab', F(ab')₂, and Fv fragments. Typically, a binding fragment or derivative retains at least 10% of its PD-1 inhibitory activity. In some embodiments, a binding fragment or derivative retains at least 25%, 50%, 60%, 70%, 80%, 90%, 95%, 99% or 100% (or more) of its PD-1 inhibitory activity, although any binding fragment with sufficient affinity to exert the desired biological effect will be useful. In some embodiments, an antigen binding fragment binds to its antigen with an affinity that is at least two fold greater, preferably at least ten times greater, more preferably at least 20-times greater, and most preferably at least 100-times greater than the affinity with unrelated antigens. In one embodiment the antibody has an affinity that is greater than about 10⁹ liters/mol, as determined, e.g., by Scatchard analysis. Munsen et al. (1980) Analyt. Biochem. 107:220-239. It is also intended that a PD-1 binding fragment can include variants having conservative amino acid substitutions that do not substantially alter its biologic activity.

"Human antibody" refers to an antibody that comprises human immunoglobulin protein sequences only. A human antibody may contain murine carbohydrate chains if produced in a mouse, in a mouse cell, or in a hybridoma derived from a mouse cell. Similarly, "mouse antibody" or "rat antibody" refer to an antibody that comprises only mouse or rat immunoglobulin sequences, respectively.

"Humanized antibody" refers to forms of antibodies that contain sequences from non-human (*e.g*., murine) antibodies as well as human antibodies. Such antibodies contain minimal sequence derived from non-human immunoglobulin. In general, the humanized antibody will comprise substantially all of at least one, and typically two, variable domains, in which all or substantially all of the hypervariable loops correspond to those of a non-human immunoglobulin and all or substantially all of the FR regions are those of a human immunoglobulin sequence. The humanized antibody optionally also will comprise at least a portion of an immunoglobulin constant region (Fc), typically that of a human immunoglobulin. The humanized forms of rodent antibodies will generally comprise the same CDR sequences of the parental rodent antibodies, although certain amino acid substitutions may be included to increase affinity, increase stability of the humanized antibody, or for other reasons.

Antibodies useful in the compositions of the present invention also include antibodies with modified (or blocked) Fc regions to provide altered effector functions. *See, e.g.,* U.S. Pat. No. 5,624,821; WO2003/086310; WO2005/120571; WO2006/0057702; Presta (2006) Adv. Drug Delivery Rev. 58:640-656. Such modification can be used to enhance or suppress various reactions of the immune system, with possible beneficial effects in diagnosis and therapy. Alterations of the Fc region include amino acid changes (substitutions, deletions and insertions), glycosylation or deglycosylation, and adding multiple Fc. Changes to the Fc can also alter the half-life of antibodies in therapeutic antibodies, and a longer half-life would result in less frequent dosing, with the concomitant increased convenience and decreased use of material. *See* Presta (2005) J. *Allergy Clin. Immunol.* 116:731 at 734-35.

"Fully human antibody" refers to an antibody that comprises human immunoglobulin protein sequences only. A fully human antibody may contain murine carbohydrate chains if produced in a mouse, in a mouse cell, or in a hybridoma derived from a mouse cell. Similarly, "mouse antibody" refers to an antibody which comprises mouse immunoglobulin sequences only. A fully human antibody may be generated in a human being, in a transgenic animal having human immunoglobulin germline sequences, by phage display or other molecular biological methods.

"Hypervariable region" refers to the amino acid residues of an antibody that are responsible for antigen-binding. The hypervariable region comprises amino acid residues from a "complementarity determining region" or "CDR" (*e.g*. residues 24-34 (CDRL1), 50-56 (CDRL2) and 89-97 (CDRL3) in the light chain variable domain and residues 31-35 (CDRH1), 50-65 (CDRH2) and 95-102 (CDRH3) in the heavy chain variable domain as measured by the Kabat numbering system (Kabat *et al.* (1991) Sequences of Proteins of Immunological Interest, 5th Ed. Public Health Service, National Institutes of Health, Bethesda, Md.) and/or those residues from a "hypervariable loop" (*i.e.* residues 26-32 (L1), 50-52 (L2) and 91-96 (L3) in the light chain variable domain and 26-32 (H1), 53-55 (H2) and 96-101 (H3) in the heavy chain variable domain (Chothia and Lesk (1987) J. Mol. Biol. 196: 901-917). As used herein, the term "framework" or "FR" residues refers to those variable domain residues other than the hypervariable region residues defined herein as CDR residues. CDR and FR residues are determined according to the standard sequence definition of Kabat. Kabat et al. (1987) Sequences of Proteins of Immunological Interest, National Institutes of Health, Bethesda Md.

"Conservatively modified variants" or "conservative substitution" refers to substitutions of amino acids are known to those of skill in this art and may be made generally without altering the biological activity of the resulting molecule, even in essential regions of the polypeptide. Such exemplary substitutions are preferably made in accordance with those set forth in Table 1 as follows:

**Table 1. Exemplary Conservative Amino Acid Substitutions**

| Original residue | Conservative substitution |
|---|---|
| Ala (A) | Gly; Ser |
| Arg (R) | Lys, His |
| Asn (N) | Gln; His |
| Asp (D) | Glu; Asn |
| Cys (C) | Ser; Ala |
| Gln (Q) | Asn |
| Glu (E) | Asp; Gln |
| Gly (G) | Ala |
| His (H) | Asn; Gln |
| Ile (I) | Leu; Val |
| Leu (L) | Ile; Val |
| Lys (K) | Arg; His |
| Met (M) | Leu; Ile; Tyr |
| Phe (F) | Tyr; Met; Leu |
| Pro (P) | Ala |
| Ser (S) | Thr |
| Thr (T) | Ser |
| Trp (W) | Tyr; Phe |
| Tyr (Y) | Trp; Phe |
| Val (V) | Ile; Leu |

In addition, those of skill in this art recognize that, in general, single amino acid substitutions in non-essential regions of a polypeptide do not substantially alter biological activity. *See, e.g.,* Watson et al. (1987) Molecular Biology of the Gene, The Benjamin/Cummings Pub. Co., p. 224 (4th Edition).

The phrase "consists essentially of," or variations such as "consist essentially of" or "consisting essentially of," as used throughout the specification and claims, indicate the inclusion of any recited elements or group of elements, and the optional inclusion of other elements, of similar or different nature than the recited elements, that do not materially change the basic or novel properties of the specified dosage regimen, method, or composition. As a nonlimiting example, a binding compound that consists essentially of a recited amino acid sequence may also include one or more amino acids, including substitutions of one or more amino acid residues, which do not materially affect the properties of the binding compound.

"Comprising" or variations such as "comprise", "comprises" or "comprised of" are used throughout the specification and claims in an inclusive sense, i.e., to specify the presence of the stated features but not to preclude the presence or addition of further features that may materially enhance the operation or utility of any of the embodiments of the invention, unless the context requires otherwise due to express language or necessary implication.

"Isolated antibody" and "isolated antibody fragment" refers to the purification status and in such context means the named molecule is substantially free of other biological molecules such as nucleic acids, proteins, lipids, carbohydrates, or other material such as cellular debris and growth media. Generally, the term "isolated" is not intended to refer to a complete absence of such material or to an absence of water, buffers, or salts, unless they are present in amounts that substantially interfere with experimental or therapeutic use of the binding compound as described herein.

"Monoclonal antibody" or "mAb" or "Mab", as used herein, refers to a population of substantially homogeneous antibodies, *i.e.,* the antibody molecules comprising the population are identical in amino acid sequence except for possible naturally occurring mutations that may be present in minor amounts. In contrast, conventional (polyclonal) antibody preparations typically include a multitude of different antibodies having different amino acid sequences in their variable domains, particularly their CDRs, which are often specific for different epitopes. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al. (1975) Nature 256: 495, or may be made by recombinant DNA methods (*see, e.g.,* U.S. Pat. No. 4,816,567). The "monoclonal antibodies" may also be isolated from phage antibody libraries using the techniques described in Clackson et al. (1991) Nature 352: 624-628 and Marks et al. (1991) J. Mol. Biol. 222: 581-597, for example. *See also* Presta (2005) J. Allergy Clin. Immunol. 116:731.

"Tumor" as it applies to a subject diagnosed with, or suspected of having, a cancer refers to a malignant or potentially malignant neoplasm or tissue mass of any size, and includes primary tumors and secondary neoplasms. A solid tumor is an abnormal growth or mass of tissue that usually does not contain cysts or liquid areas. Different types of solid tumors are named for the type of cells that form them. Examples of solid tumors are sarcomas, carcinomas, and lymphomas. Leukemias (cancers of the blood) generally do not form solid tumors (National Cancer Institute, Dictionary of Cancer Terms).

"Variable regions" or "V region" as used herein means the segment of IgG chains which is variable in sequence between different antibodies. It extends to Kabat residue 109 in the light chain and 113 in the heavy chain.

The term "buffer" encompasses those agents which maintain the solution pH of the formulations of the invention in an acceptable range, or, for Lyophilized formulations of the invention, provide an acceptable solution pH prior to lyophilization.

The term "pharmaceutical formulation" refers to preparations which are in such form as to permit the active ingredients to be effective, and which contains no additional components which are toxic to the subjects to which the formulation would be administered.

"Pharmaceutically acceptable" refers to excipients (vehicles, additives) and compositions that can reasonably be administered to a subject to provide an effective dose of the active ingredient employed and that are "generally regarded as safe" *e.g*., that are physiologically tolerable and do not typically produce an allergic or similar untoward reaction, such as gastric upset and the like, when administered to a human. In another embodiment, this term refers to molecular entities and compositions approved by a regulatory agency of the federal or a state government or listed in the U.S. Pharmacopeia or another generally recognized pharmacopeia for use in animals, and more particularly in humans.

A "stable formulation" is one in which the API therein essentially retains its physical stability and/or chemical stability and/or biological activity upon storage. Various analytical techniques for measuring protein stability are available in the art and are reviewed in Peptide and Protein Drug Delivery, 247-301, Vincent Lee Ed., Marcel Dekker, Inc., New York, N.Y., Pubs. (1991) and Jones, A. Adv. Drug Delivery Rev. 10:29-90 (1993). Stability can be measured at a selected temperature for a selected time period.

A "stable" pharmaceutical antibody formulation is a pharmaceutical antibody formulation with no significant changes observed at a refrigerated temperature (2-8°C) for at least 3 months, preferably 6 months, and more preferably 1 year, and even more preferably up through 2 years. Additionally, a "stable" liquid formulation includes one that exhibits desired features at temperatures including at 25°C and 40°C for periods including 1 month, 3 months, 6 months, 12 months, and/or 24 months. Typical acceptable criteria for stability are as follows. Typically, no more than about 10%, preferably about 5%, of antibody monomer is degraded as measured by SEC-HPLC. The pharmaceutical antibody formulation is colorless, or clear to slightly opalescent by visual analysis. The concentration, pH and osmolality of the formulation have no more than +/-10% change. Potency is typically within 50-150 % of the reference. Typically, no more than about 10%, preferably about 5% of clipping is observed. Typically, no more than about 10%, preferably about 5% of aggregation is formed.

An antibody "retains its physical stability" in a pharmaceutical formulation if it shows no significant increase of aggregation, precipitation and/or denaturation upon visual examination of color and/or clarity, or as measured by UV light scattering, size exclusion chromatography (SEC) and dynamic light scattering. The changes of protein conformation can be evaluated by fluorescence spectroscopy, which determines the protein tertiary structure, and by FTIR spectroscopy, which determines the protein secondary structure.

An antibody "retains its chemical stability" in a pharmaceutical formulation, if it shows no significant chemical alteration. Chemical stability can be assessed by detecting and quantifying chemically altered forms of the protein. Degradation processes that often alter the protein chemical structure include hydrolysis or clipping (evaluated by methods such as size exclusion chromatography and SDS-PAGE), oxidation (evaluated by methods such as by peptide mapping in conjunction with mass spectroscopy or MALDI/TOF/MS), deamidation (evaluated by methods such as ion-exchange chromatography, capillary isoelectric focusing, peptide mapping, isoaspartic acid measurement), and isomerization (evaluated by measuring the isoaspartic acid content, peptide mapping, etc.).

An antibody "retains its biological activity" in a pharmaceutical formulation, if the biological activity of the antibody at a given time is within a predetermined range of the biological activity exhibited at the time the pharmaceutical formulation was prepared. The biological activity of an antibody can be determined, for example, by an antigen binding assay.

One embodiment of the present invention is a compound of Formula I, or a pharmaceutically acceptable salt thereof, as originally defined or as defined in any of the foregoing embodiments, sub-embodiments, aspects, classes or sub-classes, wherein the compound or its salt is in a substantially pure form. As used herein "substantially pure" means suitably at least about 60 wt.%, typically at least about 70 wt.%, preferably at least about 80 wt.%, more preferably at least about 90 wt.% (*e.g*., from about 90 wt.% to about 99 wt.%), even more preferably at least about 95 wt.% (*e.g*., from about 95 wt.% to about 99 wt.%, or from about 98 wt.% to 100 wt.%), and most preferably at least about 99 wt.% (*e.g*., 100 wt.%) of a product containing a compound of Formula I, or its salt (*e.g*., the product isolated from a reaction mixture affording the compound or salt) consists of the compound or salt. The level of purity of the compounds and salts can be determined using a standard method of analysis such as thin layer chromatography, gel electrophoresis, high performance liquid chromatography, and/or mass spectrometry. If more than one method of analysis is employed and the methods provide experimentally significant differences in the level of purity determined, then the method providing the highest level of purity governs. A compound or salt of 100% purity is one which is free of detectable impurities as determined by a standard method of analysis.

With respect to a compound of the invention which has one or more asymmetric centers and can occur as mixtures of stereoisomers, a substantially pure compound can be either a substantially pure mixture of the stereoisomers or a substantially pure individual diastereomer or enantiomer unless expressly depicted otherwise. The present invention encompasses all stereoisomeric forms of the compounds of Formula I. Unless a specific stereochemistry is indicated, the present invention is meant to comprehend all such isomeric forms of these compounds. Centers of asymmetry that are present in the compounds of Formula I can all independently of one another have (R) configuration or (S) configuration. When bonds to the chiral carbon are depicted as straight lines in the structural Formulas of the invention, it is understood that both the (R) and (S) configurations of the chiral carbon, and hence both enantiomers and mixtures thereof, are embraced within the Formula. Similarly, when a compound name is recited without a chiral designation for a chiral carbon, it is understood that both the (R) and (S) configurations of the chiral carbon, and hence individual enantiomers, diastereomers and mixtures thereof, are embraced by the name. The production of specific stereoisomers or mixtures thereof may be identified in the Examples where such stereoisomers or mixtures were obtained, but this in no way limits the inclusion of all stereoisomers and mixtures thereof from being within the scope of this invention.

The invention includes all possible enantiomers and diastereomers and mixtures of two or more stereoisomers, for example mixtures of enantiomers and/or diastereomers, in all ratios. Thus, enantiomers are a subject of the invention in enantiomerically pure form, both as levorotatory and as dextrorotatory antipodes, in the form of racemates and in the form of mixtures of the two enantiomers in all ratios. In the case of a cis/trans isomerism the invention includes the cis form and the trans form, as well as mixtures of these forms in all ratios. The preparation of individual stereoisomers can be carried out, if desired, by separation of a mixture by customary methods, for example by chromatography or crystallization, by the use of stereochemically uniform starting materials for the synthesis or by stereoselective synthesis. Optionally a derivatization can be carried out before a separation of stereoisomers. The separation of a mixture of stereoisomers can be carried out at an intermediate step during the synthesis of a compound of Formula I or it can be done on a final racemic product. Absolute stereochemistry may be determined by X-ray crystallography of crystalline products or crystalline intermediates which are derivatized, if necessary, with a reagent containing a stereogenic center of known configuration. Unless a particular isomer, salt, solvate (including hydrates) or solvated salt of such racemate, enantiomer, or diastereomer is indicated, the present invention includes all such isomers, as well as salts, solvates (including hydrates) and solvated salts of such racemates, enantiomers, diastereomers and mixtures thereof.

"Oxo" means an oxygen atom connected to another atom by a double bond and is can be represented "=O".

When any variable (*e.g.,* n) occurs more than one time in any constituent or in Formula I, its definition on each occurrence is independent of its definition at every other occurrence. Also, combinations of substituents and/or variables are permissible only if such combinations result in stable compounds.

A wavy line 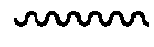 , as used herein, indicates a point of attachment to the rest of the compound.

Under standard nomenclature used throughout this disclosure, the terminal portion of the designated side chain is described last, preceded by the adjacent functionality toward the point of attachment.

In choosing compounds of the present invention, one of ordinary skill in the art will recognize that the various substituents, *i.e.* R¹, R², etc., are to be chosen in conformity with well-known principles of chemical structure connectivity and stability.

Unless expressly stated to the contrary, all ranges cited herein are inclusive. For example, in Formula I, "n is from 1 to 5" means n can be 1, 2, 3, 4, or 5. It is also to be understood that any range cited herein includes within its scope all of the sub-ranges within that range. Thus, for example, "n is from 1 to 5" is intended to include as aspects thereof, n is from 1 to 4, n is from 1 to 3, n is 1 or 2, n is from 2 to 5, n is from 2 to 4, and n is 2 or 3.

A "stable" compound is a compound which can be prepared and isolated and whose structure and properties remain or can be caused to remain essentially unchanged for a period of time sufficient to allow use of the compound for the purposes described herein (*e.g*., therapeutic administration to a subject). The compounds of the present invention are limited to stable compounds embraced by Formulas I.

The term "compound" refers to the compound and, in certain embodiments, to the extent they are stable, any hydrate or solvate thereof. A hydrate is the compound complexed with water, and a solvate is the compound complexed with an organic solvent.

As indicated above, the compounds of the present invention can be employed in the form of pharmaceutically acceptable salts. Those skilled in the art will recognize those instances in which the compounds of the invention may form salts. The term "pharmaceutically acceptable salt" refers to a salt (including an inner salt such as a zwitterion) which possesses effectiveness similar to the parent compound and which is not biologically or otherwise undesirable (*e.g.*, is neither toxic nor otherwise deleterious to the recipient thereof). Thus, an embodiment of the invention provides pharmaceutically acceptable salts of the compounds of the invention. The term "salt(s)", as employed herein, denotes any of the following: acidic salts formed with inorganic and/or organic acids, as well as basic salts formed with inorganic and/or organic bases. Salts of compounds of the invention may be formed by methods known to those of ordinary skill in the art, for example, by reacting a compound of the invention with an amount of acid or base, such as an equivalent amount, in a medium such as one in which the salt precipitates or in aqueous medium followed by lyophilization. All such acid salts and base salts are intended to be pharmaceutically acceptable salts within the scope of the invention and all acid and base salts are considered equivalent to the free forms of the corresponding compounds for purposes of the invention.

As set forth herein, the present invention includes pharmaceutical compositions comprising a compound of Formula I, an active biological ingredient, optionally one or more other active components (*e.g*., a second ABI or an API), and a pharmaceutically acceptable carrier. The characteristics of the carrier will depend on the route of administration. By "pharmaceutically acceptable" is meant that the ingredients of the pharmaceutical composition must be compatible with each other, do not interfere with the effectiveness of the active ingredient(s), and are not deleterious (*e.g*., toxic) to the recipient thereof. Thus, compositions according to the invention may, in addition to the inhibitor, contain diluents, fillers, salts, buffers, stabilizers, solubilizers, and other materials well known in the art.

The administration of a composition of the present invention may be suitably parenteral, wherein the composition is suitably formulated for administration by the selected route using formulation methods well known in the art, including, for example, the methods for preparing and administering formulations described in chapters 39, 41, 42, 44 and 45 in Remington - The Science and Practice of Pharmacy, 21st edition, 2006. In one embodiment, compounds of the invention are administered intravenously in a hospital setting. In another embodiment, administration is subcutaneous.

### II. The Compounds of the Invention:

The compounds of the invention (i.e. the Compounds of Formula I) are useful as excipients in pharmaceutical formulations comprising active biological ingredients (i.e. an antibody or antigen binding fragment thereof), especially formulations comprising a high concentration of active biological ingredient, to reduce the viscosity of the formulation. Compositions comprising a high concentration ABI and a compound of the invention are capable of being administered via intravenous or subcutaneous administration to a patient in need thereof.

In each of the various embodiments of the compounds of the invention described herein, each variable including those of Formula I, and the various embodiments thereof, is selected independently of the other variables unless otherwise indicated.

The present invention encompasses for each of the various embodiments of the compounds of the invention described herein, including those of Formula I, and the various embodiments thereof and the compounds of the examples, all forms of the compounds such as, for example, any solvates, hydrates, stereoisomers, and tautomers of said compounds and of any pharmaceutically acceptable salts thereof, unless otherwise indicated. Additionally, in the examples described herein, the compounds of the invention may be depicted in the salt form. In such cases, it is to be understood that the compounds of the invention include the free acid or free base forms of such salts, and any pharmaceutically acceptable salt of said free acid or free base forms.

In one aspect, the present invention includes compounds of Formula I: 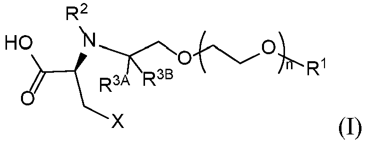 or a pharmaceutically acceptable salt thereof, wherein X, n, R¹, R², R^{3A}, and R^{3B} are as defined herein for the Compounds of Formula (I) (i.e. as defined in the Summary of the Invention); wherein the compounds may be suitable for use as an excipient in a liquid pharmaceutical formulation for lowering the overall viscosity of the solution.

A first embodiment of the invention (Embodiment E1) is a compound of Formula I, or a pharmaceutically acceptable salt thereof, wherein X, n, R¹, R², R^{3A}, and R^{3B} are as defined in Formula (I) in the Summary of the Invention. A compound having R² in the meaning of hydrogen and X in the meaning of unsubstituted phenyl is not part of the class of compounds according to claim 1.

A second embodiment (Embodiment E2) is a compound of Formula I, or a pharmaceutically acceptable salt thereof, wherein X is: 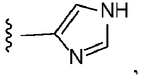 and all other variables are as defined in Embodiment E1.

A third embodiment (Embodiment E3) is a compound of Formula I, or a pharmaceutically acceptable salt thereof, wherein X is: 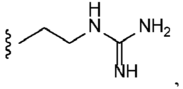 and all other variables are as defined in Embodiment E1.

A fourth embodiment (Embodiment E4) is a compound of Formula I, or a pharmaceutically acceptable salt thereof, wherein X is: 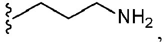 and all other variables are as defined in Embodiment E1.

A fifth embodiment (Embodiment E5) is a compound of Formula I, or a pharmaceutically acceptable salt thereof, wherein X is: A compound having X in 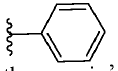 and all other variables are as defined in Embodiment E1. the meaning of unsubstituted phenyl is not part of the class of compounds according to claim 1.

A sixth embodiment (Embodiment E6) is a compound of Formula I, or a pharmaceutically acceptable salt thereof, wherein X is: 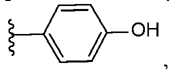 and all other variables are as defined in Embodiment E1.

A seventh embodiment (Embodiment E7) is a compound of Formula I, or a pharmaceutically acceptable salt thereof, wherein X is defined in any of Embodiments E2-E6, R¹ is H, and all other variables are as defined in Embodiment E1.

An eighth embodiment (Embodiment E8) is a compound of Formula I, or a pharmaceutically acceptable salt thereof, wherein X is defined in any of Embodiments E2-E6, R¹ is methyl, and all other variables are as defined in Embodiment E1.

A ninth embodiment (Embodiment E9) is a compound of Formula I, or a pharmaceutically acceptable salt thereof, wherein X is defined in any of Embodiments E2-E6, R¹ is defined in any of Embodiments E7-E8, R² is H, and all other variables are as defined in Embodiment E1. A compound having R2 in the meaning of hydrogen is not part of the class of compounds according to claim 1.

A tenth embodiment (Embodiment E10) is a compound of Formula I, or a pharmaceutically acceptable salt thereof, wherein X is defined in any of Embodiments E2-E6, R¹ is defined in any of Embodiments E7-E8, R² is 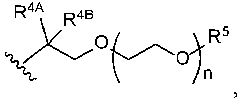 and all other variables are as defined in Embodiment E1.

An eleventh embodiment (Embodiment E11) is a compound of Formula I, or a pharmaceutically acceptable salt thereof, wherein X is defined in any of Embodiments E2-E6, R¹ is defined in any of Embodiments E7-E8, R² is 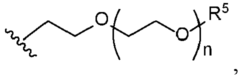 and all other variables are as defined in Embodiment E1.

In a sub-embodiment of Embodiment E11, R² is

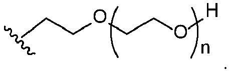

In a further sub-embodiment of Embodiment E11, R² is

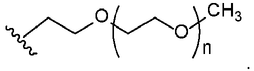

In another sub-embodiment of Embodiment E11, R² is

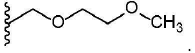

A twelfth embodiment (Embodiment E12) is a compound of Formula I, or a pharmaceutically acceptable salt thereof, wherein X is defined in any of Embodiments E2-E6, R¹ is defined in any of Embodiments E7-E8, R² is 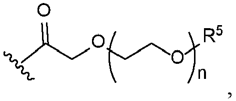 and all other variables are as defined in Embodiment E1.

In a sub-embodiment of Embodiment E12, R² is

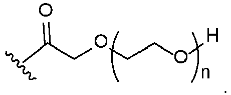

In a further sub-embodiment of Embodiment E12, R² is

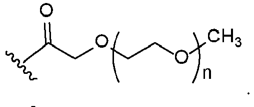

In a sub-embodiment of Embodiments E10-E12, the n in R² is 1. In further sub-embodiments of Embodiments E10-E12, the n in R² is 2. In additional sub-embodiments of Embodiments E10-E12, the n in R ² is 3. In still other sub-embodiments of Embodiments E10-E12, the n in R² is 4. In yet additional sub-embodiments of Embodiments E10-E12, the n in R² is 5. In other sub-embodiments of Embodiments E10-E12, the n in R² is 1 to 4, 1 to 3, or 1 to 2.

A thirteenth embodiment (Embodiment E13) is a compound of Formula I, or a pharmaceutically acceptable salt thereof, wherein X is defined in any of Embodiments E2-E6, R¹ is defined in any of Embodiments E7-E8, R² is defined in any of Embodiments E9-E12, R^{3A} and R^{3B} are each H, and all other variables are as defined in Embodiment E1.

A fourteenth embodiment (Embodiment E14) is a compound of Formula I, or a pharmaceutically acceptable salt thereof, wherein X is defined in any of Embodiments E2-E6, R¹ is defined in any of Embodiments E7-E8, R² is defined in any of Embodiments E9-E13, R^{3A} and R^{3B} together form oxo, and all other variables are as defined in Embodiment E1.

A fifteenth embodiment of the invention (Embodiment E15) is a compound of Formula I, having or consisting of the structure: 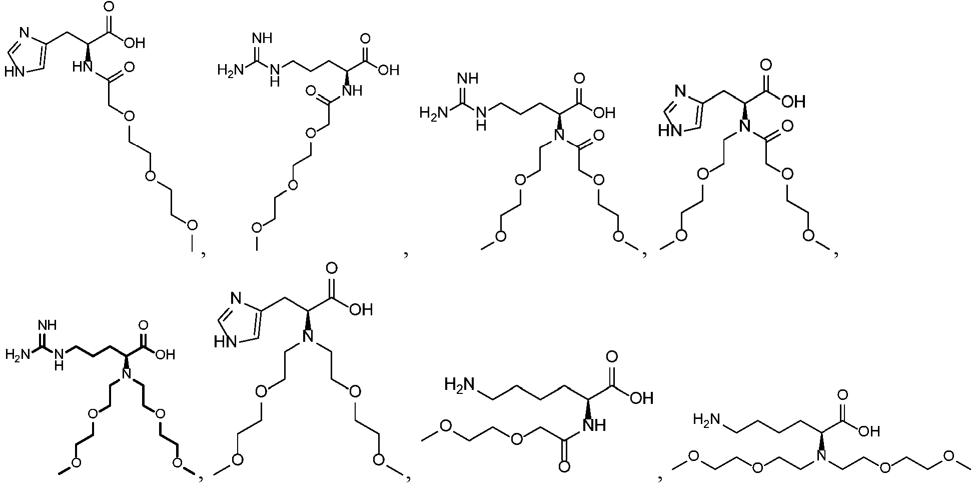 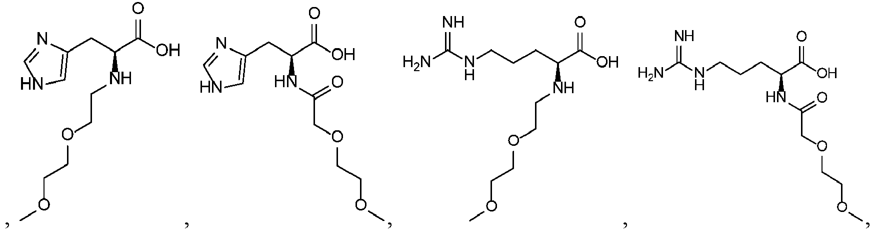 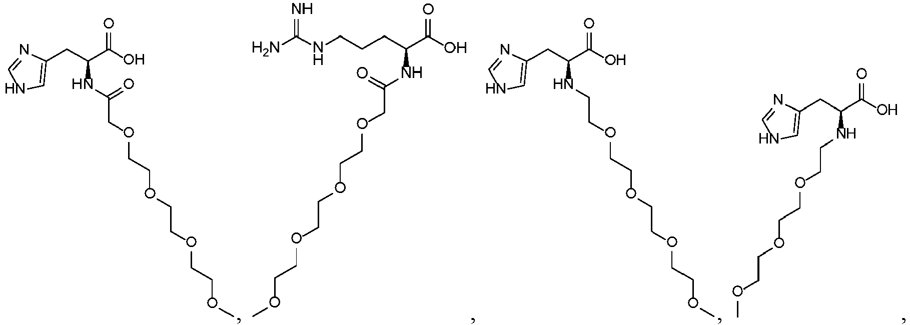 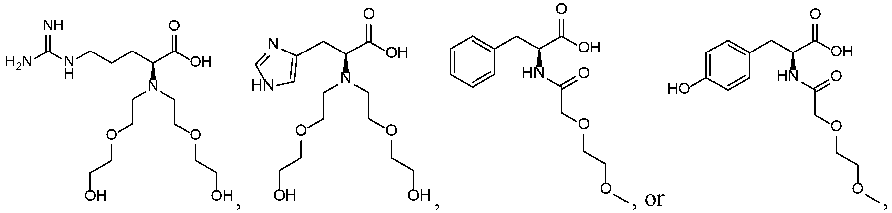 or a pharmaceutically acceptable salt thereof.

Other embodiments of the invention include the following:
(a) A pharmaceutical composition comprising an effective amount of an active biological ingredient, a compound of Formula I, as defined above, or a pharmaceutically acceptable salt thereof, and a pharmaceutically acceptable carrier.
(b) The pharmaceutical composition of (a), further comprising an effective amount of a second active biological ingredient or an active pharmaceutical ingredient.
(c) The pharmaceutical composition of (a), wherein the ABI is an antibody or antigen-binding fragment thereof.
(d) The pharmaceutical composition of (c), wherein the antibody or antigen-binding fragment thereof specifically binds to an antigen selected from the group consisting of: PD-1, PD-L1, PD-L2, CTLA4, LAG3, BTLA, TIM3, HVEM, GITR, CD27, TIGIT, ILT2, ILT3, ILT4, ILT5, SIRPα, NKG2A, NKG2C, NKG2E, TSLP, IL10, VISTA, VEGF, EGFR, Her2/neu, VEGF receptors, other growth factor receptors, CD20, CD28, CD40, CD-40L, CD70, OX-40, 4-1BB, and ICOS.
(e) The pharmaceutical composition of (d), wherein the antibody or antigen-binding fragment thereof specifically binds to an antigen selected from the group consisting of: PD-1, PD-L1, PD-L2, CTLA4, LAG3, GITR, CD27, and TIGIT.
(f) The pharmaceutical composition of (c), wherein the antibody or antigen-binding fragment thereof specifically binds to PD-1, PD-L1 or PD-L2.
(g) The pharmaceutical composition of (f), wherein the ABI is pembrolizumab, nivolumab, pidilizumab, atezolizumab, avelumab, or durvalumab.
(h) The pharmaceutical composition of (c), wherein the antibody or antigen-binding fragment thereof specifically binds to PD-1.
(i) The pharmaceutical composition of (a), wherein the ABI is pembrolizumab.
(j) The pharmaceutical composition of (f), (g) or (h), further comprising a second ABI, wherein the second ABI is an antibody or antigen-binding fragment thereof that specifically binds to an antigen selected from the group consisting of: CTLA4, LAG3, GITR, CD27, and TIGIT.
(k) A method for treating a disease or other pathological condition which comprises administering to a subject in need of such treatment a composition comprising an effective amount of a compound of Formula I, as defined above, or a pharmaceutically acceptable salt thereof, and a therapeutically effective amount of an ABI.
(l) A method for treating a cancerous condition which comprises administering to a subject in need of such treatment an effective amount of a compound of Formula I, as defined above, or a pharmaceutically acceptable salt thereof, in combination with a therapeutically effective amount of an ABI.
(m) A method for treating a disease or other pathological condition which comprises administering to a subject in need of such treatment a therapeutically effective amount of the pharmaceutical composition of (a), (b), (c), (d), (e), (f), (g), (h), (i), or (j).
(n) A method for treating a cancerous condition which comprises administering to a subject in need of such treatment a therapeutically effective amount of the pharmaceutical composition of (d), (e), (f), (g), (h), (i) or (j).
(q) A method of treating a cancerous condition as set forth in (l) or (n) wherein the cancer is selected from the group consisting of: melanoma, lung cancer, head and neck cancer, bladder cancer, breast cancer, gastrointestinal cancer, multiple myeloma, hepatocellular cancer, lymphoma, renal cancer, mesothelioma, ovarian cancer, esophageal cancer, anal cancer, biliary tract cancer, colorectal cancer, cervical cancer, thyroid cancer, salivary cancer, prostate cancer (e.g. hormone refractory prostate adenocarcinoma), pancreatic cancer, colon cancer, esophageal cancer, liver cancer, thyroid cancer, glioblastoma, glioma, and other neoplastic malignancies.

The references to the methods of treatment by therapy of this description are to be interpreted as references to compounds, pharmaceutical compositions and medicaments of the present invention for use in those methods. The present invention also includes a compound of Formula I, or a pharmaceutically acceptable salt thereof, for use in lowering the viscosity of a biological formulation (i.e. a formulation comprising an ABI).

The present invention further includes a pharmaceutical formulation comprising a compound of Formula I, or a pharmaceutically acceptable salt thereof, and an ABI selected from: (a) an anti-PD-1 antibody, or antigen-binding fragment thereof, (b) an anti-PDL1 antibody, or antigen-binding fragment thereof, (c) an anti-PDL2 antibody, or antigen-binding fragment thereof, (d) an anti-Her2/Neu antibody, or antigen-binding fragment thereof, (e) an anti-OX40 antibody, or antigen-binding fragment thereof, (e) an anti-4-1BB antibody, or antigen-binding fragment thereof, (f) an anti-CTLA4 antibody, or antigen-binding fragment thereof, (g) an anti-LAG3 antibody, or antigen-binding fragment thereof, (h) an anti-GITR antibody, or antigen-binding fragment thereof, (i) an anti-CD27 antibody, or antigen-binding fragment thereof, and (j) an anti-TIGIT antibody, or antigen-binding fragment thereof, (i) for use in, (ii) for use as a medicament for, or (iii) for use in the preparation (or manufacture) of a medicament for, treating cancer. In these uses, the compounds of the present invention can optionally be employed in combination with one or more additional therapeutic agents.

In the embodiments of the compounds and salts of the invention, it is to be understood that each embodiment may be combined with one or more other embodiments, to the extent that such a combination provides a stable compound or salt and is consistent with the description of the embodiments. It is further to be understood that the embodiments of compositions and methods provided as (a) through (q) above are understood to include all embodiments of the compounds and/or salts, including such embodiments as result from combinations of embodiments.

Additional embodiments of the invention include the pharmaceutical compositions, combinations and methods set forth above and the uses set forth in the preceding paragraph, wherein the compound of the present invention employed therein is a compound of one of the embodiments, sub-embodiments, classes or sub-classes described above. The compound may optionally be used in the form of a pharmaceutically acceptable salt in these embodiments.

Additional embodiments of the present invention include each of the pharmaceutical compositions, combinations, methods and uses set forth in the preceding paragraphs, wherein the compound of the present invention or its salt employed therein is substantially pure.

### III. Compositions of the Invention

The invention provides stable biological formulations (i.e. pharmaceutical compositions) comprising a compound of the invention (i.e. a compound of Formula I), or a pharmaceutically acceptable salt thereof, and an active biological ingredient (ABI), wherein the ABI is an antibody or antigen binding fragment thereof, or a therapeutic protein or peptide. In additional embodiments, the invention provides stable formulations comprising a compound of the invention and a therapeutically effective amount of an active pharmaceutical ingredient. In specific embodiments, the API is a small molecule.

The invention further provides pharmaceutical compositions comprising a compound of the invention, or a pharmaceutically acceptable salt thereof, and an antibody or antigen-binding fragment thereof, that specifically binds to an antigen selected from the group consisting of: PD-1, PD-L1, PD-L2, CTLA4, LAG3, BTLA, TIM3, HVEM, GITR, CD27, TIGIT, ILT2, ILT3, ILT4, ILT5, SIRPα, NKG2A, NKG2C, NKG2E, TSLP, IL10, VISTA, VEGF, EGFR, Her2/neu, VEGF receptors, other growth factor receptors, CD20, CD28, CD40, CD-40L, CD70, OX-40, 4-1BB, and ICOS.

In embodiments of the invention, the amount of ABI is a therapeutically acceptable amount.

In specific embodiments, the invention relates to a pharmaceutical composition comprising a compound of the invention (i.e. a compound of formula (I)), or a pharmaceutically acceptable salt thereof, and an anti-human PD-1 antibody or antigen binding fragment thereof, which specifically binds to human PD-1 (e.g. a human or humanized anti-PD-1 antibody) as the active biological ingredient (PD-1 ABI), as well as methods for using the formulations of the invention. Any anti-PD-1 antibody or antigen binding fragment thereof can be used in the compositions and methods of the invention. In particular embodiments, the PD-1 ABI is an anti-PD-1 antibody, which is selected from pembrolizumab (including any pembrolizumab biosimilar), and nivolumab (including any nivolumab biosimilar). In specific embodiments, the anti-PD-1 antibody is pembrolizumab. In alternative embodiments, the anti-PD-1 antibody is nivolumab. Table 2 provides amino acid sequences for exemplary anti-human PD-1 antibodies pembrolizumab and nivolumab. Alternative PD-1 antibodies and antigen-binding fragments that are useful in the formulations and methods of the invention are shown in Table 3.

In some embodiments ,an anti-human PD-1 antibody or antigen binding fragment thereof for use in the pharmaceutical compositions of the invention comprises three light chain CDRs of CDRL1, CDRL2 and CDRL3 and/or three heavy chain CDRs of CDRH1, CDRH2 and CDRH3.

In one embodiment of the invention, CDRL1 is SEQ ID NO:1 or a variant of SEQ ID NO:1, CDRL2 is SEQ ID NO:2 or a variant of SEQ ID NO:2, and CDRL3 is SEQ ID NO:3 or a variant of SEQ ID NO:3.

In one embodiment, CDRH1 is SEQ ID NO:6 or a variant of SEQ ID NO:6, CDRH2 is SEQ ID NO: 7 or a variant of SEQ ID NO:7, and CDRH3 is SEQ ID NO:8 or a variant of SEQ ID NO:8.

In one embodiment, the three light chain CDRs are SEQ ID NO: 1, SEQ ID NO:2, and SEQ ID NO:3 and the three heavy chain CDRs are SEQ ID NO:6, SEQ ID NO:7 and SEQ ID NO:8.

In an alternative embodiment of the invention, CDRL1 is SEQ ID NO:11 or a variant of SEQ ID NO:11, CDRL2 is SEQ ID NO:12 or a variant of SEQ ID NO:12, and CDRL3 is SEQ ID NO:13 or a variant of SEQ ID NO:13.

In one embodiment, CDRH1 is SEQ ID NO:16 or a variant of SEQ ID NO:16, CDRH2 is SEQ ID NO:17 or a variant of SEQ ID NO:17, and CDRH3 is SEQ ID NO:18 or a variant of SEQ ID NO:18.

In one embodiment, the three light chain CDRs are SEQ ID NO: 1, SEQ ID NO:2, and SEQ ID NO:3 and the three heavy chain CDRs are SEQ ID NO:6, SEQ ID NO:7 and SEQ ID NO:8.

In an alternative embodiment, the three light chain CDRs are SEQ ID NO: 11, SEQ ID NO:12, and SEQ ID NO:13 and the three heavy chain CDRs are SEQ ID NO:16, SEQ ID NO:17 and SEQ ID NO:18.

In a further embodiment of the invention, CDRL1 is SEQ ID NO:21 or a variant of SEQ ID NO:21, CDRL2 is SEQ ID NO:22 or a variant of SEQ ID NO:22, and CDRL3 is SEQ ID NO:23 or a variant of SEQ ID NO:23.

In yet another embodiment, CDRH1 is SEQ ID NO:24 or a variant of SEQ ID NO:24, CDRH2 is SEQ ID NO: 25 or a variant of SEQ ID NO:25, and CDRH3 is SEQ ID NO:26 or a variant of SEQ ID NO:26.

In another embodiment, the three light chain CDRs are SEQ ID NO:21, SEQ ID NO:22, and SEQ ID NO:23 and the three heavy chain CDRs are SEQ ID NO:24, SEQ ID NO:25 and SEQ ID NO:26.

In certain embodiments, the invention provides a pharmaceutical composition comprising a compound of formula (I), or a pharmaceutically acceptable salt thereof, and an anti-human PD-1 antibody, or an antigen binding fragment thereof, wherein the anti-PD-1 antibody or antigen binding fragment comprises a light chain variable region and a heavy chain variable region. In some embodiments, the light chain variable region comprises SEQ ID NO:4 or a variant of SEQ ID NO:4, and the heavy chain variable region comprises SEQ ID NO:9 or a variant of SEQ ID NO:9. In further embodiments, the light chain variable region comprises SEQ ID NO:14 or a variant of SEQ ID NO:14, and the heavy chain variable region comprises SEQ ID NO:19 or a variant of SEQ ID NO:19. In further embodiments, the heavy chain variable region comprises SEQ ID NO:27 or a variant of SEQ ID NO:27 and the light chain variable region comprises SEQ ID NO:28 or a variant of SEQ ID NO:28, SEQ ID NO:29 or a variant of SEQ ID NO:29, or SEQ ID NO:30 or a variant of SEQ ID NO:30. In such embodiments, a variant light chain or heavy chain variable region sequence is identical to the reference sequence except having one, two, three, four or five amino acid substitutions. In some embodiments, the substitutions are in the framework region (i.e., outside of the CDRs). In some embodiments, one, two, three, four or five of the amino acid substitutions are conservative substitutions.

In one embodiment of the pharmaceutical compositions of the invention, the anti-human PD-1 antibody or antigen binding fragment comprises a light chain variable region comprising or consisting of SEQ ID NO:4 and a heavy chain variable region comprising or consisting SEQ ID NO:9. In a further embodiment, the anti-human PD-1 antibody or antigen binding fragment comprises a light chain variable region comprising or consisting of SEQ ID NO:14 and a heavy chain variable region comprising or consisting of SEQ ID NO:19. In one embodiment of the formulations of the invention, the anti-human PD-1 antibody or antigen binding fragment comprises a light chain variable region comprising or consisting of SEQ ID NO:28 and a heavy chain variable region comprising or consisting SEQ ID NO:27. In a further embodiment, the anti-human PD-1 antibody or antigen binding fragment comprises a light chain variable region comprising or consisting of SEQ ID NO:29 and a heavy chain variable region comprising or consisting SEQ ID NO:27. In another embodiment, the antibody or antigen binding fragment comprises a light chain variable region comprising or consisting of SEQ ID NO:30 and a heavy chain variable region comprising or consisting SEQ ID NO:27.

In another embodiment, the pharmaceutical compositions of the invention comprise a compound of formula (I), or a pharmaceutically acceptable salt thereof, and an anti-human PD-1antibody or antigen binding protein that has a V_{L} domain and/or a V_{H} domain with at least 95%, 90%, 85%, 80%, 75% or 50% sequence homology to one of the V_{L} domains or V_{H} domains described above, and exhibits specific binding to PD-1. In another embodiment, the anti-human PD-1 antibody or antigen binding protein of the pharmaceutical compositions of the invention comprises V_{L} and V_{H} domains having up to 1, 2, 3, 4, or 5 or more amino acid substitutions, and exhibits specific binding to PD-1.

In any of the embodiments above, the PD-1 ABI may be a full-length anti-PD-1 antibody or an antigen binding fragment thereof that specifically binds human PD-1. In certain embodiments, the PD-1 ABI is a full-length anti-PD-1 antibody selected from any class of immunoglobulins, including IgM, IgG, IgD, IgA, and IgE. Preferably, the antibody is an IgG antibody. Any isotype of IgG can be used, including IgG₁, IgG₂, IgG₃, and IgG₄. Different constant domains may be appended to the V_{L} and V_{H} regions provided herein. For example, if a particular intended use of an antibody (or fragment) of the present invention were to call for altered effector functions, a heavy chain constant domain other than IgG1 may be used. Although IgG1 antibodies provide for long half-life and for effector functions, such as complement activation and antibody-dependent cellular cytotoxicity, such activities may not be desirable for all uses of the antibody. In such instances an IgG4 constant domain, for example, may be used.

In embodiments of the invention, the PD-1 ABI is an anti-PD-1 antibody comprising a light chain comprising or consisting of a sequence of amino acid residues as set forth in SEQ ID NO:5 and a heavy chain comprising or consisting of a sequence of amino acid residues as set forth in SEQ ID NO: 10. In alternative embodiments, the PD-1 ABI is an anti-PD-1 antibody comprising a light chain comprising or consisting of a sequence of amino acid residues as set forth in SEQ ID NO: 15 and a heavy chain comprising or consisting of a sequence of amino acid residues as set forth in SEQ ID NO:20. In further embodiments, the PD-1 API is an anti-PD-1 antibody comprising a light chain comprising or consisting of a sequence of amino acid residues as set forth in SEQ ID NO:32 and a heavy chain comprising or consisting of a sequence of amino acid residues as set forth in SEQ ID NO:31. In additional embodiments, the PD-1 API is an anti-PD-1 antibody comprising a light chain comprising or consisting of a sequence of amino acid residues as set forth in SEQ ID NO:33 and a heavy chain comprising or consisting of a sequence of amino acid residues as set forth in SEQ ID NO:31. In yet additional embodiments, the PD-1 API is an anti-PD-1 antibody comprising a light chain comprising or consisting of a sequence of amino acid residues as set forth in SEQ ID NO:34 and a heavy chain comprising or consisting of a sequence of amino acid residues as set forth in SEQ ID NO:31. In some co-formulations of the invention, the PD-1 API is pembrolizumab or a pembrolizumab biosimilar. In some co-formulations of the invention, the PD-1 API is nivolumab or a nivolumab biosimilar.

Ordinarily, amino acid sequence variants of the anti-PD-1 antibodies and antigen binding fragments of the pharmaceutical compositions of the invention will have an amino acid sequence having at least 75% amino acid sequence identity with the amino acid sequence of a reference antibody or antigen binding fragment (e.g. heavy chain, light chain, V_{H}, V_{L}, or humanized sequence), more preferably at least 80%, more preferably at least 85%, more preferably at least 90%, and most preferably at least 95, 98, or 99%. Identity or homology with respect to a sequence is defined herein as the percentage of amino acid residues in the candidate sequence that are identical with the anti-PD-1 residues, after aligning the sequences and introducing gaps, if necessary, to achieve the maximum percent sequence identity, and not considering any conservative substitutions as part of the sequence identity. None of N-terminal, C-terminal, or internal extensions, deletions, or insertions into the antibody sequence shall be construed as affecting sequence identity or homology.

Sequence identity refers to the degree to which the amino acids of two polypeptides are the same at equivalent positions when the two sequences are optimally aligned. Sequence identity can be determined using a BLAST algorithm wherein the parameters of the algorithm are selected to give the largest match between the respective sequences over the entire length of the respective reference sequences. The following references relate to BLAST algorithms often used for sequence analysis: BLAST ALGORITHMS: Altschul, S.F., et al., (1990) J. Mol. Biol. 215:403-410; Gish, W., et al., (1993) Nature Genet. 3:266-272; Madden, T.L., et al., (1996) Meth. Enzymol. 266: 131-141; Altschul, S.F., et al., (1997) Nucleic Acids Res. 25:3389-3402; Zhang, J., et al., (1997) Genome Res. 7:649-656; Wootton, J.C., et al., (1993) Comput. Chem. 17:149-163; Hancock, J.M. et al., (1994) Comput. Appl. Biosci. 10:67-70; ALIGNMENT SCORING SYSTEMS: Dayhoff, M.O., et al., "A model of evolutionary change in proteins." in Atlas of Protein Sequence and Structure, (1978) vol. 5, suppl. 3. M.O. Dayhoff (ed.), pp. 345-352, Natl. Biomed. Res. Found., Washington, DC; Schwartz, R.M., et al., "Matrices for detecting distant relationships." in Atlas of Protein Sequence and Structure, (1978) vol. 5, suppl. 3." M.O. Dayhoff (ed.), pp. 353-358, Natl. Biomed. Res. Found., Washington, DC; Altschul, S.F., (1991) J. Mol. Biol. 219:555-565; States, D.J., et al., (1991) Methods 3:66-70; Henikoff, S., et al., (1992) Proc. Natl. Acad. Sci. USA 89: 10915-10919; Altschul, S.F., et al., (1993) J. Mol. Evol. 36:290-300; ALIGNMENT STATISTICS: Karlin, S., et al., (1990) Proc. Natl. Acad. Sci. USA 87:2264-2268; Karlin, S., et al., (1993) Proc. Natl. Acad. Sci. USA 90:5873-5877; Dembo, A., et al., (1994) Ann. Prob. 22:2022-2039; and Altschul, S.F. "Evaluating the statistical significance of multiple distinct local alignments." in Theoretical and Computational Methods in Genome Research (S. Suhai, ed.), (1997) pp. 1-14, Plenum, New York.

Likewise, either class of light chain can be used in the compositions and methods herein. Specifically, kappa, lambda, or variants thereof are useful in the present compositions and methods.

**Table 2. Exemplary PD-1 Antibody Sequences**

| **Antibody Feature** | **Amino Acid Sequence** | **SEQ ID NO.** |
|---|---|---|
| **Pembrolizumab Light Chain** | | |
| CDR1 | RASKGVSTSGYSYLH | 1 |
| CDR2 | LASYLES | 2 |
| CDR3 | QHSRDLPLT | 3 |
| Variable Region | | 4 |
| Light Chain | EIVLTQSPATLSLSPGERATLSCRASKGVSTSGYSYLHWY QQKPGQAPRLLIYLASYLESGVPARFSGSGSGTDFTLTISS LEPEDFAVYYCQHSRDLPLTFGGGTKVEIKRTVAAPSVFI FPPSDEQLKSGTASVVCLLNNFYPREAKVQWKVDNALQ SGNSQESVTEQDSKDSTYSLSSTLTLSKADYEKHKVYAC EVTHQGLSSPVTKSFNRGEC | 5 |

| **Pembrolizumab Heavy Chain** | | |
|---|---|---|
| CDR1 | NYYMY | 6 |
| CDR2 | GINPSNGGTNFNEKFKN | 7 |
| CDR3 | RDYRFDMGFDY | 8 |
| Variable Region | | 9 |
| Heavy Chain | QVQLVQSGVEVKKPGASVKVSCKASGYTFTNYYMYWV RQAPGQGLEWMGGINPSNGGTNFNEKFKNRVTLTTDSST TTAYMELKSLQFDDTAVYYCARRDYRFDMGFDYWGQG TTVTVSSASTKGPSVFPLAPCSRSTSESTAALGCLVKDYF PEPVTVSWNSGALTSGVHTFPAVLQSSGLYSLSSVVTVPS SSLGTKTYTCNVDHKPSNTKVDKRVESKYGPPCPPCPAP EFLGGPSVFLFPPKPKDTLMISRTPEVTCVVVDVSQEDPE VQFNWYVDGVEVHNAKTKPREEQFNSTYRVVSVLTVLH QDWLNGKEYKCKVSNKGLPSSIEKTISKAKGQPREPQVY TLPPSQEEMTKNQVSLTCLVKGFYPSDIAVEWESNGQPE NNYKTTPPVLDSDGSFFLYSRLTVDKSRWQEGNVFSCSV MHEALHNHYTQKSLSLSLGK | 10 |

| **Nivolumab Light Chain** | | |
|---|---|---|
| **Antibody Feature** | **Amino Acid Sequence** | **SEQ ID NO.** |
| CDR1 | RASQSVSSYLA | 11 |
| CDR2 | DASNRAT | 12 |
| CDR3 | QQSSNWPRT | 13 |
| Variable Region | | 14 |
| Light Chain | EIVLTQSPATLSLSPGERATLSCRASQSVSSYLAWYQQKP GQAPRLLIYDASNRATGIPARFSGSGSGTDFTLTISSLEPE DFAVYYCQQSSNWPRTFGQGTKVEIKRTVAAPSVFIFPPS DEQLKSGTASVVCLLNNFYPREAKVQWKVDNALQSGNS QESVTEQDSKDSTYSLSSTLTLSKADYEKHKVYACEVTH QGLSSPVTKSFNRGEC | 15 |

| **Nivolumab Heavy Chain** | | |
|---|---|---|
| CDR1 | NSGMH | 16 |
| CDR2 | VIWYDGSKRYYADSVKG | 17 |
| CDR3 | NDDY | 18 |
| Variable Region | | 19 |
| Heavy Chain | QVQLVESGGGVVQPGRSLRLDCKASGITFSNSGMHWVR QAPGKGLEWVAVIWYDGSKRYYADSVKGRFTISRDNSK NTLFLQMNSLRAEDTAVYYCATNDDYWGQGTLVTVSSA STKGPSVFPLAPCSRSTSESTAALGCLVKDYFPEPVTVSW NSGALTSGVHTFPAVLQSSGLYSLSSVVTVPSSSLGTKTY TCNVDHKPSNTKVDKRVESKYGPPCPPCPAPEFLGGPSVF LFPPKPKDTLMISRTPEVTCVVVDVSQEDPEVQFNWYVD GVEVHNAKTKPREEQFNSTYRVVSVLTVLHQDWLNGKE YKCKVSNKGLPSSIEKTISKAKGQPREPQVYTLPPSQEEM TKNQVSLTCLVKGFYPSDIAVEWESNGQPENNYKTTPPV LDSDGSFFLYSRLTVDKSRWQEGNVFSCSVMHEALHNH YTQKSLSLSLGK | 20 |

**Table 3. Additional PD-1 Antibodies and Antigen Binding Fragments Useful in the Compositions of the Invention.**

| A. Antibodies and antigen binding fragments comprising light and heavy chain CDRs of hPD-1.08A in WO2008/156712 | |
|---|---|
| CDRL1 | SEQ ID NO:21 |
| CDRL2 | SEQ ID NO:22 |
| CDRL3 | SEQ ID NO:23 |
| CDRH1 | SEQ ID NO:24 |
| CDRH2 | SEQ ID NO:25 |
| CDRH3 | SEQ ID NO:26 |

| B. Antibodies and antigen binding fragments comprising the mature h109A heavy chain variable region and one of the mature K09A light chain variable regions in WO 2008/156712 | |
|---|---|
| Heavy chain VR | SEQ ID NO:27 |
| Light chain VR | SEQ ID NO:28 or SEQ ID NO:29 or SEQ ID NO:30 |

| C. Antibodies and antigen binding fragments comprising the mature 409 heavy chain and one of the mature K09A light chains in WO 2008/156712 | |
|---|---|
| Heavy chain | SEQ ID NO:31 |
| Light chain | SEQ ID NO:32 or SEQ ID NO:33 or SEQ ID NO:34 |

In some embodiments of the pharmaceutical compositions of the invention, the ABI (e.g. the anti-PD-1 antibody or antigen binding fragment thereof) is present in a concentration of from about 10 mg/mL to about 250 mg/mL. In additional embodiments the ABI is present in a concentration of from about 25 mg/mL to about 250 mg/mL, from about 50 mg/mL to about 250 mg/mL, from about 75 mg/mL to about 250 mg/mL, from about 100 mg/mL to about 250 mg/mL, from about 10 mg/mL to about 200 mg/mL, from about 25 mg/mL to about 200 mg/mL, from about 50 mg/mL to about 200 mg/mL, from about 75 mg/mL to about 200 mg/mL, from about 100 mg/mL to about 200 mg/mL, from about 10 mg/mL to about 150 mg/mL, from about 25 mg/mL to about 150 mg/mL, from about 50 mg/mL to about 150 mg/mL, from about 75 mg/mL to about 150 mg/mL, from about 100 mg/mL to about 150 mg/mL, from about 100 mg/mL to about 250 mg/mL, or from about 100 mg/mL to about 200 mg/mL.

In alternative embodiments, the ABI is present in a concentration of about 10 mg/mL, about 20 mg/mL, about 25 mg/mL, about 30 mg/mL, about 40 mg/mL, about 50 mg/mL, about 60 mg/mL, about 70 mg/mL, about 75 mg/mL, about 80 mg/mL, about 90 mg/mL, about 100 mg/mL, about 110 mg/mL, about 120 mg/mL, about 130 mg/mL, about 140 mg/mL, about 150 mg/mL, about 160 mg/mL, about 170 mg/mL, about 180 mg/mL, about 190 mg/mL, about 200 mg/mL, about 210 mg/mL, about 220 mg/mL, about 230 mg/mL, about 240 mg/mL, or about 250 mg/mL.

As noted herein, compounds of Formula I are useful for lowering the viscosity of a solution, thereby allowing a high concentrations of ABI to be utilized in a solution formulation (i.e. without the need for lyophilization/reconsitution to achieve a high concentration of ABI). Thus, in certain embodiments, the pharmaceutical composition of the invention is an aqueous solution. In embodiments of this aspect of the invention, the viscosity of the solution is ≤30 mPa.s (milliPascal.second or cP (centipoise)), mPa-S, ≤29 mPa-S, ≤28 mPa-S, ≤27 mPa-S, ≤26 mPa-S, ≤25 mPa-S, ≤24 mPa-S, ≤23 mPa-S, ≤22 mPa-S, ≤21 mPa-S, ≤20 mPa-S, ≤19 mPa-S, or ≤18 mPa-S. In further embodiments, the viscosity of the solution is from about 15 mPa-S to about 30 mPa-S, from about 17 mPa-S to about 28 mPa-S, from about 17 mPa-S to about 27 mPa-S, from about 17 mPa-S to about 26 mPa-S, from about 17 mPa-S to about 25 mPa-S, from about 18 mPa-S to about 28 mPa-S, from about 18 mPa-S to about 27 mPa-S, from about 18 mPa-S to about 26 mPa-S, from about 18 mPa-S to about 25 mPa-S, from about 19 mPa-S to about 28 mPa-S, from about 19 mPa-S to about 27 mPa-S, from about 19 mPa-S to about 26 mPa-S, from about 19 mPa-S to about 25 mPa-S, from about 20 mPa-S to about 28 mPa-S, from about 20 mPa-S to about 27 mPa-S, from about 20 mPa-S to about 26 mPa-S, or from about 20 mPa-S to about 25 mPa-S. Viscosity can measured using any viscometer known in the art, e.g. an mVROC (Viscometer/Rheometer-On-A-Chip, RheoSense, Inc. San Ramon, CA).

As dictated by the need of the particular formulation being developed, one or more additional excipients may be added to the pharmaceutical compositions of the invention. Such additional excipients are safe for use in pharmaceutical compositions and do not detrimentally impact the stability of the formulation. One example of an additional excipient that may be added to the formulations of the invention include adjuvants, which may be added to increase the immune response of the patient's immune system to the ABI. Other excipients that may be added to the formulations include, but are not limited to: a buffer, a stabilizer, a solubilizer, a tonicity modifier, a chelating agent, a preservative, dextran, dextran sulfate, dextran T40, diethanolamine, guanidine, calcium chloride, sodium citrate, albumin, gelatin, polyethylene glycol (PEG), lipids, and heparin. The skilled artisan is readily able to determine which additional excipients should be included in a desired pharmaceutical formulation, dependant on its function in the formulation, as well as the projected mode of administration, dosage, and other factors such as the expected storage time and temperature of the formulation. The skilled artisan recognizes that the amount of the additional excipients may vary, and can readily determine a proper amount that is both safe for administration to humans or animals and effective for the desired function. Typically, an additional excipient may be present at a concentration of about 10 to about 500 mM.

### IV. Methods of Use

In one aspect, the invention relates to a method for lowering the viscosity of a pharmaceutical formulation comprising adding a compound of Formula I to the formulation, wherein the formulation is an aqueous solution. In embodiments of the invention, the pharmaceutical formulation comprises an ABI. In specific embodiments, the ABI is present in a concentration of 50 mg/mL or more, 75 mg/mL or more, 100 mg/mL or more, 125 mg/mL or more, 150 mg/mL or more, 175 mg/mL or more or 200 mg/mL or more.

Thus, the invention provides a method for lowering the viscosity of a pharmaceutical formulation comprising:
(a) providing a pharmaceutical formulation comprising an ABI and a compound of Formula I, wherein the ABI is present at a concentration of about 50 mg/mL to about 250 mg/mL, wherein the formulation is in aqueous solution; and
(b) adding a compound of Formula I to the solution;
wherein the viscosity of the pharmaceutical formulation following addition of the compound is ≤30 mPa-S, ≤29 mPa-S, ≤28 mPa-S, ≤27 mPa-S, ≤26 mPa-S, ≤25 mPa-S, ≤24 mPa-S, ≤23 mPa-S, ≤22 mPa-S, ≤21 mPa-S, ≤20 mPa-S, ≤19 mPa-S, or ≤18 mPa-S.

The viscosity of a pharmaceutical composition of the invention in solution is lower than a pharmaceutical composition comprising the same excipients and at the same pH, but absent a compound of Formula I. In certain embodiments of the invention, the viscosity of a pharmaceutical composition of the invention in solution is lower than the same composition comprising arginine, or a pharmaceutically acceptable salt thereof, histidine, or a pharmaceutically acceptable salt thereof, phenylalanine, or a pharmaceutically acceptable salt thereof, tyrosine, or a pharmaceutically acceptable salt thereof, or lysine, or a pharmaceutically acceptable salt thereof (i.e. without a compound of Formula I).

Disclosed herein, but not claimed, is a method of treating cancer in a subject, the method comprising administering to the subject an effective amount of a pharmaceutical composition comprising (1) an ABI (2) a compound of Formula (I), or a pharmaceutically acceptable salt thereof and (3) a pharmaceutically acceptable carrier, wherein the ABI is an antibody, or antigen-binding fragment thereof, or a thereapeutic protein or peptide, that treats cancer. In specific embodiments of this method, the composition is administered to the subject via intravenous administration. In other embodiments, the composition is administered to the subject by subcutaneous administration.

In any of the methods of the invention, the cancer can be selected from the group consisting of: melanoma, lung cancer, head and neck cancer, bladder cancer, breast cancer, gastrointestinal cancer, multiple myeloma, hepatocellular cancer, lymphoma, renal cancer, mesothelioma, ovarian cancer, esophageal cancer, anal cancer, biliary tract cancer, colorectal cancer, cervical cancer, thyroid cancer, salivary cancer,. prostate cancer (e.g. hormone refractory prostate adenocarcinoma), pancreatic cancer, colon cancer, esophageal cancer, liver cancer, thyroid cancer, glioblastoma, glioma, and other neoplastic malignancies.

In some embodiments, the lung cancer in non-small cell lung cancer.

In alternate embodiments, the lung cancer is small-cell lung cancer.

In some embodiments, the lymphoma is Hodgkin lymphoma.

In other embodiments, the lymphoma is non-Hodgkin lymphoma. In particular embodiments, the lymphoma is mediastinal large B-cell lymphoma.

In some embodiments, the breast cancer is triple negative breast cancer.

In further embodiments , the breast cancer is ER+/HER2- breast cancer.

In some embodiments, the bladder cancer is urothelial cancer.

In some embodiments, the head and neck cancer is nasopharyngeal cancer. In some embodiments, the cancer is thyroid cancer. In other embodiments, the cancer is salivary cancer. In other embodiments, the cancer is squamous cell carcinoma of the head and neck.

In some embodiments, the cancer is metastatic colorectal cancer with high levels of microsatellite instability (MSI-H).

In some embodiments, the cancer is selected from the group consisting of: melanoma, non-small cell lung cancer, relapsed or refractory classical Hodgkin lymphoma, mediastinal large B-cell lymphoma, head and neck squamous cell carcinoma, urothelial cancer, esophageal cancer, gastric cancer, cervical cancer, and hepatocellular cancer.

In other embodiments of the above treatment methods, the cancer is a Heme malignancy. In certain embodiments, the Heme malignancy is acute lymphoblastic leukemia (ALL), acute myeloid leukemia (AML), chronic lymphocytic leukemia (CLL), chronic myeloid leukemia (CML), diffuse large B-cell lymphoma (DLBCL), EBV-positive DLBCL, primary mediastinal large B-cell lymphoma, T-cell/histiocyte-rich large B-cell lymphoma, follicular lymphoma, Hodgkin's lymphoma (HL), mantle cell lymphoma (MCL), multiple myeloma (MM), myeloid cell leukemia-1 protein (Mcl-1), myelodysplastic syndrome (MDS), non-Hodgkin lymphoma (NHL), or small lymphocytic lymphoma (SLL).

Malignancies that demonstrate improved disease-free and overall survival in relation to the presence of tumor-infiltrating lymphocytes in biopsy or surgical material, e.g. melanoma, colorectal, liver, kidney, stomach/esophageal, breast, pancreas, and ovarian cancer are encompassed in the methods and treatments described herein. Such cancer subtypes are known to be susceptible to immune control by T lymphocytes. Additionally, included are refractory or recurrent malignancies whose growth may be inhibited using the antibodies described herein.

In some embodiments, the compositions of the invention are administered to a subject having a cancer characterized by elevated expression of PD-L1 and/or PD-L2 in tested tissue samples, including: ovarian, renal, colorectal, pancreatic, breast, liver, gastric, esophageal cancers and melanoma. Additional cancers that can benefit from treatment with the compositions of the invention include those associated with persistent infection with viruses such as human immunodeficiency viruses, hepatitis viruses class A, B and C, Epstein Barr virus, human papilloma viruses that are known to be causally related to for instance Kaposi's sarcoma, liver cancer, nasopharyngeal cancer, lymphoma, cervical, vulval, anal, penile and oral cancers.

Additional aspects include methods of using a pharmaceutical composition of the invention to treat a patient having, suspected of having, or at risk for having an infection or infectious disease. In specific embodiments of this aspect of the invention, the ABI of the pharmaceutical compsotion is an antagonist anti-PD-1 antibody or antigen-binding fragment. Thus, the invention provides a method for treating chronic infection in a mammalian subject comprising administering an effective amount of a composition of the invention to the subject. In some specific embodiments of this method, the composition is administered to the subject via intravenous administration. In other embodiments, the composition is administered to the subject by subcutaneous administration.

In this aspect, the compositions of the invention can be used alone, or in combination with vaccines, to stimulate the immune response to pathogens, toxins, and self-antigens. The compositions of the invention can be used to stimulate immune response to viruses infectious to humans, including but not limited to: human immunodeficiency viruses, hepatitis viruses class A, B and C, Epstein Barr virus, human cytomegalovirus, human papilloma viruses, and herpes viruses. Compositions of the invention that comprise antagonist anti-PD-1 antibodies or antibody fragments can be used to stimulate immune response to infection with bacterial or fungal parasites, and other pathogens. Viral infections with hepatitis B and C and HIV are among those considered to be chronic viral infections.

The compositions of the invention may be administered to a patient in combination with one or more "additional therapeutic agents". The additional therapeutic agent may be a biotherapeutic agent (including but not limited to antibodies to VEGF, EGFR, Her2/neu, VEGF receptors, other growth factor receptors, CD20, CD40, CD-40L, OX-40, 4-1BB, and ICOS), a growth inhibitory agent, an immunogenic agent (for example, attenuated cancerous cells, tumor antigens, antigen presenting cells such as dendritic cells pulsed with tumor derived antigen or nucleic acids, immune stimulating cytokines (for example, IL-2, IFNα2, GM-CSF), and cells transfected with genes encoding immune stimulating cytokines such as but not limited to GM-CSF).

As noted above, in some embodiments of the methods of the invention, the method further comprises administering an additional therapeutic agent. In particular embodiments, the additional therapeutic agent is an anti-LAG3 antibody or antigen binding fragment thereof, an anti-GITR antibody, or antigen binding fragment thereof, an anti-TIGIT antibody, or antigen binding fragment thereof, an anti-CD27 antibody or antigen binding fragment thereof, an ILT2 antibody, or antigen binding fragment thereof, an ILT3 antibody, or antigen binding fragment thereof, an ILT4 antibody, or antigen binding fragment thereof, an ILT5 antibody, or antigen binding fragment thereof, or an IL-10 antibody, or antigen binding fragment thereof. In one embodiment, the additional therapeutic agent is a Newcastle disease viral vector expressing IL-12. In a further embodiment, the additional therapeutic agent is dinaciclib. In still further embodiments, the additional therapeutic agent is a STING agonist. In a further embodiment, the additional therapeutic agent is dinaciclib. In still further embodiments, the additional therapeutic agent is a PARP inhibitor. In a further embodiment, the additional therapeutic agent is dinaciclib. In additional embodiments, the additional therapeutic agent is a MEK inhibitor. In additional embodiments, the additional therapeutic agent is a CXCR2 antagonist. In additional embodiments, the additional therapeutic agent is navarixin. In additional embodiments, the additional therapeutic agent is olarparib. In additional embodiments, the additional therapeutic agent is selumetinib.

Suitable routes of administration may, for example, include parenteral delivery, including intramuscular, subcutaneous, as well as intrathecal, direct intraventricular, intravenous, intraperitoneal. Drugs can be administered in a variety of conventional ways, such as intraperitoneal, parenteral, intraarterial or intravenous injection.

Selecting a dosage of the additional therapeutic agent depends on several factors, including the serum or tissue turnover rate of the entity, the level of symptoms, the immunogenicity of the entity, and the accessibility of the target cells, tissue or organ in the individual being treated. The dosage of the additional therapeutic agent should be an amount that provides an acceptable level of side effects. Accordingly, the dose amount and dosing frequency of each additional therapeutic agent (e.g. biotherapeutic or chemotherapeutic agent) will depend in part on the particular therapeutic agent, the severity of the cancer being treated, and patient characteristics. Guidance in selecting appropriate doses of antibodies, cytokines, and small molecules are available. *See, e.g.,* Wawrzynczak (1996) Antibody Therapy, Bios Scientific Pub. Ltd, Oxfordshire, UK; Kresina (ed.) (1991) Monoclonal Antibodies, Cytokines and Arthritis, Marcel Dekker, New York, NY; Bach (ed.) (1993) Monoclonal Antibodies and Peptide Therapy in Autoimmune Diseases, Marcel Dekker, New York, NY; Baert et al. (2003) New Engl. J. Med. 348:601-608; Milgrom et al. (1999) New Engl. J. Med. 341:1966-1973; Slamon et al. (2001) New Engl. J. Med. 344:783-792; Beniaminovitz et al. (2000) New Engl. J. Med. 342:613-619; Ghosh et al. (2003) New Engl. J. Med. 348:24-32; Lipsky et al. (2000) New Engl. J. Med. 343:1594-1602; Physicians' Desk Reference 2003 (Physicians' Desk Reference, 57th Ed); Medical Economics Company; ISBN: 1563634457; 57th edition (November 2002). Determination of the appropriate dosage regimen may be made by the clinician, e.g., using parameters or factors known or suspected in the art to affect treatment or predicted to affect treatment, and will depend, for example, the patient's clinical history (e.g., previous therapy), the type and stage of the cancer to be treated and biomarkers of response to one or more of the therapeutic agents in the combination therapy.

Various literature references are available to facilitate selection of pharmaceutically acceptable carriers or excipients for the additional therapeutic agent. *See, e.g.,* Remington's Pharmaceutical Sciences and U.S. Pharmacopeia: National Formulary, Mack Publishing Company, Easton, PA (1984); Hardman et al. (2001) Goodman and Gilman's The Pharmacological Basis of Therapeutics, McGraw-Hill, New York, NY; Gennaro (2000) Remington: The Science and Practice of Pharmacy, Lippincott, Williams, and Wilkins, New York, NY; Avis et al. (eds.) (1993) Pharmaceutical Dosage Forms: Parenteral Medications, Marcel Dekker, NY; Lieberman, et al. (eds.) (1990) Pharmaceutical Dosage Forms: Tablets, Marcel Dekker, NY; Lieberman et al. (eds.) (1990) Pharmaceutical Dosage Forms: Disperse Systems, Marcel Dekker, NY; Weiner and Kotkoskie (2000) Excipient Toxicity and Safety, Marcel Dekker, Inc., New York, NY.

In some embodiments, a composition of the invention is administered by continuous infusion, or by doses at intervals of, *e.g.,* one day, 1-7 times per week, one week, two weeks, three weeks, monthly, bimonthly, etc. A preferred dose protocol is one involving the maximal dose or dose frequency that avoids significant undesirable side effects. A total weekly dose is generally at least 0.05 µg/kg, 0.2 µg/kg, 0.5 µg/kg, 1 µg/kg, 10 µg/kg, 100 µg/kg, 0.2 mg/kg, 1.0 mg/kg, 2.0 mg/kg, 10 mg/kg, 25 mg/kg, 50 mg/kg body weight or more. *See, e.g.,* Yang et al. (2003) New Engl. J. Med. 349:427-434; Herold et al. (2002) New Engl. J. Med. 346:1692-1698; Liu et al. (1999) J. Neurol. Neurosurg. Psych. 67:451-456; Portielji et al. (20003) Cancer Immunol. Immunother. 52:133-144. The desired dose of a small molecule therapeutic, *e.g*., a peptide mimetic, natural product, or organic chemical, is about the same as for an antibody or polypeptide, on a moles/kg basis.

In certain embodiments, dosing will comprise administering to a subject escalating doses of 1.0, 3.0, and 10 mg/kg of the pharmaceutical formulation, *i.e*, a formulation comprising an ABI and a compound of Formula I, over the course of treatment. The formulation can be a reconstituted liquid formulation, or it can be a liquid formulation not previously lyophilized. Time courses can vary, and can continue as long as desired effects are obtained. In certain embodiments, dose escalation will continue up to a dose of about 10mg/kg. In certain embodiments, the subject will have a histological or cytological diagnosis of melanoma, or other form of solid tumor, and in certain instances, a subject may have non-measurable disease. In certain embodiments, the subject will have been treated with other chemotherapeutics, while in other embodiments, the subject will be treatment naive.

In yet additional embodiments, the dosing regimen will comprise administering a dose of 1, 3, or 10 mg/kg of any of the pharmaceutical formulations described herein (*i.e,* a formulation comprising an ABI and a compound of Formula I), throughout the course of treatment. For such a constant dosing regimen, the interval between doses will be about 14 days (± 2 days). In certain embodiments, the interval between doses will be about 21 days (± 2 days).

In certain embodiments, the dosing regimen will comprise administering a dose of from about 0.005mg/kg to about 10mg/kg, with intra-patient dose escalation. In certain embodiments, a dose of 5 mg/kg or 10 mg/kg will be administered at intervals of every 3 weeks, or every 2 weeks. In yet additional embodiments, a dose of 3mg/kg will be administered at three week intervals for melanoma patients or patients with other solid tumors. In these embodiments, patients should have non-resectable disease; however, patients may have had previous surgery.

In certain embodiments, a subject will be administered a 30 minute IV infusion of any of the pharmaceutical formulations described herein. In certain embodiments for the escalating dose, the dosing interval will be about 28 days ((± 1 day) between the first and second dose. In certain embodiments, the interval between the second and third doses will be about 14 days (± 2 days). In certain embodiments, the dosing interval will be about 14 days (± 2 days), for doses subsequent to the second dose.

Subcutaneous administration may performed by injected using a syringe, or using other injection devices (e.g. the Inject-ease^{®} device); injector pens; or needleless devices (e.g. MediJector and BioJector^{®}).

Embodiments of the invention also include one or more of the biological formulations described herein (i) for use in, (ii) for use as a medicament or composition for, or (iii) for use in the preparation of a medicament for: (a) therapy (e.g., of the human body); (b) medicine; (c) induction of or increasing of an antitumor immune response (d) decreasing the number of one or more tumor markers in a patient; (e) halting or delaying the growth of a tumor or a blood cancer; (f) halting or delaying the progression of PD-1-related disease; (g) halting or delaying the progression cancer; (h) stabilization of PD-1-related disease; (i) inhibiting the growth or survival of tumor cells; (j) eliminating or reducing the size of one or more cancerous lesions or tumors; (k) reduction of the progression, onset or severity of PD-1-related disease; (l) reducing the severity or duration of the clinical symptoms of PD-1-related disease such as cancer (m) prolonging the survival of a patient relative to the expected survival in a similar untreated patient n) inducing complete or partial remission of a cancerous condition or other PD-1 related disease, (o) treatment of cancer, or (p) treatment of infection or infectious disease.

Some publications mentioned herein are describing and disclosing methodologies and materials that might be used in connection with the present invention.

Having described different embodiments of the invention herein with reference to the accompanying drawings, it is to be understood that the invention is not limited to those precise embodiments.

### EXAMPLE 1

### Preparation of Compound 1 (C1): (S)-12-((1H-imidazol-4-yl)methyl)-10-oxo-2,5,8-trioxa-11-azatridecan-13-oic acid

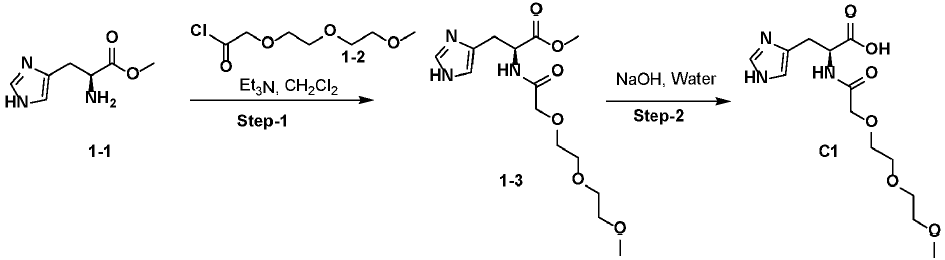

Step 1. Preparation of (S)-methyl 12-((1H-imidazol-4-yl)methyl)-10-oxo-2,5,8-trioxa-11-azatridecan-13-oate:
   To a solution of (S)-methyl 2-amino-3-(1H-imidazol-4-yl)propanoate (**1-1**) (5g, 29.6 mmol) in dichloromethane (25 ml) and triethyl amine (8.97, 89 mmol), was added 2-(2-(2-methoxyethoxy)ethoxy)acetyl chloride (**1-2**) (5.81 g, 29.6 mmol) (freshly prepared from acid) in dichloromethane (25 ml) dropwise at 0°C. The reaction mixture was stirred for 3 hours at room temperature. Reaction completion was confirmed by TLC. Saturated sodium bicarbonate was added to the reaction mixture, which was then concentrated to afford the crude product. Crude product was purified by flash column chromatography on silica gel eluting with 10 % methanol in dichloromethane to yield (S)-methyl 12-((1H-imidazol-4-yl)methyl)-10-oxo-2,5,8-trioxa-11-azatridecan-13-oate (**1-3**) as a liquid. LC-MS ESI/APCI calc'd. for C₁₄H₂₃N₃O₆ [M + 1]⁺ 330.35, found 330.2. ¹H NMR 400 MHz, DMSO-d6: δ 8.15 (d, J = 10.40 Hz, 1H), 7.63 (s, 1H), 6.86 (s, 1H), 4.58 (t, J = 9.20 Hz, 1H), 3.92 (s, 2H), 3.65 (s, 3H), 3.58 (s, 3H), 3.50-3.52 (m, 8H), 2.97 (d, J = 9.20 Hz, 2H).
Step 2. Preparation of (S)-12-((1H-imidazol-4-yl)methyl)-10-oxo-2,5,8-trioxa-11-azatridecan-13-oic acid:
   To a stirred solution of (S)-methyl 12-((1H-imidazol-4-yl)methyl)-10-oxo-2,5,8-trioxa-11-azatridecan-13-oate (**1-3**) (4.7 g, 14.2 mmol) in MeOH (50.0 mL) and water (10.0 mL) was added sodium hydroxide (0.85 g, 21.3 mmol). The reaction mixture was stirred at room temperature for 5 hours. Reaction completion was confirmed by TLC. 1.5 N HCl was added to the reaction mixture and it was concentrated under reduced pressure. The crude residue was dissolved in methanol and filtered through a CELITE bed and the filtrate was again concentrated. Crude product was first purified by flash column chromatography on silica gel eluting with 5% ammonia in methanol and again purified by reverse phase chromatography eluting with water to yield (S)-12-((1H-imidazol-4-yl)methyl)-10-oxo-2,5,8-trioxa-11-azatridecan-13-oic acid (**C1**) as a solid. LC-MS ESEAPCI calc'd. for C₁₃H₂₁N₃O₆ [M + H]⁺ 316.33, found 316.4. ¹H NMR 400 MHz, D₂O: δ 8.22 (s, 1H), 7.06 (s, 1H), 4.41 (t, J = 4.80 Hz, 1H), 3.94 (s, 2H), 3.52-3.54 (m, 8H), 3.26 (s, 3H), 3.16 (q, J = 4.80 Hz, 1H), 2.98 (q, J = 8.40 Hz, 1H).

### EXAMPLE 2

### Preparation of Compound 2 (C2): (S)-12-(3-guanidinopropyl)-10-oxo-2,5,8-trioxa-11-azatridecan-13-oic acid

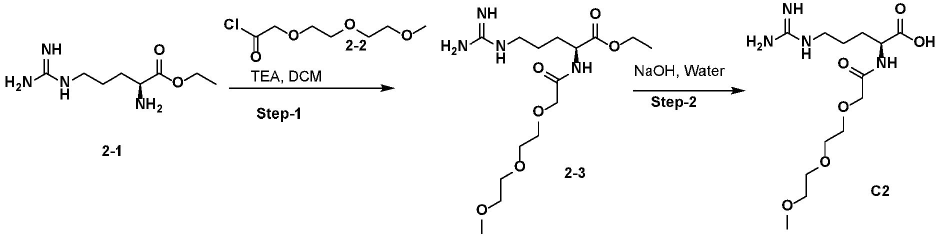

Step 1. Preparation of (S)-ethyl 12-(3-guanidinopropyl)-10-oxo-2,5,8-trioxa-11-azatridecan-13-oate:
   To a solution of (S)-ethyl 2-amino-5-guanidinopentanoate (**2-1**) (5g, 24.72 mmol) in dichloromethane (25 ml) and triethylamine (10.34 ml, 74.2 mmol), was added 2-(2-(2-methoxyethoxy)ethoxy)acetyl chloride (**2-2**) (4.86 g, 24.72 mmol) (freshly prepared from acid) in dichloromethane (25 ml) dropwise at 0°C. The reaction mixture was stirred for 3 hours at room temperature. Reaction completion was confirmed by TLC. Saturated sodium bicarbonate was then added to the reaction mixture, which was concentrated to afford the crude product. Crude product was purified by flash column chromatography on silica gel eluting with 10 % methanol in dichloromethane to afford (S)-ethyl 12-(3-guanidinopropyl)-10-oxo-2,5,8-trioxa-11-azatridecan-13-oate (**2-3**) as a liquid. LC-MS ESI/APCI calc'd. for C₁₅H₃₀N₄O₆ [M + 1]⁺ 363.42, found 363.2. ¹H NMR 400 MHz, DMSO-d₆: δ 8.01 (s, 1H), 7.83 (s, 1H), 4.26 (t, J = 8.00 Hz, 1H), 4.14 (q, J = 1.20 Hz, 2H), 4.09-4.10 (m, 2H), 3.95 (s, 2H), 3.53-3.54 (m, 8H), 3.43 (s, 3H), 1.87-1.84 (m, 2H), 1.46-1.48 (m, 2H), 1.19 (t, J = 9.60 Hz, 3H).
Step 2. Preparation of (S)-12-(3-guanidinopropyl)-10-oxo-2,5,8-trioxa-11-azatridecan-13-oic acid:
   To a stirred solution of (S)-ethyl 12-(3-guanidinopropyl)-10-oxo-2,5,8-trioxa-11-azatridecan-13-oate (**2-3**) (4g, 11.04 mmol) in methanol (50 ml) and water (10 ml) was added sodium hydroxide (0.662 g, 16.56 mmol). The reaction mixture was stirred at room temperature for 5 hours. Reaction completion was confirmed by TLC. The reaction mixture was neutralized with 1.5N HCl and concentrated under reduced pressure. The crude residue was dissolved in methanol and filtered through a CELITE bed and the filtrate was again concentrated. Crude product was first purified by flash column chromatography on silica gel eluting with 5% ammonia in methanol and again purified by reverse phase chromatography, eluting with water to afford (S)-12-(3-guanidinopropyl)-10-oxo-2,5,8-trioxa-11-azatridecan-13-oic acid (**C2**) as a solid. LC-MS ESI/APCI calc'd. for C₁₃H₂₆N₄O₆ [M + H]⁺ 335.19, found 335.2. ¹H NMR 400 MHz, D₂O: δ 4.14-4.16 (m, 1H), 4.01 (s, 2H), 3.60-3.61 (m, 6H), 3.53-3.53 (m, 2H), 3.30 (s, 3H), 3.11 (t, J = 6.36 Hz, 2H), 1.79-1.81 (m, 1H), 1.66-1.68 (m, 1H), 1.51-1.52 (m, 2H).

### EXAMPLE 3

### Preparation of Compound 3 (C3): (S)-5-guanidino-2-(2-(2-methoxyethoxy)-N-(2-(2-methoxyethoxy)ethyl)acetamido)pentanoic acid

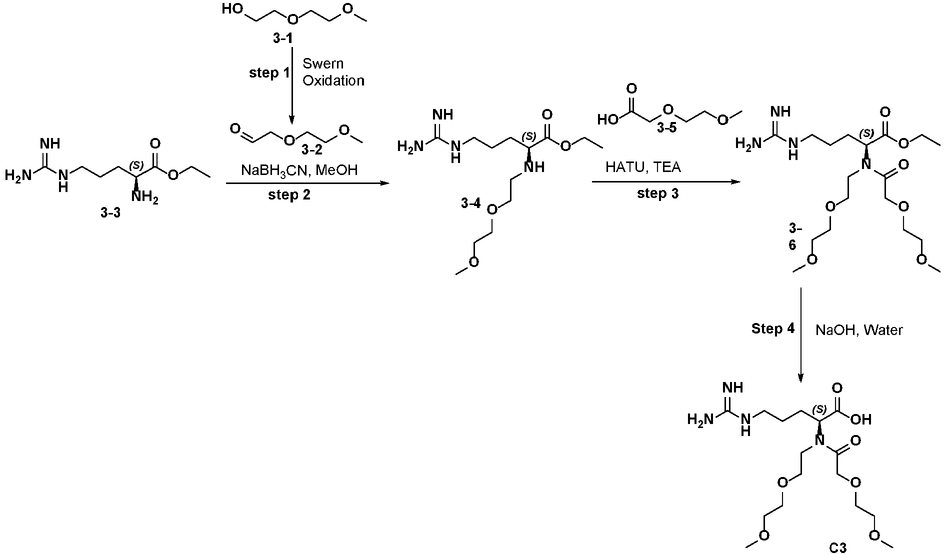

The various starting materials and reagents used in the schemes are commercially available or are readily made by persons skilled in the art. Compound **3-2** was synthesized following the literature protocol disclosed in Seifert et al. J. Med. Chem. 2014, 57, 9870-9888.
Step 2. Preparation of (S)-ethyl 5-guanidino-2-((2-(2-methoxyethoxy)ethyl)amino)pentanoate:
   To a solution (S)-ethyl 2-amino-5-guanidinopentanoate (**3-3**) (30.0 g, 148 mmol) in MeOH (300.0 mL) was added 2-(2-methoxyethoxy) acetaldehyde (**3-2**) (26.2 g, 222 mmol) and acetic acid (1.69 ml, 29.6 mmol). The reaction mixture was stirred at room temperature for 15 minutes. The reaction mixture was cooled to 0°C and sodium cyanoborohydride (13.8 g, 222 mmol) was added portion wise. The reaction mixture was stirred for 6 hours at room temperature. Reaction completion was confirmed by TLC. Saturated sodium bicarbonate was then added to the reaction mixture and concentrated to afford the crude product. Crude product was purified by flash column chromatography on silica gel, eluting with 7% methanol to afford (S)-ethyl 5-guanidino-2-((2-(2-methoxyethoxy)ethyl)amino)pentanoate (**3-4**). LC-MS ESI/APCI calc'd. for C₁₃H₂₈N₄O₄ [M + H]⁺ 305.3, found 305.2.
Step 3. Preparation of (S)-ethyl 5-guanidino-2-(2-(2-methoxyethoxy)-N-(2-(2-methoxyethoxy)ethyl)acetamido)pentanoate:
   HATU (25.5 g, 67.1 mmol), triethylamine (12.47 ml, 89 mmol), (S)-ethyl 5-guanidino-2-((2-(2-methoxyethoxy)ethyl)amino)pentanoate (**3-4**) (17.70 g, 58.2 mmol) in DMF (100 ml) were added to a solution of 2-(2-methoxyethoxy)acetic acid (**3-5**) (6g, 44.7 mmol) in DMF(30ml). The reaction mixture was stirred for 5 hours at room temperature. Reaction completion was confirmed by TLC. Saturated sodium bicarbonate was then added to the reaction mixture and the product was extracted with dichloromethane (5 X 100 mL). The combined organic layer was washed with brine and dried over anhydrous sodium sulfate and concentrated under reduced pressure. Crude product was purified by flash column chromatography on silica gel eluting with 10 % methanol in dichloromethane to afford (S)-ethyl 5-guanidino-2-(2-(2-methoxyethoxy)-N-(2-(2-methoxyethoxy)ethyl)acetamido)pentanoate (**3-6**) as a solid. LC-MS ESEAPCI calc'd. for C₁₈H₃₆N₄O₇ [M + H]⁺ 421.5, found 421.2.
Step 4. Preparation of (S)-5-guanidino-2-(2-(2-methoxyethoxy)-N-(2-(2-methoxyethoxy)ethyl)acetamido)pentanoic acid:
   To a stirred solution of (S)-ethyl 5-guanidino-2-(2-(2-methoxyethoxy)-N-(2-(2-methoxyethoxy)ethyl)acetamido)pentanoate (3-6) (4g, 9.07 mmol) in MeOH (40.0 mL) and water (8.0 mL) was added sodium hydroxide (0.571 g, 14.27 mmol) and the reaction mixture was stirred at room temperature for 5 hours. Reaction completion was confirmed by TLC. The reaction mixture was neutralized with 1.5N HCl and concentrated under reduced pressure. The crude residue was dissolved in methanol and filtered through a CELITE bed and the filtrate was again concentrated. Crude product was first purified by flash column chromatography on silica gel eluting with 10% ammonia in methanol and again purified by reverse phase chromatography eluting with water to yield (S)-5-guanidino-2-(2-(2-methoxyethoxy)-N-(2-(2-methoxyethoxy)ethyl)acetamido)pentanoic acid (**C3**) as a solid. LC-MS ESEAPCI calc'd. for C₁₆H₃₂N₄O₇ [M + H]⁺ 393.44, found 393.4. ¹H NMR 400 MHz, D₂O: δ 4.33-4.31 (m, 1H), 4.24 (s, 2H), 3.58-3.62 (m, 12H), 3.23 (s, 3H), 3.28 (s, 3H), 3.09-3.11 (m, 2H), 1.89-1.92 (m, 1H), 1.64-1.67 (m, 1H), 1.47-1.48 (m, 2H).

### EXAMPLE 4

### Preparation of Compound 4 (C4): (S)-3-(1H-imidazol-4-yl)-2-(2-(2-methoxyethoxy)-N-(2-(2-methoxyethoxy)ethyl)acetamido)propanoic acid

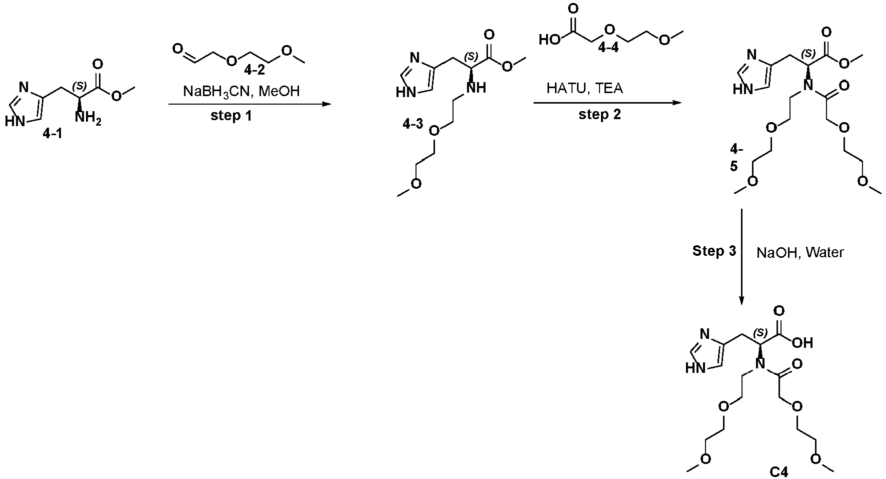

Step 1. Preparation of (S)-methyl 3-(1H-imidazol-4-yl)-2-((2-(2-methoxyethoxy)ethyl)amino)propanoate:
   To a solution (S)-methyl 2-amino-3-(1H-imidazol-4-yl)propanoate (**4-1**) (15 g, 89 mmol) in methanol (150 ml) was added 2-(2-methoxyethoxy)acetaldehyde (**4-2**) (15.6 g, 133 mmol) and acetic acid (1.01 ml, 17.8 mmol). The reaction mixture was stirred at room temperature for 15 minutes. The reaction mixture was cooled to 0°C and sodium cyanoborohydride (8.36 g, 133 mmol) was added portionwise. The reaction mixture was stirred for 6 hours at room temperature. Reaction completion was confirmed by TLC. Saturated sodium bicarbonate was then added to the reaction mixture and concentrated to afford the crude product. Crude product was purified by flash column chromatography on silica gel eluting with 7% methanol to afford (S)-methyl 3-(1H-imidazol-4-yl)-2-((2-(2-methoxyethoxy)ethyl)amino)propanoate (**4-3**). LC-MS ESI/APCI calc'd. for C₁₂H₂₁N₃O₄ [M + H]⁺ 272.3, found 272.2.
Step 2. Preparation of (S)-methyl 3-(1H-imidazol-4-yl)-2-(2-(2-methoxyethoxy)-N-(2-(2-methoxyethoxy)ethyl)acetamido)propanoate:
   To a solution of 2-(2-methoxyethoxy)acetic acid (4-4) (4.2g, 31.3 mmol) in dichloromethane (40ml), HATU (17.86 g, 47.0 mmol), triethylamine (8.80 ml, 62.6 mmol) and (S)-methyl 3-(1H-imidazol-4-yl)-2-((2-(2-methoxyethoxy)ethyl)amino)propanoate (**4-3**) (8.5 g, 31.3 mmol) in dichloromethane( 60 mL) were added. The reaction mixture was stirred for 5 hours at room temperature. Reaction completion was confirmed by TLC. Saturated sodium bicarbonate was then added to the reaction mixture and product was extracted with dichloromethane (5 X 100 mL). Then combined organic layer was washed with brine and dried over anhydrous sodium sulfate and concentrated under reduced pressure. Crude product was purified by flash column chromatography on silica gel eluting with 5 % methanol in dichloromethane to afford (S)-methyl 3-(1H-imidazol-4-yl)-2-(2-(2-methoxyethoxy)-N-(2-(2-methoxyethoxy)ethyl)acetamido)propanoate (**4-4**) as a solid. LC-MS ESI/APCI calc'd. for C₁₇H₂₉N₃O₇ [M + H]⁺ 388.42, found 388.2.
Step 4. Preparation of (S)-3-(1H-imidazol-4-yl)-2-(2-(2-methoxyethoxy)-N-(2-(2-methoxyethoxy)ethyl)acetamido)propanoic acid:
   To a stirred solution of (S)-methyl 3-(1H-imidazol-4-yl)-2-(2-(2-methoxyethoxy)-N-(2-(2-methoxyethoxy)ethyl)acetamido)propanoate (**4-5**) (7.1 g, 15.21 mmol) in MeOH (70.0 mL) and water (15.0 mL) was added sodium hydroxide (1.08 g, 27.1 mmol) and the reaction mixture was stirred at room temperature for 5 hours. Reaction completion was confirmed by TLC. The reaction mixture was neutralized with 1.5 N HCl and concentrated under reduced pressure. The crude residue was dissolved in methanol and filtered through a CELITE bed and the filtrate was again concentrated. Crude product was first purified by flash column chromatography on silica gel eluting with 10% ammonia in methanol and again purified by reverse phase chromatography eluting with water to yield (S)-3-(1H-imidazol-4-yl)-2-(2-(2-methoxyethoxy)-N-(2-(2-methoxyethoxy)ethyl)acetamido)propanoic acid (**C4**) as a solid. LC-MS ESEAPCI calc'd. for C₁₆H₂₇N₃O₇ [M + H]⁺ 374.44, found 374.2. ¹H NMR 400 MHz, D₂O: δ 8.49 (s, 1H), 7.15 (s, 1H), 4.35-4.36 (m, 1H), 4.27 (s, 2H), 3.48-3.50 (m, 12H), 3.29-3.27 (m, 2H), 3.29 (s, 3H), 3.25 (s, 3H).

### EXAMPLE 5

### Preparation of Compound 5 (C5): (S)-2-(bis(2-(2-methoxyethoxy)ethyl)amino)-5-guanidinopentanoic acid

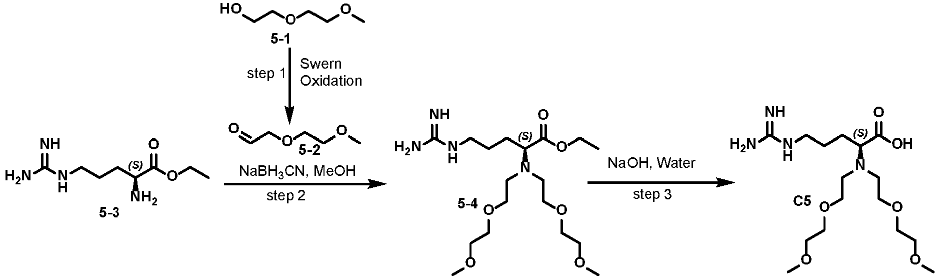

Step 2. Preparation of (S)-ethyl 2-(bis(2-(2-methoxyethoxy)ethyl)amino)-5-guanidinopentanoate:
   To a solution (S)-ethyl 2-amino-5-guanidinopentanoate (**5-3**) (10.0 g, 49.5 mmol) in MeOH (100.0 mL), was added 2-(2-methoxyethoxy)acetaldehyde (**5-2**) (23.3 g, 198 mmol) and acetic acid (0.565 ml, 9.9 mmol). The reaction mixture was stirred at room temperature for 15 minutes. The reaction mixture was cooled to 0°C and sodium cyanoborohydride (4.66 g, 74.2 mmol) was added portion wise and the reaction mixture was stirred for 6 hours at room temperature. Reaction completion was confirmed by TLC. Saturated sodium bicarbonate was then added to the reaction mixture and product was extracted with dichloromethane (5 X 100 mL). Then combined organic layer was washed with brine and dried over anhydrous sodium sulfate and concentrated under reduced pressure. Crude product was purified by flash column chromatography on silica gel eluting with 10 % methanol in dichloromethane to yield (S)-ethyl 2-(bis(2-(2-methoxyethoxy)ethyl)amino)-5-guanidinopentanoate (**5-4**). LC-MS ESI/APCI calc'd. for C₁₈H₃₈N₄O₆ [M + 1]⁺ 406.28, found 407.4. ¹H NMR (400 MHz, DMSO): δ 7.42 (bs, 1H), 7.25 (bs, 1H), 6.85 (bs, 2H), 4.12-4.05 (m, 2H), 3.49-3.47 (m, 4H), 3.43-3.40 (m, 8H), 3.38-3.36 (m, 1H), 3.24 (s, 6H), 3.11-3.09 (m, 2H), 2.80-2.66 (m, 4H), 1.63-1.60 (m, 2H), 1.50-1.43 (m, 2H), 1.20 (t, J = 7.2 Hz, 3H).
Step 3. Preparation of (S)-2-(bis(2-(2-methoxyethoxy)ethyl)amino)-5-guanidinopentanoic acid:
   To a stirred solution of (S)-ethyl 2-(bis(2-(2-methoxyethoxy)ethyl)amino)-5-guanidinopentanoate (**5-4**) (5.4 g, 13.3 mmol) in MeOH (60.0 mL) and water (10.0 mL) was added sodium hydroxide (0.797 g, 19.9mmol) and the reaction mixture was stirred at room temperature for 5 h. Reaction completion was confirmed by TLC. 1.5 N HCl was added to the reaction mixture and concentrated under reduced pressure. The crude residue was dissolved in methanol and filtered through a CELITE bed and the filtrate was again concentrated. Crude product was first purified by flash column chromatography on silica gel eluting with 10% ammonia in methanol and again purified by reverse phase chromatography eluting with water to yield (S)-2-(bis(2-(2-methoxyethoxy)ethyl)amino)-5-guanidinopentanoic acid (**C5**) as a solid. LC-MS ESI/APCI calc'd. for C₁₆H₃₄N₄O₆ [M + H]⁺ 379.4, found 379.0. ¹H NMR (400 MHz, D₂O): δ 3.86-3.66 (m, 4H), 3.86-3.76 (m, 1H), 3.62-3.60 (m, 4H), 3.57-3.55 (m, 4H), 3.47-3.39 (m, 4H), 3.29 (s, 6H), 3.17 (t, J = 6.8 Hz, 2H), 1.86-1.80 (m, 2H), 1.74-1.62 (m, 2H).

### EXAMPLE 6

### Preparation of Compound 6 (C6): (S)-2-(bis(2-(2-methoxyethoxy)ethyl)amino)-3-(1H-imidazol-4-yl)propanoic acid

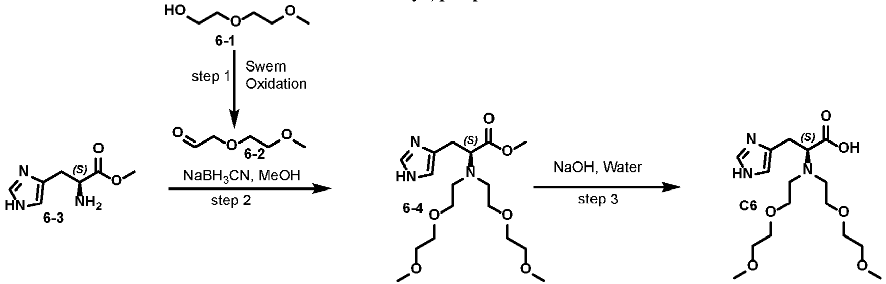

Step 2. Preparation of (S)-methyl 2-(bis(2-(2-methoxyethoxy)ethyl)amino)-3-(1H-imidazol-4-yl)propanoate:
   To a solution (S)-methyl 2-amino-3-(1H-imidazol-4-yl)propanoate (**6-3**) (10g, 59.1 mmol) in MeOH (100.0 mL), was added 2-(2-methoxyethoxy)acetaldehyde (**6-2**) (27.9 g, 236 mmol) and acetic acid (0.67 ml, 11.82 mmol). The reaction mixture was stirred at room temperature for 15 minutes. The reaction mixture was cooled to 0°C and sodium cyanoborohydride (5.57 g, 89 mmol) was added portionwise. The reaction mixture was stirred for 6 hours at room temperature. Reaction completion was confirmed by TLC. Saturated sodium bicarbonate was then added to the reaction mixture and the product was extracted with dichloromethane (5 X 100 mL). The combined organic layer was washed with brine and dried over anhydrous sodium sulfate and concentrated under reduced pressure. Crude product was purified by flash column chromatography on silica gel eluting with 7 % methanol in dichloromethane to yield (S)-methyl 2-(bis(2-(2-methoxyethoxy)ethyl)amino)-3-(1H-imidazol-4-yl)propanoate (**6-4**) as a solid. LC-MS ESI/APCI calc'd. for C₁₇H₃₁N₃O₆ [M + 1]⁺ 374.44, found 374.2 .
Step 3. Preparation of (S)-2-(bis(2-(2-methoxyethoxy)ethyl)amino)-3-(1H-imidazol-4-yl)propanoic acid:
   To a stirred solution of (S)-methyl 2-(bis(2-(2-methoxyethoxy)ethyl)amino)-3-(1H-imidazol-4-yl)propanoate (**6-4**) (6.5g, 17.30 mmol) in MeOH (65.0 mL) and water (12.0 mL) was added sodium hydroxide (1.03 g, 25.95 mmol). The reaction mixture was stirred at room temperature for 5 hours. Reaction completion was confirmed by TLC. The reaction mixture was neutralized with 1.5 N HCl and concentrated under reduced pressure. The crude residue was dissolved in methanol and filtered through a CELITE bed and the filtrate was again concentrated. Crude product was first purified by flash column chromatography on silica gel eluting with 10% ammonia in methanol and again purified by reverse phase chromatography eluting with water to yield (S)-2-(bis(2-(2-methoxyethoxy)ethyl)amino)-3-(1H-imidazol-4-yl)propanoic acid (**C6**) as a solid. LC-MS ESI/APCI calc'd. for C₁₆H₂₉N₃O₆ [M + H]⁺ 360.41, found 360.2. ¹H NMR (400 MHz, D₂O): 400 MHz, D₂O: δ 8.22 (s, 1H), 7.15 (s, 1H), 3.90 (t, J = 7.20 Hz, 1H), 3.60-3.62 (m, 4H), 3.55-3.56 (m, 4H), 3.50-3.50 (m, 4H), 3.28 (s, 6H), 3.19-3.19 (m, 4H), 3.06-3.07 (m, 2H).

### EXAMPLE 7

### Preparation of Compound 7 (C7): (S)-3-(1H-imidazol-4-yl)-2-(2-(2-methoxyethoxy)-N-(2-(2-methoxyethoxy)ethyl)acetamido)propanoic acid hydrochloride

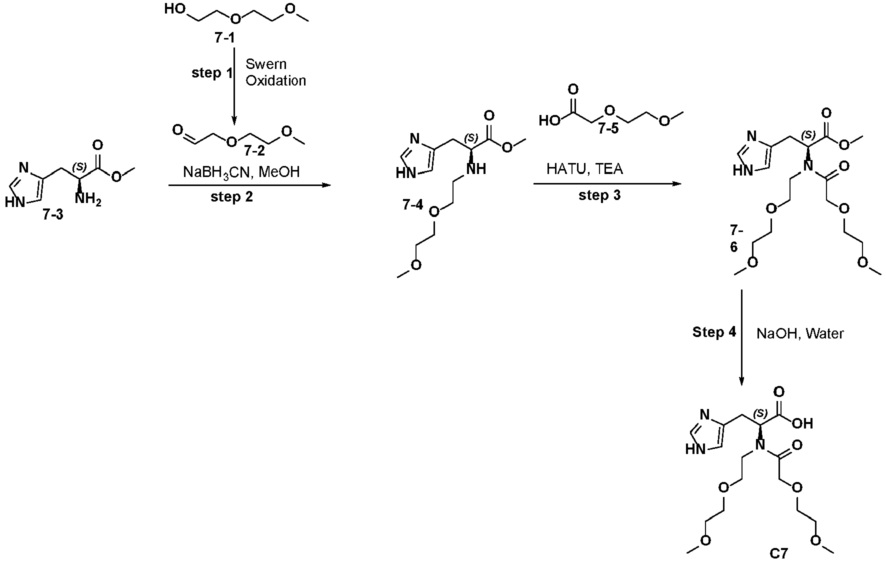

Step 2. Preparation of (S)-methyl 3-(1H-imidazol-4-yl)-2-((2-(2-methoxyethoxy)ethyl)amino)propanoate:
   To a solution (S)-methyl 2-amino-3-(1H-imidazol-4-yl)propanoate (**7-3**)(15 g, 89 mmol) in methanol (150 ml) was added 2-(2-methoxyethoxy)acetaldehyde (**7-2**) (15.6 g, 133 mmol) and acetic acid (1.01 ml, 17.8 mmol). The reaction mixture was stirred at room temperature for 15 minutes. The reaction mixture was cooled to 0°C and sodium cyanoborohydride (8.36 g, 133 mmol) was added portionwise. The reaction mixture was stirred for 6 hours at room temperature. Reaction completion was confirmed by TLC. Saturated sodium bicarbonate was then added to the reaction mixture and concentrated to afford the crude product. Crude product was purified by flash column chromatography on silica gel eluting with 7% methanol in to afford (S)-methyl 3-(1H-imidazol-4-yl)-2-((2-(2-methoxyethoxy)ethyl)amino)propanoate (**7-4**). LC-MS ESI/APCI calc'd. for C₁₂H₂₁N₃O₄ [M + H]⁺ 272.3, found 272.2.
Step 3. Preparation of (S)-methyl 3-(1H-imidazol-4-yl)-2-(2-(2-methoxyethoxy)-N-(2-(2-methoxyethoxy)ethyl)acetamido)propanoate:
   To a solution of 2-(2-methoxyethoxy)acetic acid (**7-5**) (4.2g, 31.3 mmol) in dichloromethane (40ml), HATU (17.86 g, 47.0 mmol),triethylamine (8.80 ml, 62.6 mmol) and (S)-methyl 3-(1H-imidazol-4-yl)-2-((2-(2-methoxyethoxy)ethyl)amino)propanoate (**7-4**) (8.5 g, 31.3 mmol) in dichloromethane( 60ml) were added. The reaction mixture was stirred for 5 hours at room temperature. Reaction completion was confirmed by TLC. Saturated sodium bicarbonate was then added to the reaction mixture and the product was extracted with dichloromethane (5 X 100 mL). Then combined organic layer was washed with brine and dried over anhydrous sodium sulfate and concentrated under reduced pressure. Crude product was purified by flash column chromatography on silica gel eluting with 5 % methanol in dichloromethane to afford (S)-methyl 3-(1H-imidazol-4-yl)-2-(2-(2-methoxyethoxy)-N-(2-(2-methoxyethoxy)ethyl)acetamido)propanoate (**7-6**) as a solid. LC-MS ESI/APCI calc'd. for C₁₇H₂₉N₃O₇ [M + H]⁺ 388.42, found 388.2.
Step 4. Preparation of (S)-3-(1H-imidazol-4-yl)-2-(2-(2-methoxyethoxy)-N-(2-(2-methoxyethoxy)ethyl)acetamido)propanoic acid hydrochloride:
   To a stirred solution of (S)-methyl 3-(1H-imidazol-4-yl)-2-(2-(2-methoxyethoxy)-N-(2-(2-methoxyethoxy)ethyl)acetamido)propanoate(7-6) (7.1 g, 18.34 mmol) in MeOH (70.0 mL) and water (15.0 mL) was added sodium hydroxide (1.1 g, 27.5 mmol). The reaction mixture was stirred at room temperature for 5 hours. Reaction completion was confirmed by TLC. The reaction mixture was acidified with 1.5 N HCl to pH 2-3 and concentrated under reduced pressure. Crude product was purified by reverse phase chromatography eluting with 0.1% HCl in water to afford (S)-3-(1H-imidazol-4-yl)-2-(2-(2-methoxyethoxy)-N-(2-(2-methoxyethoxy)ethyl)acetamido)propanoic acid hydrochloride (**C7**) as a solid. LC-MS ESI/APCI calc'd. for C₁₆H₂₇N₃O₇ [M + H]⁺ 374.44, found 374.2. ¹H NMR 400 MHz, D₂O: δ 8.52 (s, 1H), 7.22 (s, 1H), 4.32 (s, 2H), 4.28-4.29 (m, 1H), 3.54-3.54 (m, 12H), 3.43-3.43 (m, 2H), 3.30 (s, 3H), 3.25 (s, 3H).

### EXAMPLE 8

### Preparation of Compound 8 (C8): (S)-2-(bis(2-(2-methoxyethoxy)ethyl)amino)-5-guanidinopentanoic acid dihydrochloride

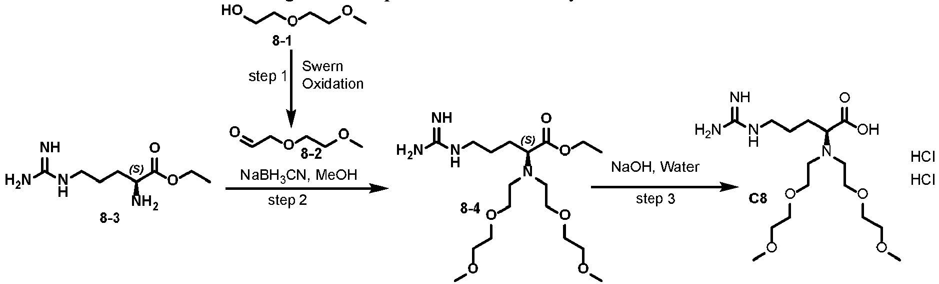

Step 2. Preparation of (S)-ethyl 2-(bis(2-(2-methoxyethoxy)ethyl)amino)-5-guanidinopentanoate:
   To a solution (S)-ethyl 2-amino-5-guanidinopentanoate (**8-3**) (10.0 g, 49.5 mmol) in MeOH (100.0 mL) was added 2-(2-methoxyethoxy)acetaldehyde (**8-2**) (23.3 g, 198 mmol) and acetic acid (0.565 ml, 9.9 mmol). The reaction mixture was stirred at room temperature for 15 minutes. The reaction mixture was cooled to 0°C and sodium cyanoborohydride (4.66 g, 74.2 mmol) was added portionwise. The reaction mixture was stirred for 6 hours at room temperature. Reaction completion was confirmed by TLC. Saturated sodium bicarbonate was then added to the reaction mixture and the product was extracted with dichloromethane (5 X 100 mL). The combined organic layer was washed with brine and dried over anhydrous sodium sulfate and concentrated under reduced pressure. Crude product was purified by flash column chromatography on silica gel eluting with 10 % methanol in dichloromethane to yield (S)-ethyl 2-(bis(2-(2-methoxyethoxy)ethyl)amino)-5-guanidinopentanoate (**8-4**). LC-MS ESI/APCI calc'd. for C₁₈H₃₈N₄O₆ [M + 1]⁺ 407.28, found 407.4. ¹H NMR (400 MHz, DMSO): δ 7.42 (bs, 1H), 7.25 (bs, 1H), 6.85 (bs, 2H), 4.12-4.05 (m, 2H), 3.49-3.47 (m, 4H), 3.43-3.40 (m, 8H), 3.38-3.36 (m, 1H), 3.24 (s, 6H), 3.11-3.09 (m, 2H), 2.80-2.66 (m, 4H), 1.63-1.60 (m, 2H), 1.50-1.43 (m, 2H), 1.20 (t, J = 7.2 Hz, 3H).
Step 3. Preparation of (S)-2-(bis(2-(2-methoxyethoxy)ethyl)amino)-5-guanidinopentanoic acid dihydrochloride:
   To a stirred solution of (S)-ethyl 2-(bis(2-(2-methoxyethoxy)ethyl)amino)-5-guanidinopentanoate (**8-4**) (5.4 g, 13.3 mmol) in MeOH (60.0 mL) and water (10.0 mL) was added sodium hydroxide (0.797 g, 19.9 mmol). The reaction mixture was stirred at room temperature for 5 hours. Reaction completion was confirmed by TLC. 1.5 N HCl was added to the reaction mixture and concentrated under reduced pressure. The crude residue was dissolved in methanol and filtered through a CELITE bed and the filtrate was again concentrated. The reaction mixture was acidified with 1.5 N HCl to pH 2-3 and concentrated under reduced pressure. Crude product was purified by reverse phase chromatography eluting with 0.1% HCl in water to afford (S)-2-(bis(2-(2-methoxyethoxy)ethyl)amino)-5-guanidinopentanoic acid dihydrochloride (**C8**) as a solid. LC-MS ESEAPCI calc'd. for C₁₆H₃₄N₄O₆ [M + H]⁺ 379.25, found 379.0. ¹H NMR (400 MHz, D₂O): δ 3.86-3.66 (m, 4H), 3.86-3.76 (m, 1H), 3.62-3.60 (m, 4H), 3.57-3.55 (m, 4H), 3.47-3.39 (m, 4H), 3.29 (s, 6H), 3.17 (t, J = 6.8 Hz, 2H), 1.86-1.80 (m, 2H), 1.74-1.62 (m, 2H).

### EXAMPLE 9

### Preparation of Compound 9 (C9): (S)-2-(bis(2-(2-methoxyethoxy)ethyl)amino)-3-(1H-imidazol-4-yl)propanoic acid dihydrochloride

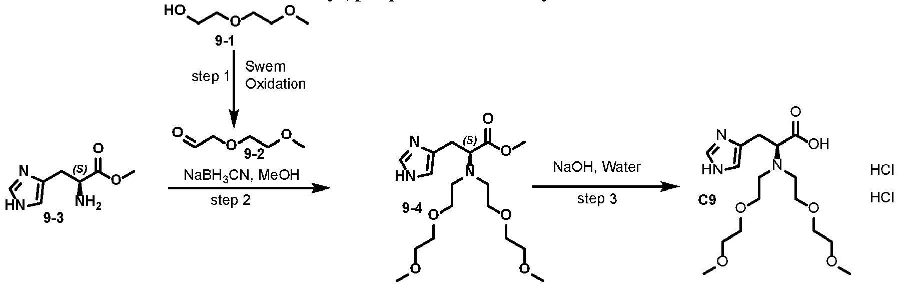

Step 2. Preparation of (S)-methyl 2-(bis(2-(2-methoxyethoxy)ethyl)amino)-3-(1H-imidazol-4-yl)propanoate:
   To a solution (S)-methyl 2-amino-3-(1H-imidazol-4-yl)propanoate (**9-3**) (10 g, 59.1 mmol) in MeOH (100.0 mL) was added 2-(2-methoxyethoxy)acetaldehyde (**9-2**) (27.9 g, 236 mmol) and acetic acid (0.67 ml, 11.82 mmol). The reaction mixture was stirred at room temperature for 15 minutes. The reaction mixture was cooled to 0°C and sodium cyanoborohydride (5.57 g, 89 mmol) was added portionwise. The reaction mixture was stirred for 6 hours at room temperature. Reaction completion was confirmed by TLC. Saturated sodium bicarbonate was then added to the reaction mixture and the product was extracted with dichloromethane (5 X 100 mL). The combined organic layer was washed with brine and dried over anhydrous sodium sulfate and concentrated under reduced pressure. Crude product was purified by flash column chromatography on silica gel eluting with 7 % methanol in dichloromethane to yield (S)-methyl 2-(bis(2-(2-methoxyethoxy)ethyl)amino)-3-(1H-imidazol-4-yl)propanoate (**9-4**) as a solid. LC-MS ESI/APCI calc'd. for C₁₇H₃₁N₃O₆ [M + 1]⁺ 374.44, found 374.2.
Step 3. Preparation of (S)-2-(bis(2-(2-methoxyethoxy)ethyl)amino)-3-(1H-imidazol-4-yl)propanoic acid dihydrochloride:
   To a stirred solution of (S)-methyl 2-(bis(2-(2-methoxyethoxy)ethyl)amino)-3-(1H-imidazol-4-yl)propanoate (**9-4**) (6.5g, 17.30 mmol) in MeOH (65.0 mL) and water (12.0 mL) was added sodium hydroxide (1.03 g, 25.95mmol) and the reaction mixture was stirred at room temperature for 5 hours. Reaction completion was confirmed by TLC. The reaction mixture was acidified with 1.5 N HCl to pH 2-3 and concentrated under reduced pressure. The residue was dissolved in methanol and filtered through a CELITE bed and the filtrate was again concentrated. Crude product was purified by reverse phase chromatography eluting with 0.1% HCl in water to afford (S)-2-(bis(2-(2-methoxyethoxy)ethyl)amino)-3-(1H-imidazol-4-yl)propanoic acid dihydrochloride (**C9**) as a solid. LC-MS ESI/APCI calc'd. for C₁₆H₂₉N₃O₆ [M + H]⁺ 360.41, found 360.2. ¹H NMR 400 MHz, D₂O: δ 8.58 (s, 1H), 7.34 (s, 1H), 4.43 (t, J = 4.60 Hz, 1H), 3.79-3.80 (m, 4H), 3.58-3.59 (m, 6H), 3.47-3.48 (m, 6H), 3.39-3.40 (m, 2H), 3.26 (s, 6H).

### EXAMPLE 10

### Preparation of Compound 10 (C10): (S)-6-amino-2-(2-(2-methoxyethoxy)acetamido)hexanoic acid

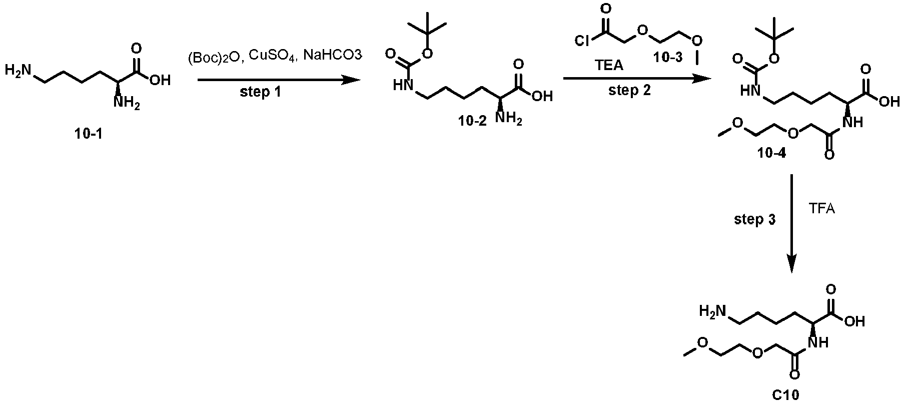

### Compound 10-2 was synthesized by following the protocol set forth in Journal of the American Chemical Society, 2015, vol. 137, 6975 - 6978

Step 2. Preparation of (S)-6-((tert-butoxycarbonyl)amino)-2-(2-(2-methoxyethoxy)acetamido)hexanoic acid:
   To a solution of (S)-2-amino-6-((tert-butoxycarbonyl)amino)hexanoic acid (10-2) (9.18 g, 37.3 mmol) in dichloromethane (50 ml) and triethylamine (11.3 g, 111 mmol) was added 2-(2-methoxyethoxy)acetyl chloride (10-3) (5.66 g, 37.3 mmol (freshly prepared from acid) in dichloromethane (50 ml) dropwise at 0°C. The reaction mixture was stirred for 3 hours at room temperature. Reaction completion was confirmed by TLC. Saturated sodium bicarbonate was then added to the reaction mixture and concentrated to afford the crude product. Crude product was purified by flash column chromatography on silica gel eluting with 15 % methanol in dichloromethane to afford (S)-6-((tert-butoxycarbonyl)amino)-2-(2-(2-methoxyethoxy)acetamido)hexanoic acid (**10-4**) as a liquid. LC-MS ESI/APCI calc'd. for C₁₆H₃₀N₂O₇ [M + 1]⁺ 363.41, found 363.2.
Step 3. Preparation of (S)-6-amino-2-(2-(2-methoxyethoxy)acetamido)hexanoic acid:
   To a stirred solution of (S)-6-((tert-butoxycarbonyl)amino)-2-(2-(2-methoxyethoxy)acetamido)hexanoic acid (**10-4**) (4g, 11.04 mmol) in dichloromethane (20.0 mL) was added TFA (8.50 ml, 110mmol). The reaction mixture was stirred at room temperature for 4 hours. Reaction completion was confirmed by TLC. The reaction mixture was neutralized with 1.5 N HCl and concentrated under reduced pressure. The crude residue was dissolved in methanol and filtered through a CELITE bed and the filtrate was again concentrated. Crude product was first purified by flash column chromatography on silica gel eluting with 10% ammonia in methanol and again purified by reverse phase chromatography eluting with water to afford (S)-6-amino-2-(2-(2-methoxyethoxy)acetamido)hexanoic acid (**C10**) as a solid. LC-MS ESEAPCI calc'd. for CₙH₂₂N₂O₅[M + H]⁺ 263.3, found 263.4. ¹H NMR 400 MHz, D₂O: δ 4.14 (t, J = 5.20 Hz, 1H), 4.01 (s, 2H), 3.65-3.66 (m, 2H), 3.56-3.57 (m, 2H), 3.31 (s, 3H), 2.89 (t, J = 7.60 Hz, 2H), 1.78-1.78 (m, 1H), 1.64-1.66 (m, 3H), 1.55-1.55 (m, 2H).

### EXAMPLE 11

### Preparation of Compound 11 (C11): (S)-6-amino-2-(bis(2-(2-methoxyethoxy)ethyl)amino)hexanoic acid

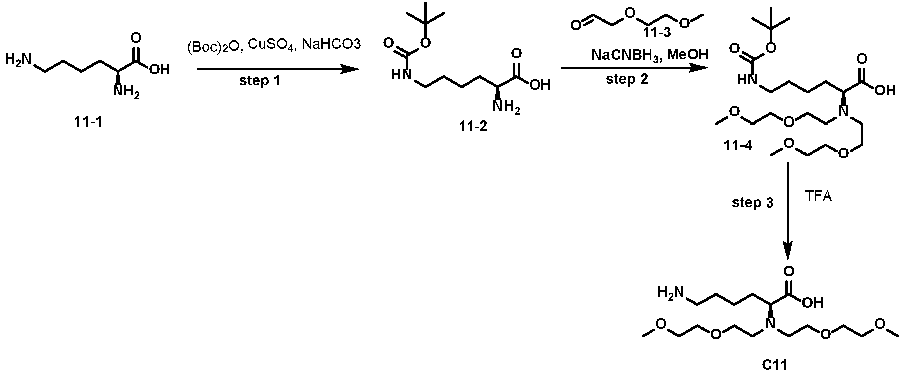

Step 2. Preparation of (S)-2-(bis(2-(2-methoxyethoxy)ethyl)amino)-6-((tert-butoxycarbonyl)amino)hexanoic acid:
   To a solution of (S)-2-amino-6-((tert-butoxycarbonyl)amino)hexanoic acid (11-2) (6 g, 24.36 mmol) in MeOH (60.0 mL) were added 2-(2-methoxyethoxy)acetaldehyde (11-3) (11.51 g, 97 mmol) and acetic acid (0.27 ml, 4.87 mmol). The reaction mixture was stirred at room temperature for 15 minutes. The reaction mixture was cooled to 0°C. Sodium cyanoborohydride (4.59 g, 73.1 mmol) was added portionwise and the reaction mixture was stirred for 20 hours at room temperature. Reaction completion was confirmed by TLC. Saturated sodium bicarbonate was then added to the reaction mixture and concentrated to afford the crude product. Crude product was purified by flash column chromatography on silica gel eluting with 7% methanol to afford (S)-2-(bis(2-(2-methoxyethoxy)ethyl)amino)-6-((tert-butoxycarbonyl)amino)hexanoic acid (**11-4**) LC-MS ESI/APCI calc'd. for C₂₁H₄₂N₂O₈ [M + H]⁺ 451.56, found 451.2.
Step 3. Preparation of (S)-6-amino-2-(bis(2-(2-methoxyethoxy)ethyl)amino)hexanoic acid:
   To a stirred solution of (S)-2-(bis(2-(2-methoxyethoxy)ethyl)amino)-6-((tert-butoxycarbonyl)amino)hexanoic acid (11-4) (3g, 6.6 mmol) in dichloromethane (15.0 mL), was added TFA (5.06 ml, 66.6mmol) and the reaction mixture was stirred at room temperature for 4 hours. Reaction completion was confirmed by TLC. The reaction mixture was neutralized with saturated sodium bicarbonate and concentrated under reduced pressure. The crude residue was dissolved in methanol and filtered through a CELITE bed and the filtrate was again concentrated. Crude product was first purified by flash column chromatography on silica gel eluting with 10% ammonia in methanol and again purified by reverse phase chromatography eluting with water to afford (S)-6-amino-2-(bis(2-(2-methoxyethoxy)ethyl)amino)hexanoic acid (**C11**) as a solid. LC-MS ESEAPCI calc'd. for C₁₆H₃₄N₂O₆ [M + H]⁺ 351.45, found 351.2. ¹H NMR 400 MHz, D₂O: δ 3.52-3.53 (m, 13H), 3.28 (s, 6H), 3.26-3.28 (m, 4H), 2.91 (t, J = 7.60 Hz, 2H), 1.74-1.76 (m, 2H), 1.58-1.60 (m, 2H), 1.43-1.44 (m, 2H).

### EXAMPLE 12

### Preparation of Compound 12 (C12): (S)- 3-(1H-imidazol-4-yl)-2-((2-(2-methoxyethoxy)ethyl)amino)propanoic acid

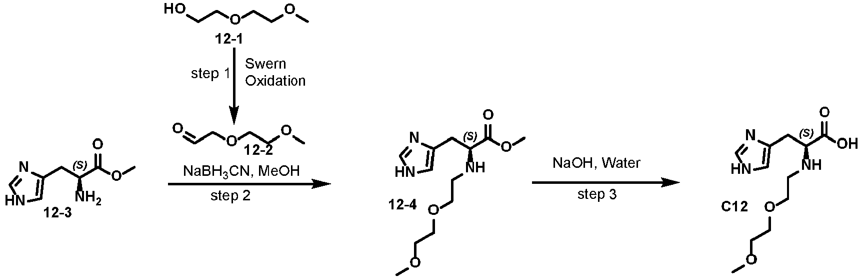

Step 2. Preparation of (S)-methyl 3-(1H-imidazol-4-yl)-2-((2-(2-methoxyethoxy)ethyl)amino)propanoate:
   To a solution (S)-methyl 2-amino-3-(1H-imidazol-4-yl)propanoate (12-3) (10 g, 59 mmol) in methanol (150 ml) was added 2-(2-methoxyethoxy)acetaldehyde (12-2) (10.47 g, 88.7 mmol) and acetic acid (1.01 ml, 17.4 mmol). The reaction mixture was stirred at room temperature for 15 minutes. The reaction mixture was cooled to 0°C and sodium cyanoborohydride (5.49 g, 88.7 mmol) was added portionwise. The reaction mixture was stirred for 6 hours at room temperature. Reaction completion was confirmed by TLC. Saturated sodium bicarbonate was then added to the reaction mixture and concentrated to afford the crude product. Crude product was purified by flash column chromatography on silica gel eluting with 7% methanol in to afford (S)-methyl 3-(1H-imidazol-4-yl)-2-((2-(2-methoxyethoxy)ethyl)amino)propanoate (12-4). LC-MS ESI/APCI calc'd. for C₁₂H₂₁N₃O₄ [M + H]⁺ 272.3, found 272.2.
Step 3. Preparation of (S)- 3-(1H-imidazol-4-yl)-2-((2-(2-methoxyethoxy)ethyl)amino)propanoic acid:
   To a stirred solution of (S)-methyl 3-(1H-imidazol-4-yl)-2-((2-(2-methoxyethoxy)ethyl)amino)propanoate (12-4) (5g, 18.4 mmol) in MeOH (50.0 mL) and water (10.0 mL) was added sodium hydroxide (1.1 g, 27.6 mmol). The reaction mixture was stirred at room temperature for 5 hours. Reaction completion was confirmed by TLC. The crude residue was dissolved in methanol and filtered through a CELITE bed and the filtrate was again concentrated. Crude product was first purified by flash column chromatography on silica gel eluting with 5% ammonia in methanol and again purified by reverse phase chromatography eluting with water to afford (S)- 3-(1H-imidazol-4-yl)-2-((2-(2-methoxyethoxy)ethyl)amino)propanoic acid (**C12**) as a solid. LC-MS ESI/APCI calc'd. for C₁₁H₁₉N₃O₄ [M + H]⁺ 258.28, found 258.2. ¹H NMR 400 MHz, D₂O: δ 7.54 (s, 1H), 6.78 (s, 1H), 3.49-3.50 (m, 6H), 3.26 (s, 3H), 3.24 (t, J = 7.20 Hz, 1H), 2.76-2.76 (m, 2H), 2.66-2.67 (m, 1H), 2.50-2.52 (m, 1H).

### EXAMPLE 13

### Preparation of Compound 13 (C13): (2-(2-methoxyethoxy)acetyl)-L-histidine

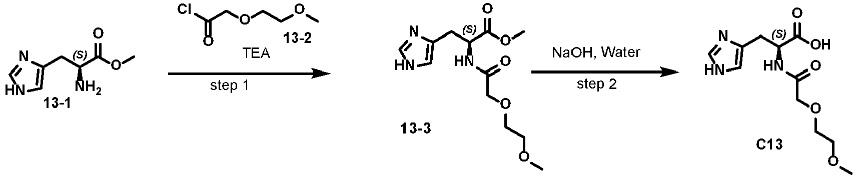

Step 1. Preparation of methyl (2-(2-methoxyethoxy)acetyl)-L-histidinate:
   To a solution of (S)-methyl 2-amino-3-(1H-imidazol-4-yl)propanoate (**13-1**) (5g, 29.6 mmol) in dichloromethane (25 ml) and triethyl amine (8.97, 89mmol) was added 2-(2-methoxyethoxy)acetyl chloride (**13-2**) (4.49g, 29.6mmol) (freshly prepared from acid) in dichloromethane (25 ml) dropwise at 0°C. The reaction mixture was stirred for 3 hours at room temperature. Reaction completion was confirmed by TLC. Saturated sodium bicarbonate was then added to the reaction mixture, which was concentrated to afford the crude product. Crude product was purified by flash column chromatography on silica gel eluting with 7 % methanol in dichloromethane to afford methyl (2-(2-methoxyethoxy)acetyl)-L-histidinate (13-3) as a liquid. LC-MS ESI/APCI calc'd. for C₁₂H₁₉N₃O₅ [M + 1]⁺ 286.30, found 286.2.
Step 2. Preparation of (2-(2-methoxyethoxy)acetyl)-L-histidine:
   To a solution of methyl (2-(2-methoxyethoxy)acetyl)-L-histidinate (**13-3**) (4.7 g, 16.4 mmol) in MeOH (47.0 mL) and water (5.0 mL) was added sodium hydroxide (0.98 g, 24.6 mmol). The reaction mixture was stirred at room temperature for 5 hours. Reaction completion was confirmed by TLC. 1.5 N HCl was added to the reaction mixture, which was concentrated under reduced pressure. The crude residue was dissolved in methanol and filtered through a CELITE bed and the filtrate was again concentrated. Crude product was first purified by flash column chromatography on silica gel eluting with 5% ammonia in methanol and again purified by reverse phase chromatography eluting with water to afford (2-(2-methoxyethoxy)acetyl)-L-histidine (**C13**) as a solid. LC-MS ESI/APCI calc'd. for C₁₁H₁₇N₃O₅ [M + H]+ 272.27, found 272.2. ¹H NMR 400 MHz, D₂O: δ 8.22 (s, 1H), 7.06 (s, 1H), 4.41 (q, J = 4.96 Hz, 1H), 3.93 (d, J = 3.00 Hz, 2H), 3.55-3.57 (m, 4H), 3.25 (s, 3H), 3.13-3.16 (m, 1H), 2.99-3.00 (m, 1H).

### EXAMPLE 14

### Preparation of Compound 14 (C14): (S)-5-guanidino-2-((2-(2-methoxyethoxy)ethyl)amino)pentanoic acid

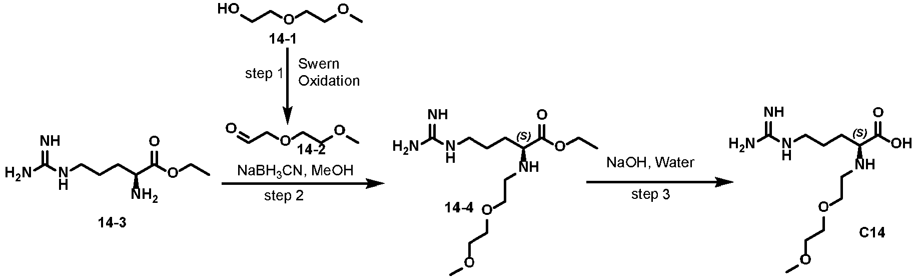

Step 2. Preparation of (S)-ethyl 5-guanidino-2-((2-(2-methoxyethoxy)ethyl)amino)pentanoate:
   To a solution (S)-ethyl 2-amino-5-guanidinopentanoate (**14-3**) (30.0 g, 148 mmol) in MeOH (300.0 mL) was added 2-(2-methoxyethoxy)acetaldehyde (**14-2**) (26.2 g, 222 mmol) and acetic acid (1.69 ml, 29.6 mmol). The reaction mixture was stirred at room temperature for 15 minutes. The reaction mixture was cooled to 0°C and sodium cyanoborohydride (13.8 g, 222 mmol) was added portionwise. The reaction mixture was stirred for 6 hours at room temperature. Reaction completion was confirmed by TLC. Saturated sodium bicarbonate was then added to the reaction mixture, which was concentrated to afford the crude product. Crude product was purified by flash column chromatography on silica gel eluting with 7% methanol in to afford (S)-ethyl 5-guanidino-2-((2-(2-methoxyethoxy)ethyl)amino)pentanoate (**14-4**). LC-MS ESI/APCI calc'd. for C₁₃H₂₈N₄O₄ [M + H]⁺ 305.3, found 305.2.
Step 3. Preparation of (S)-5-guanidino-2-((2-(2-methoxyethoxy)ethyl)amino)pentanoic acid:
   To a solution of methyl (S)-ethyl 5-guanidino-2-((2-(2-methoxyethoxy)ethyl)amino)pentanoate (14-4) (5 g, 16.4 mmol) in MeOH (50.0 mL) and water (5.0 mL) was added sodium hydroxide (0.98 g, 24.6 mmol) and the reaction mixture was stirred at room temperature for 5 hours. Reaction completion was confirmed by TLC. 1.5 N HCl was added to the reaction mixture and it was concentrated under reduced pressure. The crude residue was dissolved in methanol and filtered through a CELITE bed and the filtrate was again concentrated. Crude product was first purified by flash column chromatography on silica gel eluting with 5% ammonia in methanol and again purified by reverse phase chromatography eluting with water to afford (S)-5-guanidino-2-((2-(2-methoxyethoxy)ethyl)amino)pentanoic acid (**C14**) as a solid. LC-MS ESI/APCI calc'd. for C₁₁H₂₄N₄O₄ [M + H]⁺ 277.34, found 277.2. ¹H NMR 400 MHz, D₂O: δ 3.55-3.56 (m, 6H), 3.28 (s, 3H), 3.15-3.17 (m, 1H), 3.10 (t, J = 6.80 Hz, 2H), 2.81-2.82 (m, 1H), 2.68-2.70 (m, 1H), 1.50-1.52 (m, 4H).

### EXAMPLE 15

### Preparation of Compound 15 (C15): (2-(2-methoxyethoxy)acetyl)-L-arginine dihydrochloride

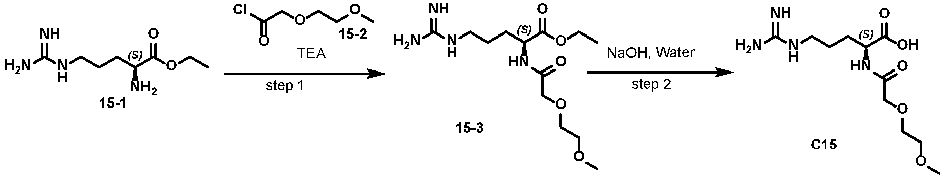

Step 1. Preparation of ethyl (2-(2-methoxyethoxy)ethyl)-L-argininate:
   To a solution of (S)-ethyl 2-amino-5-guanidinopentanoate (**15-1**) (5g, 24.72 mmol) in dichloromethane (25 ml) and triethylamine (10.34 ml, 74.2 mmol) was added 2-(2-methoxyethoxy)acetyl chloride (**15-2**) (3.76 g, 24.72 mmol)(freshly prepared from acid) in dichloromethane (25 ml) dropwise at 0°C. The reaction mixture was stirred for 3 hours at room temperature. Reaction completion was confirmed by TLC. Saturated sodium bicarbonate was then added to the reaction mixture and concentrated to afford the crude product. Crude product was purified by flash column chromatography on silica gel eluting with 7 % methanol in dichloromethane to afford ethyl (2-(2-methoxyethoxy)ethyl)-L-argininate (**15-3**) as a liquid. LC-MS ESEAPCI calc'd. for C₁₃H₂₆N₄O₅ [M + 1]⁺ 319.37, found 319.2.
Step 2. Preparation of (2-(2-methoxyethoxy)acetyl)-L-arginine dihydrochloride:
   To a solution of methyl ethyl (2-(2-methoxyethoxy)ethyl)-L-argininate (15-3) (6.1 g, 19.1 mmol) in MeOH (61.0 mL) and water (6.0 mL) was added sodium hydroxide (1.15 g, 28.7 mmol) and the reaction mixture was stirred at room temperature for 5 hours. Reaction completion was confirmed by TLC. The reaction mixture was acidified with 1.5 N HCl to pH(2-3) and concentrated under reduced pressure. The residue was dissolved in methanol and filtered through a CELITE bed and the filtrate was again concentrated. Crude product was purified by reverse phase chromatography eluting with 0.1% HCl in water to afford (2-(2-methoxyethoxy)acetyl)-L-arginine dihydrochloride (**C15**). LC-MS ESI/APCI calc'd. for C₁₁H₂₂N₄O₅ [M + 1]⁺ 291.32, found 291.2. ¹H NMR 400 MHz, DMSO-d6: δ 9.36 (s, 1H), 7.72 (s, 1H), 7.67 (s, 4H), 3.89 (t, J = 6.00 Hz, 1H), 3.85 (s, 2H), 3.58-3.59 (m, 2H), 3.44-3.47 (m, 2H), 3.26 (s, 3H), 3.02-3.04 (m, 2H), 1.64-1.65 (m, 2H), 1.56-1.57 (m, 2H).

### EXAMPLE 16

### Preparation of Compound 16 (C16): ((2,5,8,11-tetraoxatridecan-13-oyl)-L-histidine

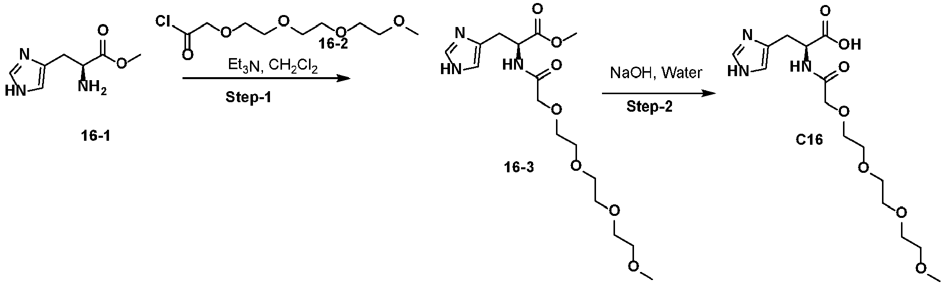

Step 1. Preparation of methyl (2,5,8,11-tetraoxatridecan-13-oyl)-L-histidinate:
   To a solution of (S)-methyl 2-amino-3-(1H-imidazol-4-yl)propanoate (**16-1**) (5g, 29.6 mmol) in dichloromethane (25 ml) and triethylamine (8.97, 89 mmol) was added 2,5,8,11-tetraoxatridecan-13-oyl chloride (**16-2**) (5.81g, 29.6mmol) (freshly prepared from acid) in dichloromethane (25 ml) dropwise at 0°C. The reaction mixture was stirred for 3 hours at room temperature. Reaction completion was confirmed by TLC. Saturated sodium bicarbonate was then added to the reaction mixture and the mixture was concentrated to afford the crude product. Crude product was purified by flash column chromatography on silica gel eluting with 10 % methanol in dichloromethane to yield methyl (2,5,8,11-tetraoxatridecan-13-oyl)-L-histidinate (**16-3**) as a liquid. LC-MS ESI/APCI calc'd. for C₁₆H₂₇N₃O₇ [M + 1]⁺ 374.18, found 374.2.
Step 2. Preparation of ((2,5,8,11-tetraoxatridecan-13-oyl)-L-histidine:
   To a stirred solution of (S)-methyl 12-((1H-imidazol-4-yl)methyl)-10-oxo-2,5,8-trioxa-11-azatridecan-13-oate (**16-3**) (4.7 g, 14.2 mmol) in MeOH (50.0 mL) and water (10.0 mL) was added sodium hydroxide (0.85 g, 21.3 mmol). The reaction mixture was stirred at room temperature for 5 hours. Reaction completion was confirmed by TLC. 1.5 N HCl was added to the reaction mixture and it was concentrated under reduced pressure. The crude residue was dissolved in methanol and filtered through a celite bed and the filtrate was again concentrated. Crude product was first purified by flash column chromatography on silica gel eluting with 5% ammonia in methanol and again purified by reverse phase chromatography, eluting with water to yield ((2,5,8,11-tetraoxatridecan-13-oyl)-L-histidine (**C16**) as a solid. LC-MS ESI/APCI calc'd. for C₁₅H₂₅N₃O₇ [M + H]⁺ 317.33, found 317.17. ¹H NMR 400 MHz, D₂O: δ 8.52 (s, 1H), 7.22 (s, 1H), 4.70-4.59 (m, 1H), 3.99-3.97 (m, 2H), 3.54-3.61 (m, 11H), 3.26-3.28 (m, 4H), 3.09-3.07 (m, 2H).

### EXAMPLE 17

### Preparation of Compound 17 (C17): (2,5,8,11-tetraoxatridecan-13-oyl)-L-arginine

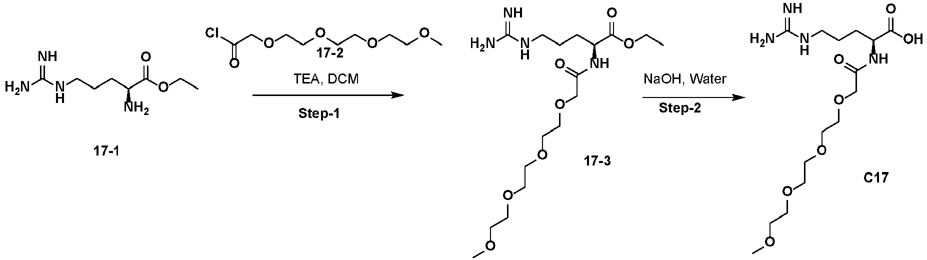

Step 1. Preparation of ethyl (2,5,8,11-tetraoxatridecan-13-oyl)-L-argininate:
   To a solution of ethyl L-argininate (**17-1**) (9g, 44.5 mmol) in dichloromethane (100 ml) and triethylamine (10.34 ml, 74.2 mmol) was added 2,5,8,11-tetraoxatridecan-13-oyl chloride (**17-2**) (10.71 g, 44.5 mmol) (freshly prepared from acid) in dichloromethane (25 ml) dropwise at 0°C. The reaction mixture was stirred for 3 hours at room temperature. Reaction completion was confirmed by TLC. Saturated sodium bicarbonate was then added to the reaction mixture and concentrated to afford the crude product. Crude product was purified by flash column chromatography on silica gel eluting with 10 % methanol in dichloromethane to afford ethyl (2,5,8,11-tetraoxatridecan-13-oyl)-L-argininate (**17-3**) as a liquid. LC-MS ESI/APCI calc'd. for C₁₇H₃₄N₄O₇ [M + 1]⁺ 407.24, found 407.2.
Step 2. Preparation of (2,5,8,11-tetraoxatridecan-13-oyl)-L-arginine:
   To a stirred solution of ethyl (2,5,8,11-tetraoxatridecan-13-oyl)-L-argininate (**17-3**) (5 g, 11.19 mmol) in methanol (50 ml) and water (10 ml) was added sodium hydroxide (0.74 g, 18.45 mmol). The reaction mixture was stirred at room temperature for 5 hours. Reaction completion was confirmed by TLC. The reaction mixture was neutralized with 1.5N HCl and concentrated under reduced pressure. The crude residue was dissolved in methanol and filtered through a CELITE bed and the filtrate was again concentrated. Crude product was first purified by flash column chromatography on silica gel eluting with 5% ammonia in methanol and again purified by reverse phase chromatography eluting with water to afford (2,5,8,11-tetraoxatridecan-13-oyl)-L-arginine (**C17**) as a solid. LC-MS ESI/APCI calc'd. for C₁₅H₃₀N₄O₇ [M + H]⁺ 379.21, found 379.2. ¹H NMR 400 MHz, D₂O: δ 4.35-4.36 (m, 1H), 4.05 (s, 2H), 3.58-3.69 (m, 10H), 3.52-3.53 (m, 2H), 3.29-0.00 (m, 3H), 3.14 (t, J = 6.80 Hz, 2H), 1.86-1.87 (m, 1H), 1.69-1.70 (m, 1H), 1.54-1.56 (m, 2H).

### EXAMPLE 18

### Preparation of Compound 18 (C18): (2,5,8,11-tetraoxatridecan-13-yl)-L-histidine

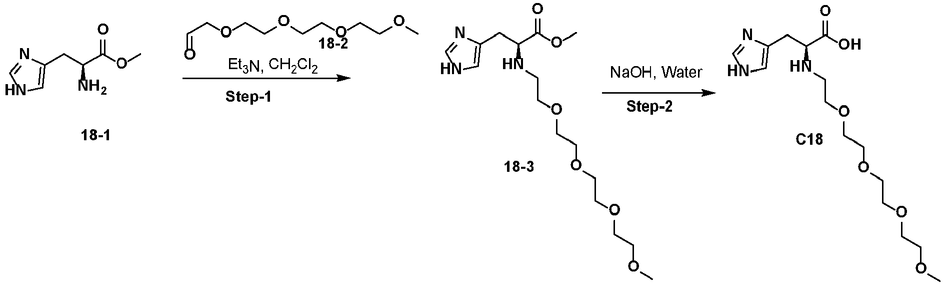

Step 2. Preparation of methyl (2,5,8,11-tetraoxatridecan-13-yl)-L-histidinate:
   To a solution methyl L-histidinate (**18-1**) (6.0 g, 53.2 mmol) in MeOH (50.0 mL), was added 2,5,8,11-tetraoxatridecan-13-al (**18-2**) (70.97 g, 53.2 mmol) and acetic acid (1.69 ml, 29.6 mmol). The reaction mixture was stirred at room temperature for 15 minutes. The reaction mixture was cooled to 0°C and sodium cyanoborohydride (3.34 g, 53.2 mmol) was added portion wise. The reaction mixture was stirred for 6 hours at room temperature. Reaction completion was confirmed by TLC. Saturated sodium bicarbonate was then added to the reaction mixture and concentrated to afford the crude product. Crude product was purified by flash column chromatography on silica gel eluting with 7% methanol in to afford methyl (2,5,8,11-tetraoxatridecan-13-yl)-L-histidinate (**18-3**). LC-MS ESI/APCI calc'd. for C₁₆H₂₉N₃O₆ [M + H]⁺ 360.21, found 360.2.
Step 3. Preparation of (2,5,8,11-tetraoxatridecan-13-yl)-L-histidine:
   To a solution of methyl (2,5,8,11-tetraoxatridecan-13-yl)-L-histidinate (**18-3**) (4.5 g, 12.5 mmol) in MeOH (50.0 mL) and water (5.0 mL) was added sodium hydroxide (0.75 g, 18.78 mmol) and the reaction mixture was stirred at room temperature for 5 hours. Reaction completion was confirmed by TLC. 1.5 N HCl was added to the reaction mixture and concentrated under reduced pressure. The crude residue was dissolved in methanol and filtered through a CELITE bed and the filtrate was again concentrated. Crude product was first purified by flash column chromatography on silica gel eluting with 5% ammonia in methanol and again purified by reverse phase chromatography eluting with water to afford (2,5,8,11-tetraoxatridecan-13-yl)-L-histidine (**C18**) as a solid. LC-MS ESI/APCI calc'd. for C₁₅H₂₇N₃O₆ [M + H]⁺ 346.19, found 346.2. ¹H NMR 400 MHz, D₂O: δ 4.37 (t, J = 4.00 Hz, 1H), 4.05 (s, 2H), 3.52-3.53 (m, 12H), 3.29 (s, 3H), 3.14 (t, J = 6.80 Hz, 2H), 1.87-1.88 (m, 1H), 1.69-1.70 (m, 1H), 1.54-1.56 (m, 2H).

### EXAMPLE 19

### Preparation of Compound 19 (C19): (2-(2-(2-methoxyethoxy)ethoxy)ethyl)-L-histidine

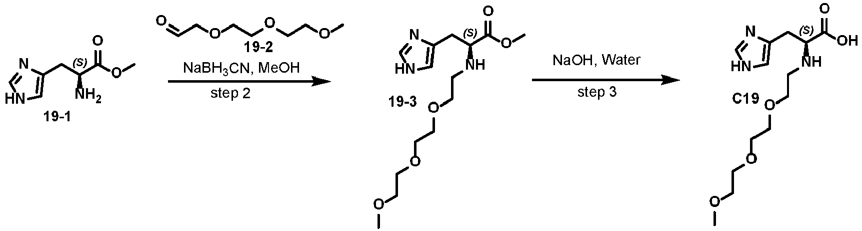

Step 2. Preparation of methyl (2-(2-(2-methoxyethoxy)ethoxy)ethyl)-L-histidinate:
   To a solution (S)-methyl 2-amino-3-(1H-imidazol-4-yl)propanoate (**19-1**) (5 g, 29.6 mmol) in methanol (75 ml) was added 2-(2-(2-methoxyethoxy)ethoxy)acetaldehyde (**19-2**) (7.1 g, 44.3 mmol) and acetic acid (1.01 ml, 17.4 mmol). The reaction mixture was stirred at room temperature for 15 minutes. The reaction mixture was cooled to 0°C and sodium cyanoborohydride (2.79 g, 44.3 mmol) was added portionwise. The reaction mixture was stirred for 6 hours at room temperature. Reaction completion was confirmed by TLC. Saturated sodium bicarbonate was then added to the reaction mixture and concentrated to afford the crude product. Crude product was purified by flash column chromatography on silica gel eluting with 7% methanol in to afford methyl (2-(2-(2-methoxyethoxy)ethoxy)ethyl)-L-histidinate (**19-3**). LC-MS ESI/APCI calc'd. for C₁₄H₂₅N₃O₅ [M + H]⁺ 316.18, found 316.2.
Step 3. Preparation of (2-(2-(2-methoxyethoxy)ethoxy)ethyl)-L-histidine:
   To a stirred solution of (S)-methyl 3-(1H-imidazol-4-yl)-2-((2-(2-methoxyethoxy)ethyl)amino)propanoate (**19-3**) (3 g, 9.51 mmol) in MeOH (50.0 mL) and water (10.0 mL) was added sodium hydroxide (0.57 g, 14.2 mmol) and the reaction mixture was stirred at room temperature for 5 h. Reaction completion was confirmed by TLC. The crude residue was dissolved in methanol and filtered through a CELITE bed and the filtrate was again concentrated. Crude product was first purified by flash column chromatography on silica gel eluting with 5% ammonia in methanol and again purified by reverse phase chromatography eluting with water to afford (2-(2-(2-methoxyethoxy)ethoxy)ethyl)-L-histidine (**C19**) as a solid. LC-MS ESI/APCI calc'd. for C₁₃H₂₃N₃O₅ [M + H]⁺ 302.16, found 302.2. ¹H NMR 400 MHz, D₂O: δ 8.20 (s, 1H), 7.16 (s, 1H), 3.85 (t, J = 6.40 Hz, 1H), 3.70 (t, J = 4.80 Hz, 2H), 3.59-3.61 (m, 6H), 3.52-3.53 (m, 2H), 3.28 (s, 3H), 3.16-3.17 (m, 4H).

### EXAMPLE 20

### Preparation of Compound 20 (C20): (S)-2-(bis(2-(2-hydroxyethoxy)ethyl)amino)-5-guanidinopentanoic acid

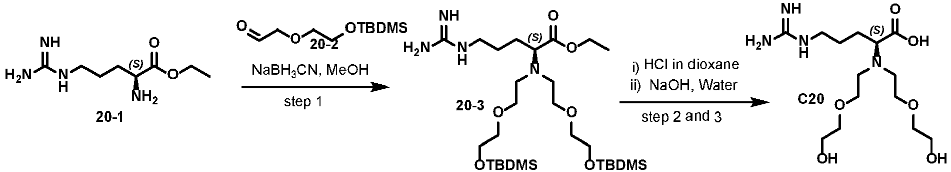

Step 1. Preparation of afford (S)-ethyl 10-(2-(2-((tert-butyldimethylsilyl)oxy)ethoxy)ethyl)-11-(3-guanidinopropyl)-2,2,3,3-tetramethyl-4,7-dioxa-10-aza-3-siladodecan-12-oate:
   To a stirred solution of (S)-ethyl 2-amino-5-guanidinopentanoate (**20-1**) (10 g, 49.6 mmol) in MeOH (100 ml), was added 2-(2-((tertbutyldimethylsilyl)oxy)ethoxy)acetaldehyde (**20-2**) (43.2 g, 198 mmol) and acetic acid (1.0 ml), and the reaction mixture was stirred at 0°C for 15 minutes. Then sodium cyanoborohydride (2.79 g, 44.3 mmol) was added at same temperature. Then the resultant mixture was stirred for 16 hours at room temperature. TLC and LCMS analysis showed formation of desired product. Saturated sodium bicarbonate solution (50 mL) was added to the reaction mixture, which was then concentrated. The aqueous layer was back extracted with DCM (5 x 100 mL). The combined organic layers were washed with brine (1 x150 mL). The organic layer was dried over Na₂SO₄, filtered and concentrated. The crude material was purified by column chromatography on silica gel 60-120 mesh, eluting with 10 % MeOH to afford (S)-ethyl 10-(2-(2-((tertbutyldimethylsilyl)oxy)ethoxy)ethyl)-11-(3-guanidinopropyl)-2,2,3,3-tetramethyl-4,7-dioxa-10-aza-3-siladodecan-12-oate (**20-3**) as a liquid. LC-MS ESI/APCI calc'd. for C₂₈H₆₂N₄O₆Si₂ [M + H]⁺ 606.42, found 606.1.
Step 2. Preparation of (S)-ethyl 2-(bis(2-(2-hydroxyethoxy)ethyl)amino)-5-guanidinopentanoate:
   To a stirred solution of (S)-ethyl 10-(2-(2-((tertbutyldimethylsilyl)oxy)ethoxy)ethyl)-11-(3-guanidinopropyl)-2,2,3,3-tetramethyl-4,7-dioxa-10-aza-3-siladodecan-12-oate (20-3) (5.1g, 8.40 mmol) in CH₂Cl₂ (20 mL) was added HCl in dioxane (10.5 mL, 42.0 mmol) and the reaction mixture was stirred at room temperature for 2 hours. The reaction mixture was concentrated to afford (S)-ethyl 2-(bis(2-(2-hydroxyethoxy)ethyl)amino)-5-guanidinopentanoate as a solid.
Step 3. Preparation of (S)-2-(bis(2-(2-hydroxyethoxy)ethyl)amino)-5-guanidinopentanoic acid:
   To a stirred solution of (S)-ethyl 2-(bis(2-(2-hydroxyethoxy)ethyl)amino)-5-guanidinopentanoate (3.11 g, 8.22 mmol) in MeOH (50 mL), water (10 mL) and NaOH (0.493 g, 12.33 mmol) were added. The reaction mixture was stirred at room temperature for 5 hours. TLC and LCMS analysis showed formation of desired product. The reaction mixture was concentrated, and neutralized with 1.5 N HCl and concentrated. The residue was dissolved in methanol, filtered through CELITE and concentrated to afford the crude product. The crude material was purified by column chromatography on silicagel eluting with 3-5% ammonia in methanol to give (S)-2-(bis(2-(2-hydroxyethoxy)ethyl)amino)-5-guanidinopentanoic acid (**C20**) as a solid. LC-MS ESI/APCI calc'd. for C₁₄H₃₀N₄O₆ [M + H]⁺ 351.22, found 351.2. ¹H NMR 400 MHz, D₂O: δ 3.82-3.83 (m, 1H), 3.77-3.78 (m, 4H), 3.64-3.65 (m, 4H), 3.55-3.56 (m, 4H), 3.42-3.48 (m, 4H), 3.17 (t, J = 6.80 Hz, 2H), 1.82-1.84 (m, 4H).

### EXAMPLE 21

### Preparation of Compound 21 (C21): (S)-2-(bis(2-(2-hydroxyethoxy)ethyl)amino)-3-(1H-imidazol-4-yl)propanoic acid

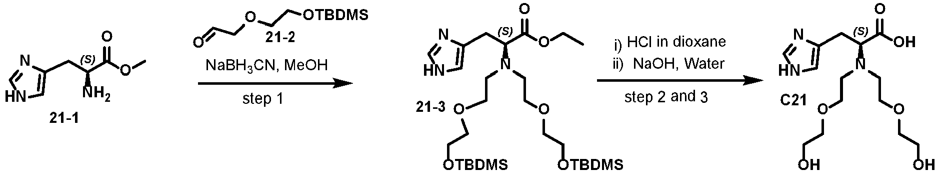

Step 1. Preparation of (S)-methyl 11-((1H-imidazol-4-yl)methyl)-10-(2-(2-((tertbutyldimethylsilyl)oxy)ethoxy)ethyl)-2,2,3,3-tetramethyl-4,7-dioxa-10-aza-3-siladodecan-12-oate:
To a stirred solution of (S)-methyl 2-amino-3-(1H-imidazol-4-yl)propanoate( **21-1**) (5 g, 29.6 mmol) in MeOH (50 mL) was added 2-(2-((tertbutyldimethylsilyl)oxy)ethoxy)acetaldehyde (**21-2**) (32.3 g, 148 mmol) and acetic acid (0.846 mL, 14.78 mmol). The reaction mixture was stirred at 0 °C for 15 minutes. Sodium cyanoborohydride (2.79 g, 44.3 mmol) was then added at same temperature. The resultant mixture was stirred for 16 hours at room temperature. TLC and LCMS analysis showed formation of desired product. Saturated sodium bicarbonate solution (50 mL) was added to the reaction mixture, which was concentrated. The aqueous layer was back extracted with CH₂Cl₂ (5 x 100 mL). The combined organic layers were washed with brine (1 x150 mL). The organic layer was dried over Na₂SO₄, filtered and concentrated. The crude material was purified by column chromatography on silica gel 60-120 mesh, eluting with 10 % MeOH in dichloromethane to afford (S)-methyl 11-((1H-imidazol-4-yl)methyl)-10-(2-(2-((tertbutyldimethylsilyl)oxy)ethoxy)ethyl)-2,2,3,3-tetramethyl-4,7-dioxa-10-aza-3-siladodecan-12-oate (**21-3**) as a liquid. LC-MS ESI/APCI calc'd. for C₂₈H₅₇N₃O₆Si₂ [M + H]⁺ 589.38, found 589.2.

Step 2: To a stirred solution of (S)-methyl 11-((1H-imidazol-4-yl)methyl)-10-(2-(2-((tert-butyldimethylsilyl)oxy)ethoxy)ethyl)-2,2,3,3-tetramethyl-4,7-dioxa-10-aza-3-siladodecan-12-oate 3 (8g, 13.94 mmol) in CH₂Cl₂ (80 mL), HCl in dioxane (8.68 g, 69.7 mmol), was added and the reaction mixture was stirred at 0 °C for 1 hour. TLC and LCMS analysis showed formation of desired product. The reaction mixture was concentrated under vacuum to afford the desired compound (S)-methyl 2-(bis(2-(2-hydroxyethoxy)ethyl)amino)-3-(1H-imidazol-4-yl)propanoate, which was used in the next step without any purification.

Step 3: To a stirred solution of (S)-methyl 2-(bis(2-(2-hydroxyethoxy)ethyl)amino)-3-(1H-imidazol-4-yl)propanoate (3.0 g, 8.69 mmol) in methanol (30 mL), NaOH (0.521 g, 13.03 mmol), was added and the reaction mixture was stirred at room temperature for 4hours. TLC and LCMS analysis showed formation of desired product. The reaction mixture was concentrated, the aqueous was neutralized with 1.5 N HCl and concentrated, the residue was dissolved in methanol, filtered through CELITE, concentrated to afford the crude product. The crude was purified by column chromatography on silica gel eluting with 3-5% ammonia in methanol to afford the product which was further purified by reverse phase chromatography, eluting with water, to give (S)-2-(bis(2-(2-hydroxyethoxy)ethyl)amino)-3-(1H-imidazol-4-yl)propanoic acid (**C21**) as a liquid. LC-MS ESI/APCI calc'd. for C₁₄H₂₅N₃O₆ [M + H]⁺ 332.17, found 332.2. ¹H NMR 400 MHz, D2O: δ 8.21 (s, 1H), 7.15 (s, 1H), 3.96 (t, J = 7.12 Hz, 1H), 3.66-3.67 (m, 4H), 3.57-3.59 (m, 4H), 3.50-3.51 (m, 4H), 3.25-3.26 (m, 6H).

### EXAMPLE 22

### Preparation of Compound 22 (C22): [(2-(2-methoxyethoxy)acetyl)-L-phenylalanine

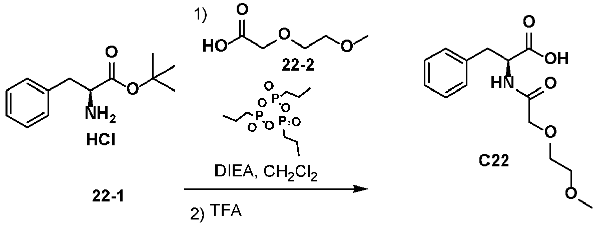

(S)-tert-butyl 2-amino-3-(4-(tert-butoxy)phenyl)propanoate hydrochloride (22-1) (2.75 g, 10.67 mmol) and 2-(2-methoxyethoxy)acetic acid (22-2) (1.717g, 12.0 mmol) were suspended in 50 mL CH₂Cl₂. 2,4,6-tripropyl-1,3,5,2,4,6-trioxatriphosphinane 2,4,6-trioxide (T3P) solution (50% in EtOAc, 13.58 mL, 21.34 mmol) was added followed by DIEA (2.79mL, 16mmol) immediately afterwards. The mixture was stirred at room temperature for 2 hours. The mixture was then treated with TFA (8.16 mL, 107 mmol) and stirred overnight. The mixture was concentrated under high vacuum. The residue was chromatographed on silica eluted with 0-70% hexane/EtOAc-EtOH(3:1) to afford (2-(2-methoxyethoxy)acetyl)-L-phenylalanine (C22) as an oil. LC-MS ESI/APCI calc'd. for C₁₄H₁₉NO₅ [M + 1]⁺ 282.13, found 282.18. ¹H NMR 400 MHz, DMSO-d6: δ 12.71 (s, 1H), 7.76 (d, J = 8.21 Hz, 1H), 7.19-7.31 (m, 5H), 4.53 (m, 1H), 3.85 (m, 2H), 3.47 (m, 4H), 3.25 (s, 1H), 3.12 (dd, J = 4.81 Hz, 14.10 Hz, 1H), 2.98 (dd, J = 9.19 Hz, 14.20 Hz, 1H)

### EXAMPLE 23

### Preparation of Compound 23 (C23): (2-(2-methoxyethoxy)acetyl)-L-tyrosine

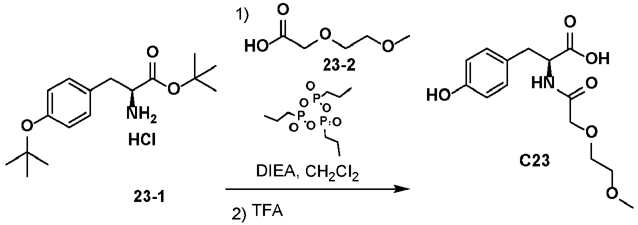

(S)-tert-butyl 2-amino-3-(4-(tert-butoxy)phenyl)propanoate hydrochloride (23-1) (3.48g, 10.55mmol) and 2-(2-methoxyethoxy)acetic acid (23-2) (1.698g, 12.66mmol) were suspended in 50 mL CH₂Cl₂. 2,4,6-tripropyl-1,3,5,2,4,6-trioxatriphosphinane 2,4,6-trioxide (T3P) solution (50% in EtOAc, 13.43mL, 21.10mmol) was added followed by DIEA (2.76 mL, 15.82mmol) immediately afterwards. The mixture was stirred at room temperature for 2 hours. The mixture was then treated with TFA (8.07 mL, 105mmol) and stirred overnight. The mixture was concentrated under high vacuum. The residue was chromatographed on silica eluted with 0-70% hexane/EtOAc-EtOH(3:1) to afford (2-(2-methoxyethoxy)acetyl)-L-tyrosine (C23) as an oil. The oil slowly solidified on stand for a week at room temperature to afford a semi-solid. LC-MS ESI/APCI calc'd. for C₁₄H₁₉NO₆ [M + 1]⁺ 298.12, found 298.19. ¹H NMR 400 MHz, DMSO-d6: δ 12.74 (s, 1H), 9.19 (s, 1H), 7.65 (d, J = 9.37 Hz, 1H), 6.99 (d, J = 8.45 Hz, 2H), 6.66 (d, J = 8.62 Hz, 2H), 4.44 (m, 1H), 3.85 (m, 2H), 3.41-3.56 (m, 4H), 3.26 (s, 1H), 2.99 (dd, J = 5.15 Hz, 14.00 Hz, 1H), 2.86 (dd, J = 8.58 Hz, 13.91 Hz, 1H)

### EXAMPLE 24

### Viscosity of Pharmaceutical Compositions Comprising Pegylated Excipients

Pembrolizumab formulations comprised the following: 10 mM histidine buffer, pembrolizumab at a concentration of 200 mg/mL, and the test excipient (pegylated excipient of the invention, as described herein) at a concentration of 200 mM, pH adjusted to pH 5.5. Pembrolizumab formulations without excipients consisted of 200 mg/mL pembrolizumab in 10 mM histidine buffer at pH 5.5. The 10 mM histidine buffer used with pembrolizumab formulations consisted of consists of 10 mM L-histidine in sterile water, pH adjusted to 5.5.

Antibody B formulations comprised the following: 10 mM histidine buffer, Antibody B at a concentration of 177 mg/mL and the test excipient (pegylated excipient of the invention, as described herein) at a concentration of 200 mM, pH adjusted to pH 6.0. The Antibody B formulation without excipients consisted of 177 mg/mL Antibody B in 10 mM histidine buffer at pH 6.0. The 10 mM histidine buffer used with Antibody B consisted of 10 mM L-histidine in sterile water, pH adjusted to 6.0.

Viscosity Measurements: All viscosity measurements were conducted using the mVROC (Viscometer/Rheometer-On-A-Chip, RheoSense, Inc., San Ramon, CA). The test formulations were placed into a 250 µl Hamilton air-tight syringe, which was then placed inside the syringe jacket of the mVROC. The instrument injected formulation into the chip for viscosity measurement at an appropriate flow rate. Most measurements were done in duplicates.

Viscosity of the test formulation is provided in Table 4 below. Results indicate that the pegylated excipients tested (i.e. compounds of Formula I) were able to reduce viscosity of the high concentration monoclonal antibody formulations relative to the same formulation without an excipient of the invention. In addition, all test formulations comprising 10 mM histidine buffer, 200 mg/mL pembrolizumab, and 0.2 M compound of Formula I had a lower viscosity than test formulations comprising 10 mM histidine buffer, 200 mg/mL pembrolizumab, 0.2 M arginine or histidine, which demonstrates that the compounds of the invention provide superior viscosity-lowering ability relative to compounds typically used in the industry. Similarly, test formulation comprising 10 mM histidine buffer, 177 mg/mL Antibody B, and 0.2 M compound of Formula I had a lower viscosity than the same formulation without a compound of Formula I.

**Table 4. Viscosity of Test Formulations**

| Excipient | Excipient Structure | Viscosity 1 (mPa-s) | Viscosity 2 (mPa-s) | Viscosity Average (mPa-s) |
|---|---|---|---|---|
| Pembrolizumab formulations: 10 mM histidine buffer, 200 mg/mL pembrolizumab, 0.2 M excipient | | | | |
| None (Pembro batch 1) | | 47.25 | 46.91 | 47.08 |
| None (Pembro batch 2) | | 47.25 | 49.91 | 48.58 |
| Arginine | 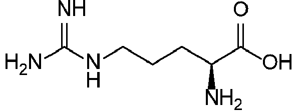 | 30.25 | 30.55 | 30.40 |
| Histidine | 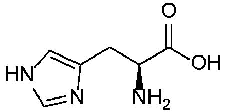 | 35.79 | 35.32 | 35.56 |
| Example 1 | 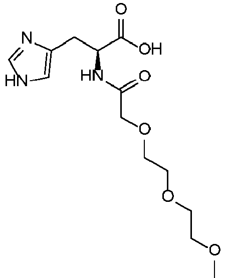 | 23.12 | 23.27 | 23.20 |
| Example 2 | 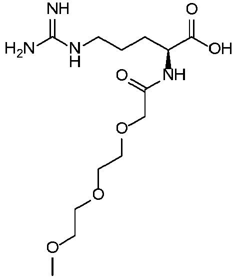 | 22.05 | 22.24 | 22.15 |
| Example 3 | 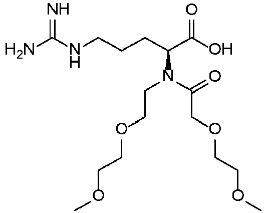 | 21.12 | 21.18 | 21.15 |
| Example 4 | 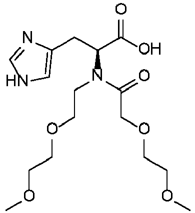 | 19.64 | 19.28 | 19.46 |
| Example 5 | 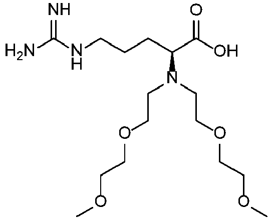 | 18.02* | 18.37* | 18.19** |
| | | *Average of 4 values | *Average of 4 values | **Average of 8 values |
| Example 6 | 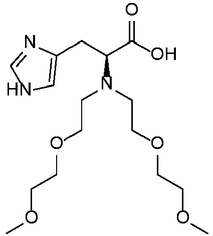 | 17.57* | 18.10* | 17.83** |
| | | *Average of 3 values | *Average of 3 values | **Average of 6 values |
| Example 10 | 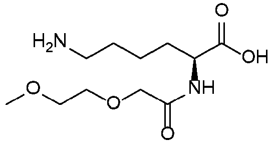 | 26.39 | 26.35 | 26.37 |
| Example 11 | 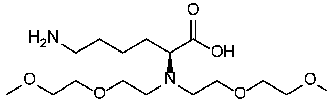 | 21.36 | 21.50 | 21.43 |
| Example 12 | 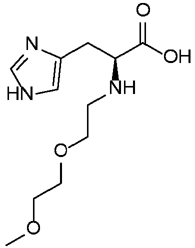 | 22.19 | 22.33 | 22.26 |
| Example 13 | 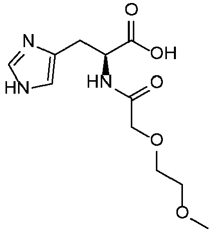 | 20.05 | 20.09 | 20.07 |
| Example 14 | 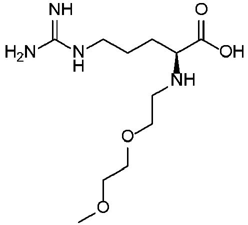 | 24.59 | 24.76 | 24.68 |
| Example 15 | 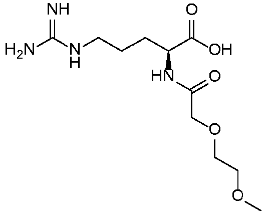 | 18.67 | ND | 18.67 |
| Example 16 | 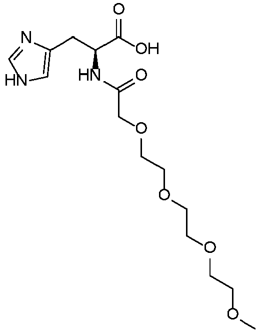 | 23.33 | 23.54 | 23.44 |
| Example 17 | 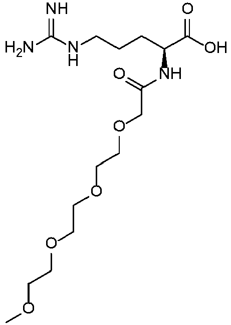 | 23.09 | 22.82 | 22.96 |
| Example 18 | 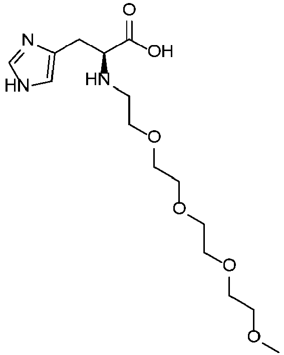 | 22.82 | 22.47 | 22.65 |
| Example 19 | 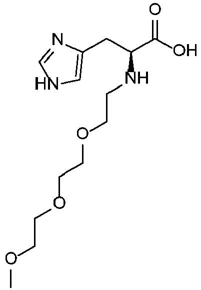 | 26.58 | 26.83 | 26.71 |
| Example 20 | 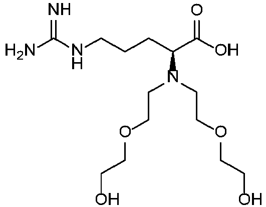 | 25.28 | 25.42 | 25.35 |
| Example 21 | 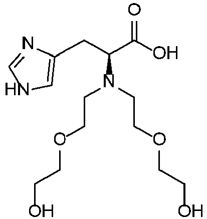 | 23.19 | 23.16 | 23.18 |
| Example 22 | 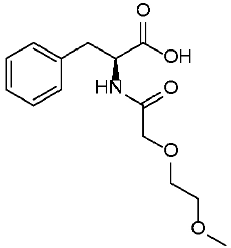 | 17.77 | 17.63 | 17.70 |
| Example 23 | 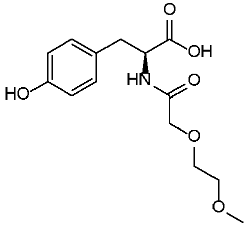 | 20.15 | 20.09 | 20.12 |

| Antibody B formulations: 10 mM histidine buffer, 177 mg/mL Antibody B, 0.2 M excipient | | | | |
|---|---|---|---|---|
| Example 8 | 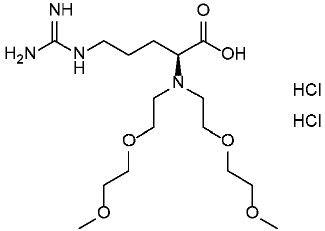 | 22.25 | 22.19 | 22.22 |
| Example 9 | 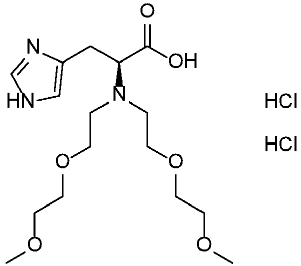 | 26.55 | 26.67 | 26.61 |
| None | | 41.52 | 41.08 | 41.30 |

## Claims

1. A compound of Formula I: 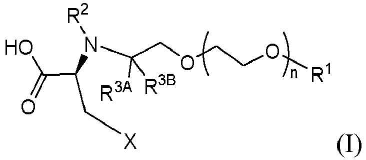
or a pharmaceutically acceptable salt thereof,
wherein:
X is 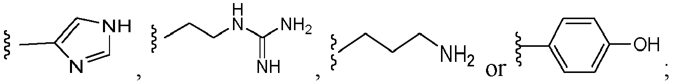
R¹ is H or methyl;
R² is 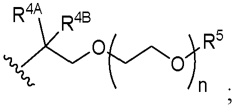
R^{3A} and R^{3B} are each H or together form oxo;
R^{4A} and R^{4B} are each H or together form oxo;
R⁵ is H or methyl; and
each occurrence of n is independently 1 to 5;
and wherein 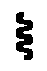 indicates the point of attachment to the rest of the compound.

2. A compound having the structure: 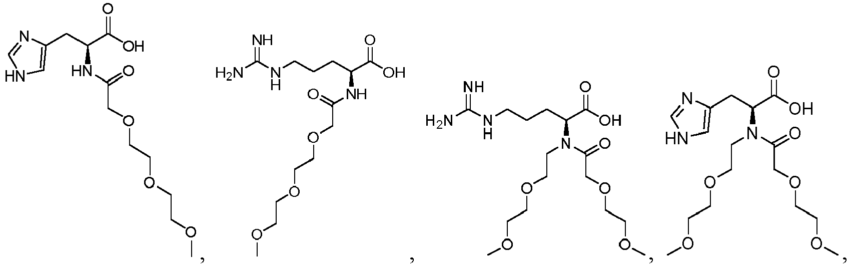 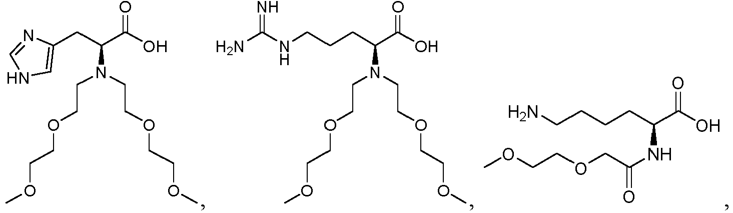 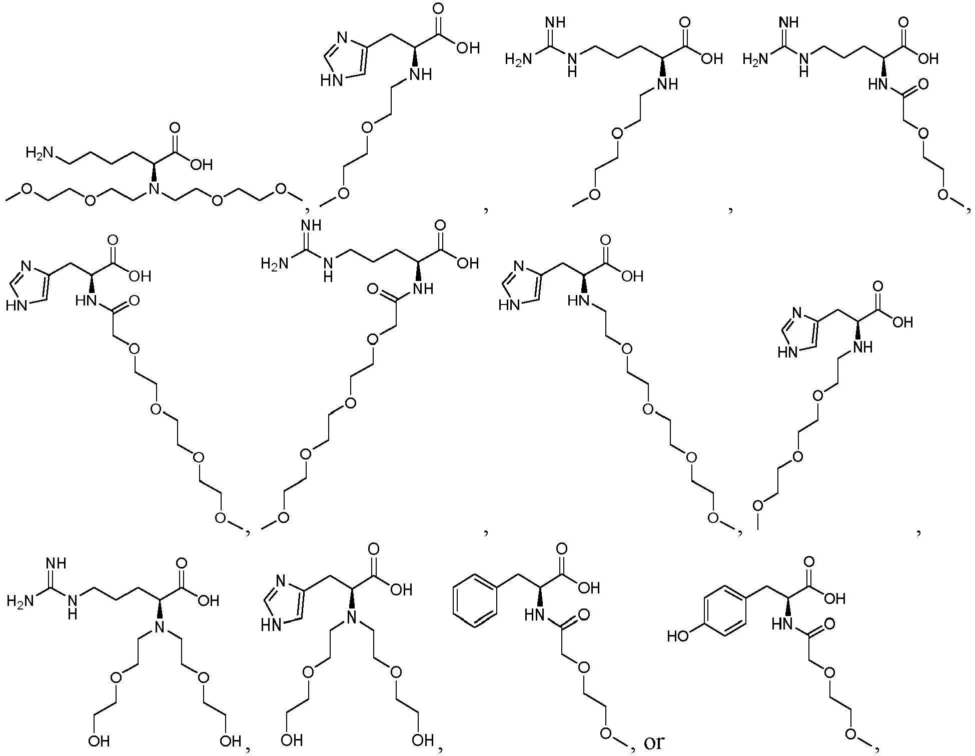 or a pharmaceutically acceptable salt thereof.

3. A pharmaceutical composition comprising an active biological ingredient (ABI) and a compound of Formula I: 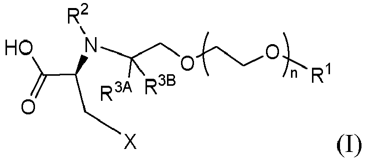
or a pharmaceutically acceptable salt thereof,
wherein:
X is 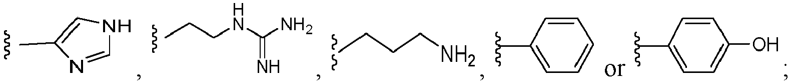
R¹ is H or methyl;
R² is H or 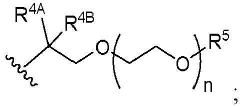
R^{3A} and R^{3B} are each H or together form oxo;
R^{4A} and R^{4B} are each H or together form oxo;
R⁵ is H or methyl; and
each occurrence of n is independently 1 to 5;
wherein 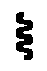 indicates the point of attachment to the rest of the compound;
wherein the ABI is an antibody or antigen binding fragment thereof or a therapeutic protein.

4. The pharmaceutical composition of claim 3, wherein the ABI is an antibody or antigen binding fragment thereof, present in a concentration of about 50-250 mg/mL.

5. The pharmaceutical composition according to claim 3 or 4, further comprising histidine buffer at about pH 5.0 to about pH 6.0 in a concentration of about 5 mM to about 20 mM, optionally
further comprising about 0.01 % to about 0.04% w/v non-ionic surfactant, optionally
wherein the non-ionic surfactant is polysorbate 80, optionally
wherein polysorbate 80 is present at a weight ratio of approximately 0.02% w/v.

6. The pharmaceutical composition according to any of claims 3-5, further comprising a stabilizer, optionally
wherein the stabilizer is sucrose, optionally
wherein the sucrose is present at a weight ratio of about 6% to about 8% w/v.

7. The pharmaceutical composition according to claims 3-6, wherein the ABI is an anti-PD-1 antibody or antigen binding fragment thereof that specifically binds human programmed death receptor 1 (PD-1), optionally
wherein the anti-PD-1 antibody or antigen binding fragment thereof, comprises three light chain complementarity determining regions (CDRs) comprising SEQ ID NO: 1, SEQ ID NO:2 and SEQ ID NO:3 and three heavy chain CDRs comprising SEQ ID NO:6, SEQ ID NO:7 and SEQ ID NO:8, optionally
wherein the anti-human PD-1 antibody or antigen binding fragment thereof comprises a V_{L} region which comprises the amino acid sequence set forth in SEQ ID NO:4, and a V_{H} region which comprises the amino acid sequence set forth in SEQ ID NO:9, optionally
wherein the the anti-PD-1 antibody or antigen binding fragment thereof comprises a light chain comprising or consisting of a sequence of amino acids as set forth in SEQ ID NO:5 and a heavy chain comprising or consisting of a sequence of amino acids as set forth in SEQ ID NO: 10, optionally
wherein the anti-human PD-1 antibody or antigen binding fragment thereof is pembrolizumab.

8. The pharmaceutical composition of any of claims 4 or 7, wherein the antibody or antigen binding fragment thereof is present at a concentration of 100 -250 mg/mL and wherein the composition comprises 10 mM histidine, 7% sucrose, and 0.02% polysorbate 80.

9. The pharmaceutical composition of any of claims 3-8, wherein the composition is an aqueous solution, optionally
further comprising a second ABI.

10. A pharmaceutical composition comprising from 100 to 200 mg/mL pembrolizumab, 10 mM histidine buffer and a compound of Formula I: 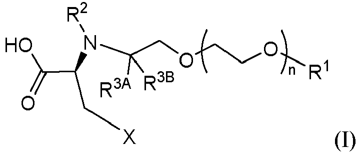
or a pharmaceutically acceptable salt thereof,
wherein:
X is 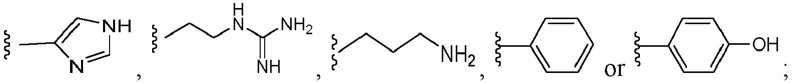
R¹ is H or methyl;
R² is H or 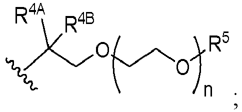
R^{3A} and R^{3B} are each H or together form oxo;
R^{4A} and R^{4B} are each H or together form oxo;
R⁵ is H or methyl; and
each occurrence of n is independently 1 to 5;
and wherein 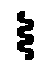 indicates the point of attachment to the rest of the compound, the composition optionally further comprising sucrose and polysorbate 80.

11. The pharmaceutical composition of any of claims 3-10, wherein R² is H.

12. The compound of claim 1, or the pharmaceutical composition of any of claims 3-10, wherein R² is

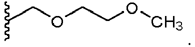

13. The compound of claim 1, or the pharmaceutical composition of any of claims 3-12, wherein R¹ is methyl, optionally
wherein R^{3A} and R^{3B} are H or
wherein R^{3A} and R^{3B} join to form oxo.

14. The compound of claim 1, or the pharmaceutical composition of any of claims 3-13, wherein each occurrence of n is independently 1 to 3

15. The pharmaceutical composition of any of claims 3-10, wherein the compound of Formula I has the following structure: 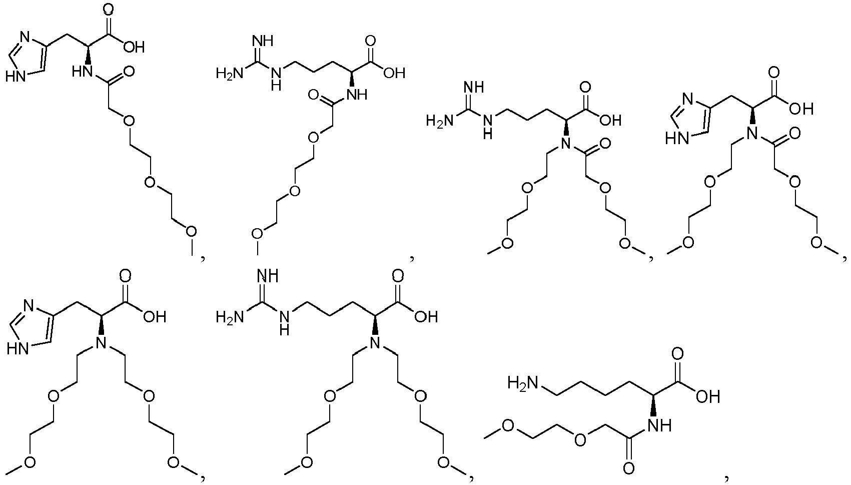 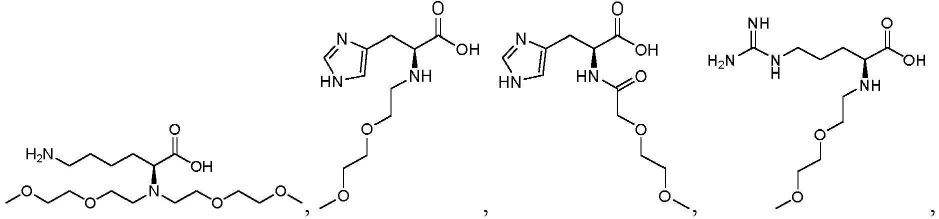 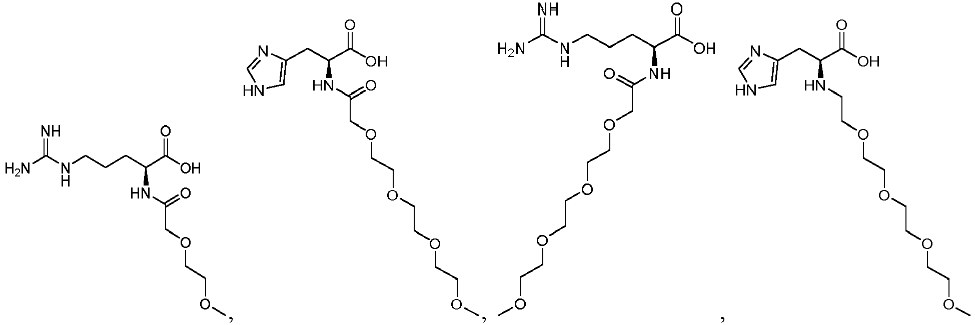 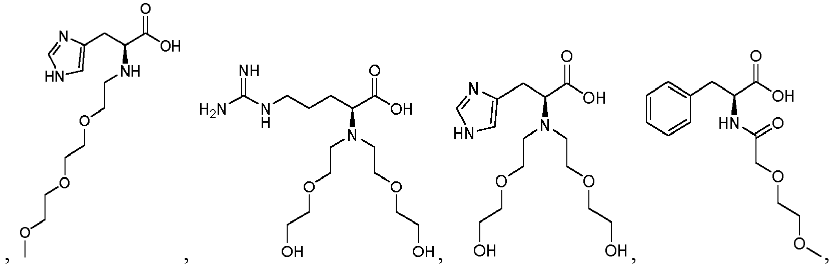 or

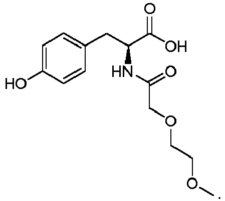

16. A method for lowering the viscosity of a pharmaceutical formulation comprising:
(a) providing a pharmaceutical formulation comprising an ABI at a concentration of about 50 mg/mL to about 250 mg/mL, wherein the formulation is in aqueous solution; and
(b) adding a compound of Formula I to the solution: 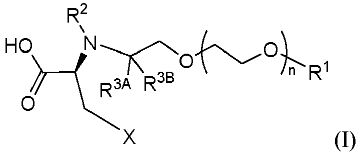
wherein:
Xis 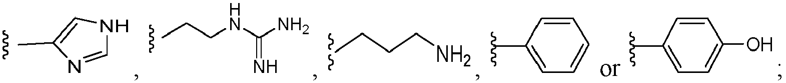
R¹ is H or methyl;
R² is H or 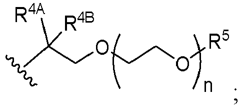
R^{3A} and R^{3B} are each H or together form oxo;
R^{4A} and R^{4B} are each H or together form oxo;
R⁵ is H or methyl; and
each occurrence of n is independently 1 to 5;
and wherein 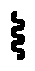 indicates the point of attachment to the rest of the compound;
wherein the viscosity of the pharmaceutical formulation following addition of the compound is ≤25 mPa-S.

17. A pharmaceutical composition according to any one of claims 3-15 for use in treating a disease or pathological condition comprising administering to a subject in need of such treatment a therapeutically effective amount of the pharmaceutical composition, optionally
wherein the pharmaceutical composition is administered by intravenous or
subcuntaneous administration.

18. Use of a compound of Formula I: 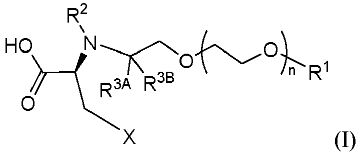
or a pharmaceutically acceptable salt thereof,
wherein:
X is 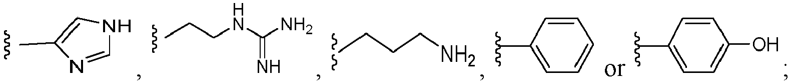
R¹ is H or methyl;
R² is H or 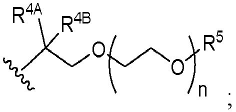
R^{3A} and R^{3B} are each H or together form oxo;
R^{4A} and R^{4B} are each H or together form oxo;
R⁵ is H or methyl; and
each occurrence of n is independently 1 to 5;
and wherein 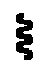 indicates the point of attachment to the rest of the compound,
for lowering the viscosity of a pharmaceutical composition in aqueous solution, wherein the pharmaceutical composition comprises an active biological ingredient (ABI) which is present at a concentration of about 50-250 mg/mL.

## Patentansprüche

1. Eine Verbindung der Formel I: 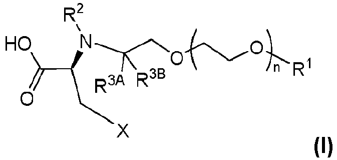
oder ein pharmazeutisch annehmbares Salz davon,
wobei:
X 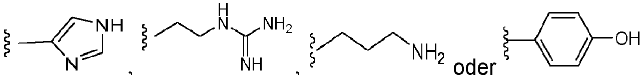 ist,
R¹ H oder Methyl ist,
R² 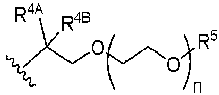 ist,
R^{3A} und R^{3B} jeweils H sind oder zusammen Oxo bilden,
R^{4A} und R^{4B} jeweils H sind oder zusammen Oxo bilden,
R⁵ H oder Methyl ist, und
jedes Vorkommen von n unabhängig 1 bis 5 ist,
und wobei 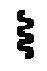 den Verknüpfungspunkt zum Rest der Verbindung bedeutet.

2. Eine Verbindung mit der Struktur: 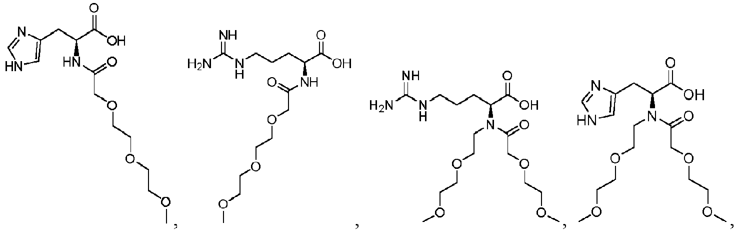 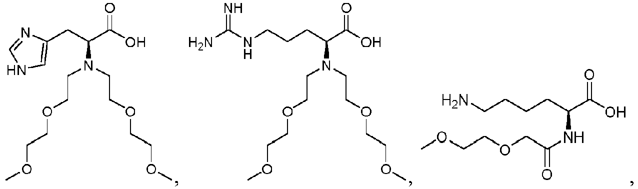 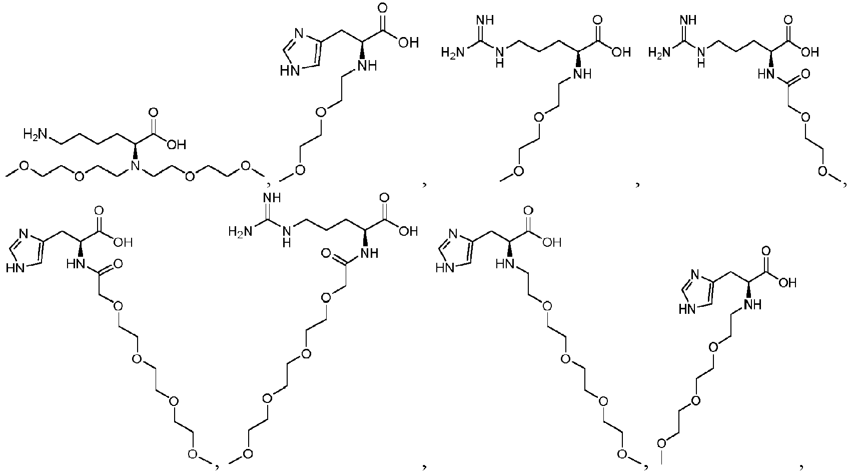 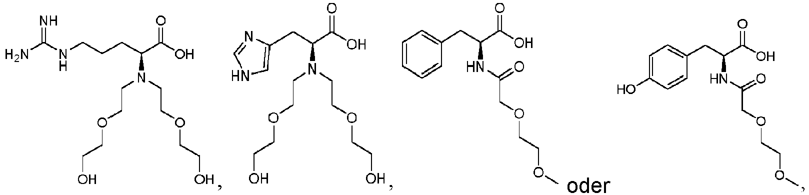 oder ein pharmazeutisch annehmbares Salz davon.

3. Eine pharmazeutische Zusammensetzung, die einen biologischen Wirkstoff (ABI) und eine Verbindung der Formel I: 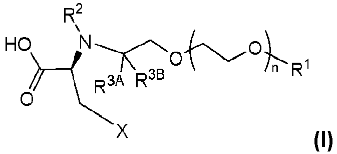
oder ein pharmazeutisch annehmbares Salz davon umfasst,
wobei:
X 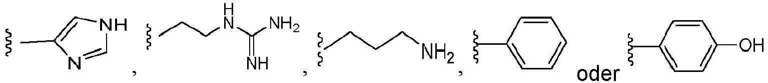 ist,
R¹ H oder Methyl ist,
R² H oder 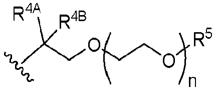 ist,
R^{3A} und R^{3B} jeweils H sind oder zusammen Oxo bilden,
R^{4A} und R^{4B} jeweils H sind oder zusammen Oxo bilden,
R⁵ H oder Methyl ist, und
jedes Vorkommen von n unabhängig 1 bis 5 ist,
wobei 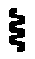 den Verknüpfungspunkt zum Rest der Verbindung bedeutet,
wobei der ABI ein Antikörper oder Antigen-bindendes Fragment davon oder ein therapeutisches Protein ist.

4. Die pharmazeutische Zusammensetzung nach Anspruch 3, wobei der ABI ein Antikörper oder Antigen-bindendes Fragment davon ist, der/das in einer Konzentration von etwa 50-250 mg/ml vorliegt.

5. Die pharmazeutische Zusammensetzung gemäß Anspruch 3 oder 4, ferner umfassend Histidin-Puffer bei etwa pH 5,0 bis etwa pH 6,0 in einer Konzentration von etwa 5 mM bis etwa 20 mM, gegebenenfalls
ferner umfassend etwa 0,01 % bis etwa 0,04 % Gew./Vol. nichtionisches Tensid, gegebenenfalls
wobei das nichtionische Tensid Polysorbat 80 ist, gegebenenfalls
wobei Polysorbat 80 in einem Gewichtsverhältnis von etwa 0,02 % Gew./Vol. vorliegt.

6. Die pharmazeutische Zusammensetzung gemäß einem der Ansprüche 3-5, ferner umfassend einen Stabilisator, gegebenenfalls
wobei der Stabilisator Saccharose ist, gegebenenfalls
wobei die Saccharose in einem Gewichtsverhältnis von etwa 6 % bis etwa 8 %
Gew./Vol. vorliegt.

7. Die pharmazeutische Zusammensetzung gemäß den Ansprüchen 3-6, wobei der ABI ein Anti-PD-1-Antikörper oder Antigen-bindendes Fragment davon ist, der/das spezifisch menschlichen Programmed-Death-Rezeptor 1 (PD-1) bindet, gegebenenfalls
wobei der Anti-PD-1-Antikörper oder das Antigen-bindende Fragment davon drei komplementaritätsbestimmende Regionen (CDRs) der leichten Kette, umfassend SEQ ID NO:1, SEQ ID NO:2 und SEQ ID NO:3, und drei CDRs der schweren Kette, umfassend SEQ ID NO:6, SEQ ID NO:7 und SEQ ID NO:8, umfasst, gegebenenfalls
wobei der anti-menschliche PD-1-Antikörper oder das Antigen-bindende Fragment davon eine V_{L}-Region, die die in SEQ ID NO:4 angegebene Aminosäuresequenz umfasst, und eine V_{H}-Region, die die in SEQ ID NO:9 angegebene Aminosäuresequenz umfasst, umfasst, gegebenenfalls
wobei der Anti-PD-1-Antikörper oder das Antigen-bindende Fragment davon eine leichte Kette, die eine wie in SEQ ID NO:5 angegebene Sequenz von Aminosäuren umfasst oder daraus besteht, und eine schwere Kette, die eine wie in SEQ ID NO:10 angegebene Sequenz von Aminosäuren umfasst oder daraus besteht, umfasst, gegebenenfalls
wobei der anti-menschliche PD-1-Antikörper oder das Antigen-bindende Fragment davon Pembrolizumab ist.

8. Die pharmazeutische Zusammensetzung nach einem der Ansprüche 4 oder 7, wobei der Antikörper oder das Antigen-bindende Fragment davon in einer Konzentration von 100-250 mg/ml vorliegt, und wobei die Zusammensetzung 10 mM Histidin, 7 % Saccharose und 0,02 % Polysorbat 80 umfasst.

9. Die pharmazeutische Zusammensetzung nach einem der Ansprüche 3-8, wobei die Zusammensetzung eine wässrige Lösung ist, gegebenenfalls ferner umfassend einen zweiten ABI.

10. Eine pharmazeutische Zusammensetzung, die 100 bis 200 mg/ml Pembrolizumab, 10 mM Histidin-Puffer und eine Verbindung der Formel I: 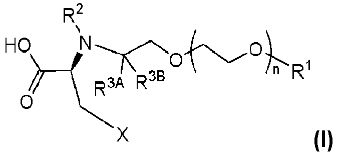
oder ein pharmazeutisch annehmbares Salz davon umfasst,
wobei
X 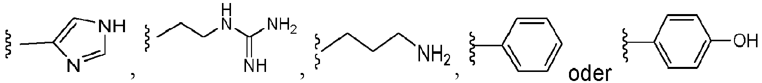 ist,
R¹ H oder Methyl ist,
R² H oder 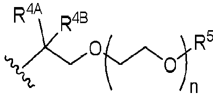 ist,
R^{3A} und R^{3B} jeweils H sind oder zusammen Oxo bilden,
R^{4A} und R^{4B} jeweils H sind oder zusammen Oxo bilden,
R⁵ H oder Methyl ist, und
jedes Vorkommen von n unabhängig 1 bis 5 ist,
und wobei 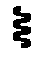 den Verknüpfungspunkt zum Rest der Verbindung bedeutet,
wobei die Zusammensetzung gegebenenfalls ferner Saccharose und Polysorbat 80 umfasst.

11. Die pharmazeutische Zusammensetzung nach einem der Ansprüche 3-10, wobei R² H ist.

12. Die Zusammensetzung nach Anspruch 1 oder die pharmazeutische Zusammensetzung nach einem der Ansprüche 3-10, wobei R² 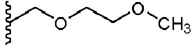 ist.

13. Die Verbindung nach Anspruch 1 oder die pharmazeutische Zusammensetzung nach einem der Ansprüche 3-12, wobei R¹ Methyl ist, gegebenenfalls
wobei R^{3A} und R^{3B} H sind, oder
wobei R^{3A} und R^{3B} verbunden sind unter Bildung von Oxo.

14. Die Verbindung nach Anspruch 1 oder die pharmazeutische Zusammensetzung nach einem der Ansprüche 3-13, wobei jedes Vorkommen von n unabhängig 1 bis 3 ist.

15. Die pharmazeutische Zusammensetzung nach einem der Ansprüche 3-10, wobei die Verbindung der Formel I die folgende Struktur besitzt: 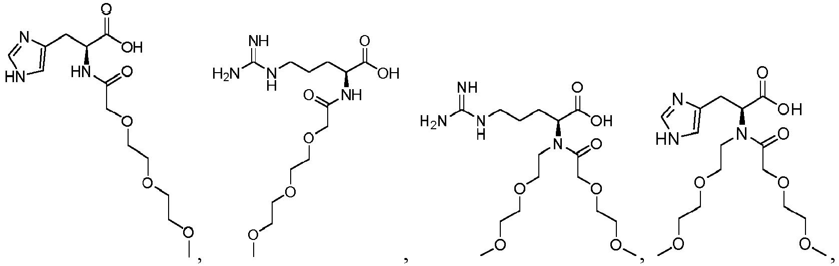 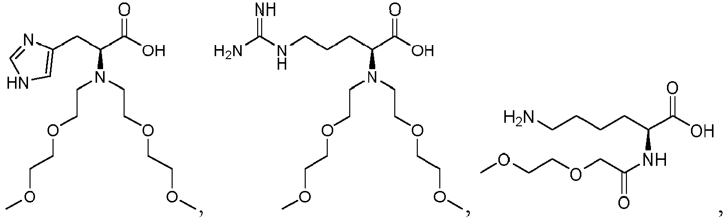 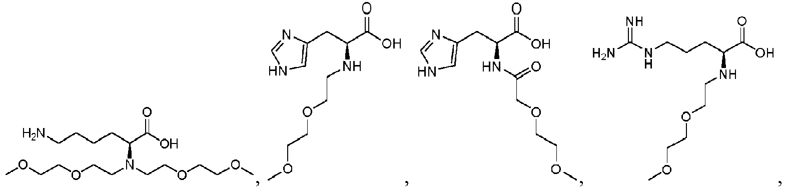 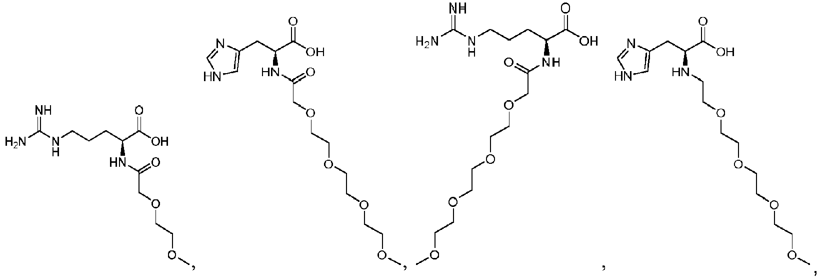 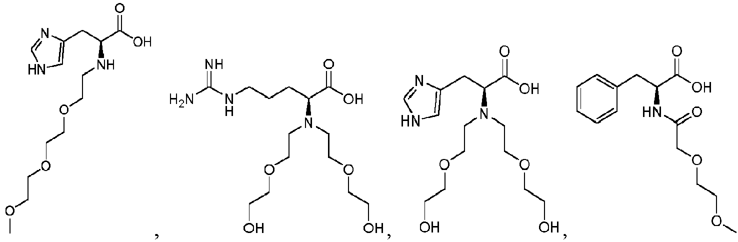 oder

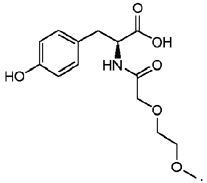

16. Ein Verfahren zur Verringerung der Viskosität einer pharmazeutischen Formulierung, umfassend:
(a) Bereitstellen einer pharmazeutischen Formulierung, die einen ABI in einer Konzentration von etwa 50 mg/ml bis etwa 250 mg/ml umfasst, wobei die Formulierung in wässriger Lösung ist, und
(b) Hinzufügen einer Verbindung der Formel I zur Lösung: 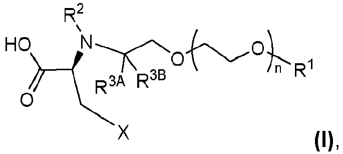
wobei:
X 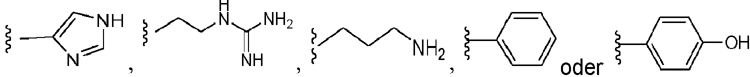 ist,
R¹ H oder Methyl ist,
R² H oder 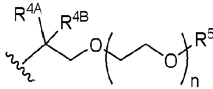 ist,
R^{3A} und R^{3B} jeweils H sind oder zusammen Oxo bilden,
R^{4A} und R^{4B} jeweils H sind oder zusammen Oxo bilden,
R⁵ H oder Methyl ist, und
jedes Vorkommen von n unabhängig 1 bis 5 ist, und wobei 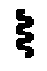 den Verknüpfungspunkt zum Rest der Verbindung bedeutet,
wobei die Viskosität der pharmazeutischen Formulierung nach der Zugabe der Verbindung ≤25 mPa-S ist.

17. Eine pharmazeutische Zusammensetzung gemäß einem der Ansprüche 3-15 zur Verwendung bei der Behandlung einer Erkrankung oder eines pathologischen Zustands, umfassend das Verabreichen einer therapeutisch wirksamen Menge der pharmazeutischen Zusammensetzung an ein Subjekt, das eine solche Behandlung benötigt, gegebenenfalls
wobei die pharmazeutische Zusammensetzung durch intravenöse oder subkutane Verabreichung verabreicht wird.

18. Verwendung einer Verbindung der Formel I: 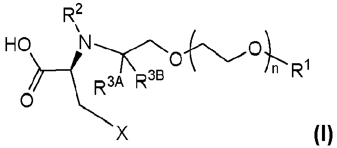
oder eines pharmazeutisch annehmbaren Salzes davon,
wobei:
X 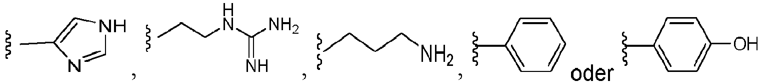 ist,
R¹ H oder Methyl ist,
R² H oder 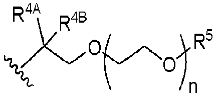 ist,
R^{3A} und R^{3B} jeweils H sind oder zusammen Oxo bilden,
R^{4A} und R^{4B} jeweils H sind oder zusammen Oxo bilden,
R⁵ H oder Methyl ist, und
jedes Vorkommen von n unabhängig 1 bis 5 ist,
und wobei 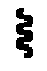 den Verknüpfungspunkt zum Rest der Verbindung bedeutet, zur Verringerung der Viskosität einer pharmazeutischen Zusammensetzung in wässriger Lösung, wobei die pharmazeutische Zusammensetzung einen biologischen Wirkstoff (ABI) umfasst, der in einer Konzentration von etwa 50-250 mg/ml vorliegt.

## Revendications

1. Composé de Formule 1 : 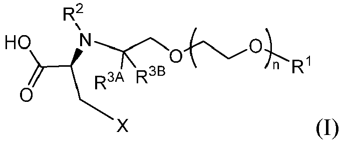
ou un sel pharmaceutiquement acceptable de celui-ci,
où :
X est 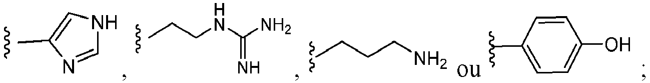
R¹ est H ou un méthyle ;
R² est 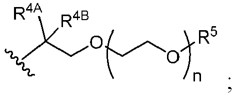
R^{3A} et R^{3B} sont chacun H ou forment ensemble un oxo ;
R^{4A} et R^{4B} sont chacun H ou forment ensemble un oxo ;
R⁵ est H ou un méthyle ; et
chaque occurrence de n a indépendamment une valeur de 1 à 5 ;
et dans lequel 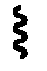 indique le point de fixation au reste du composé.

2. Composé présentant la structure : 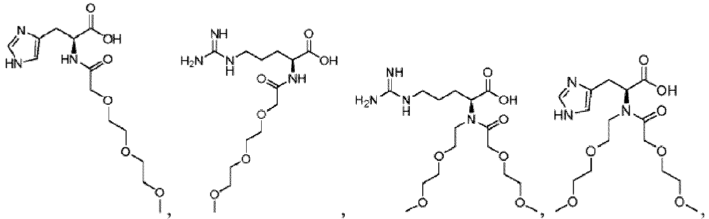 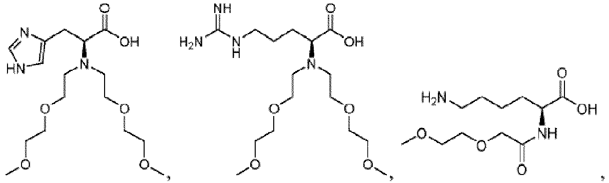 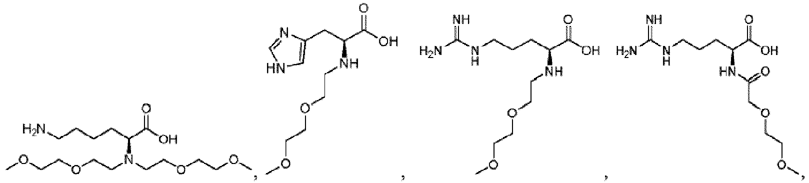 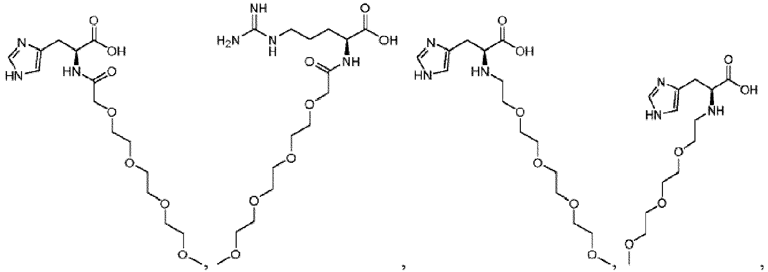 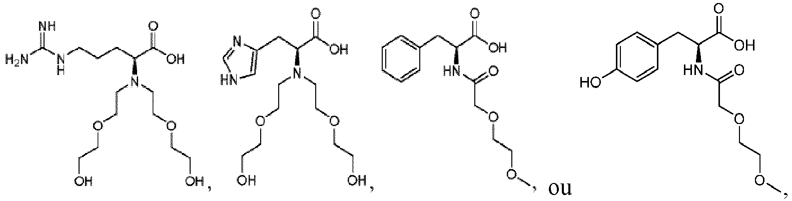 ou un sel pharmaceutiquement acceptable de ceux-ci.

3. Composition pharmaceutique comprenant un ingrédient biologique actif (ABI) et un composé de Formule 1 : 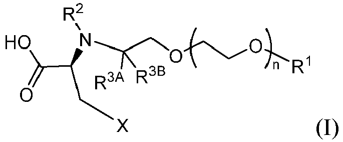
ou un sel pharmaceutiquement acceptable de celui-ci,
où :
X est 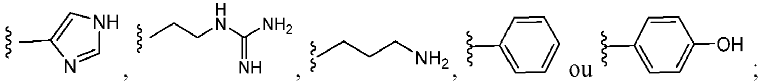
R¹ est H ou un méthyle ;
R² est H ou 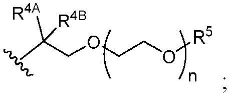
R^{3A} et R^{3B} sont chacun H ou forment ensemble un oxo ;
R^{4A} et R^{4B} sont chacun H ou forment ensemble un oxo ;
R⁵ est H ou un méthyle ; et
chaque occurrence de n a indépendamment une valeur de 1 à 5 ;
où 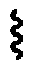 indique le point de fixation au reste du composé ;
le ABI étant un anticorps ou un fragment de liaison à un antigène de celui-ci ou une protéine thérapeutique.

4. Composition pharmaceutique selon la revendication 3, dans laquelle le ABI est un anticorps ou un fragment de liaison à un antigène de celui-ci, présent en une concentration d'environ 50-250 mg/ml.

5. Composition pharmaceutique selon la revendication 3 ou 4, comprenant en outre un tampon d'histidine à environ 5,0 de pH à environ 6,0 de pH en une concentration d'environ 5 mM à environ 20 mM, optionnellement
comprenant en outre d'environ 0,01 % à environ 0,04 % p/v d'un tensioactif non ionique, optionnellement
dans laquelle le tensioactif non ionique est le polysorbate 80, optionnellement
dans laquelle le polysorbate 80 est présent en un rapport en poids d'approximativement 0,02 % p/v.

6. Composition pharmaceutique selon l'une quelconque des revendications 3-5, comprenant en outre un stabilisant, optionnellement
dans laquelle le stabilisant est un saccharose, optionnellement
dans laquelle le saccharose est présent en un rapport en poids d'environ 6 % à environ 8 % p/v.

7. Composition pharmaceutique selon l'une quelconque des revendications 3-6, dans laquelle le ABI est un anticorps anti-PD-1 ou un fragment de liaison à un antigène de celui-ci qui se lie spécifiquement à un récepteur de mort programmée 1 (PD-1) humain, optionnellement
dans laquelle l'anticorps anti-PD-1, ou un fragment de liaison à un antigène de celui-ci, comprend trois régions déterminant la complémentarité (CDR) de chaîne légère comprenant la SEQ ID NO : 1, la SEQ ID NO :2 et la SEQ ID NO :3 et trois CDR de chaîne lourde comprenant la SEQ ID NO :6, la SEQ ID NO :7 et la SEQ ID NO :8, optionnellement
dans laquelle l'anticorps anti-PD-1 humain ou un fragment de liaison à un antigène de celui-ci comprend une région V_{L} qui comprend la séquence d'acides aminés présentée dans la SEQ ID NO :4 et une région V_{H} qui comprend la séquence d'acides aminés présentée dans la SEQ ID NO :9, optionnellement
dans laquelle l'anticorps anti-PD-1 ou un fragment de liaison à un antigène de celui-ci comprend une chaîne légère comprenant ou constituée d'une séquence d'acides aminés telle que présentée dans la SEQ ID NO :5 et une chaîne lourde comprenant ou constituée d'une séquence d'acides aminés telle que présentée dans la SEQ ID NO : 10, optionnellement
dans laquelle l'anticorps anti-PD-1 humain ou un fragment de liaison à un antigène de celui-ci est le pembrolizumab.

8. Composition pharmaceutique selon l'une quelconque des revendications 4 ou 7, dans laquelle l'anticorps ou un fragment de liaison à un antigène de celui-ci est présent en une concentration de 100-250 mg/ml et où la composition comprend 10 mM d'histidine, 7 % de saccharose et 0,02 % de polysorbate 80.

9. Composition pharmaceutique selon l'une quelconque des revendications 3-8, où la composition est une solution aqueuse, optionnellement
comprenant en outre un deuxième ABI.

10. Composition pharmaceutique comprenant de 100 à 200 mg/ml de pembrolizumab, 10 mM de tampon d'histidine et un composé de Formule 1 : 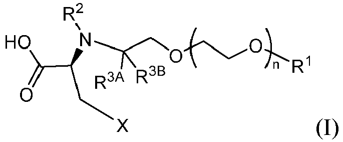
ou un sel pharmaceutiquement acceptable de celui-ci,
où :
X est 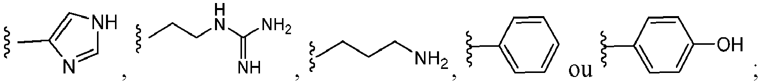
R¹ est H ou un méthyle ;
R² est H ou 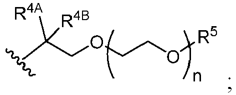
R^{3A} et R^{3B} sont chacun H ou forment ensemble un oxo ;
R^{4A} et R^{4B} sont chacun H ou forment ensemble un oxo ;
R⁵ est H ou un méthyle ; et
chaque occurrence de n a indépendamment une valeur de 1 à 5 ;
et dans lequel 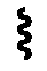 indique le point de fixation au reste du composé,
la composition comprenant en outre optionnellement un saccharose et du polysorbate 80.

11. Composition pharmaceutique selon l'une quelconque des revendications 3-10, dans laquelle R² est H.

12. Composé selon la revendication 1 ou composition pharmaceutique selon l'une quelconque des revendications 3-10, dans lesquels R² est

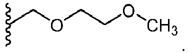

13. Composé selon la revendication 1 ou composition pharmaceutique selon l'une quelconque des revendications 3-12, dans lesquels R¹ est un méthyle, optionnellement
dans lesquels R^{3A} et R^{3B} sont H ou
dans lesquels R^{3A} et R^{3B} se joignent pour former un oxo.

14. Composé selon la revendication 1 ou composition pharmaceutique selon l'une quelconque des revendications 3-13, dans lesquels chaque occurrence de n a indépendamment une valeur de 1 à 3.

15. Composition pharmaceutique selon l'une quelconque des revendications 3-10, dans laquelle le composé de Formule 1 présente la structure suivante : 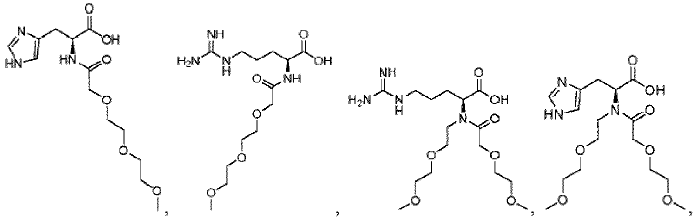 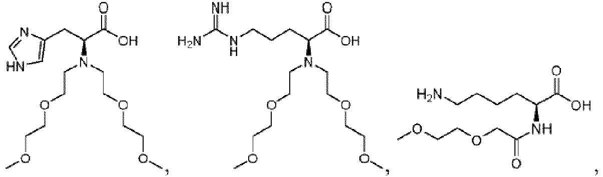 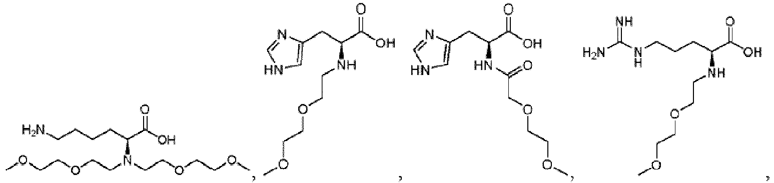 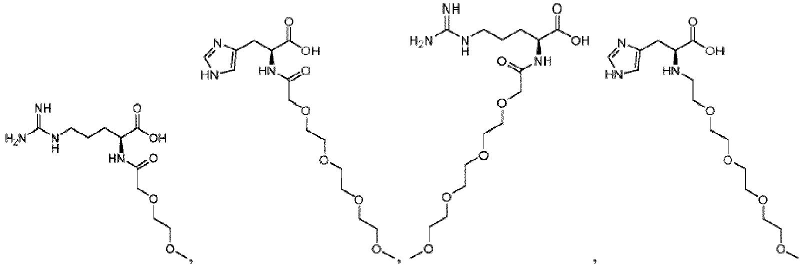 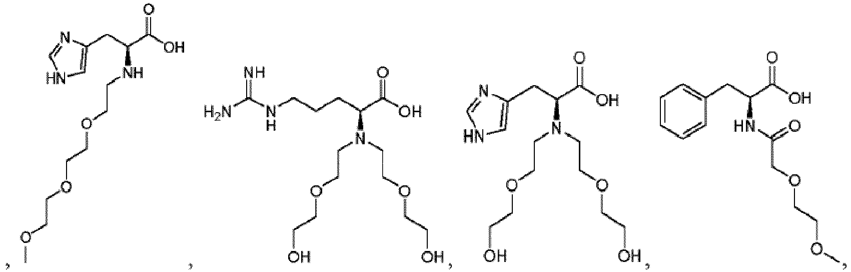 ou

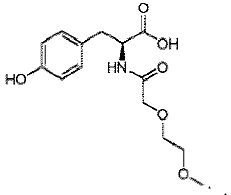

16. Procédé permettant d'abaisser la viscosité d'une formulation pharmaceutique comprenant :
(a) la fourniture d'une formulation pharmaceutique comprenant un ABI en une concentration d'environ 50 mg/ml à environ 250 mg/ml, dans lequel la formulation est dans une solution aqueuse ; et
(b) l'ajout d'un composé de Formule 1 à la solution : 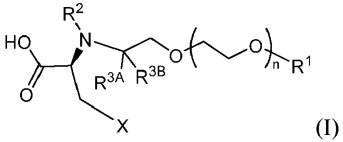
dans lequel :
X est 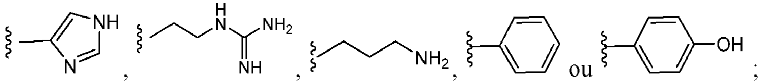
R¹ est H ou un méthyle ;
R² est H ou 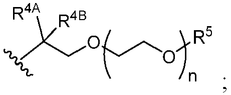
R^{3A} et R^{3B} sont chacun H ou forment ensemble un oxo ;
R^{4A} et R^{4B} sont chacun H ou forment ensemble un oxo ;
R⁵ est H ou un méthyle ; et
chaque occurrence de n a indépendamment une valeur de 1 à 5 ;
et dans lequel 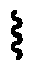 indique le point de fixation au reste du composé ;
dans lequel la viscosité de la formulation pharmaceutique suivant l'ajout du composé est < 25 mPa-S.

17. Composition pharmaceutique selon l'une quelconque des revendications 3-15 pour utilisation dans le traitement d'une maladie ou d'un état pathologique comprenant l'administration, à un sujet ayant besoin d'un tel traitement, d'une quantité thérapeutiquement efficace de la composition pharmaceutique, optionnellement
où la composition pharmaceutique est administrée par une administration intraveineuse ou sous-cutanée.

18. Utilisation d'un composé de Formule 1 : 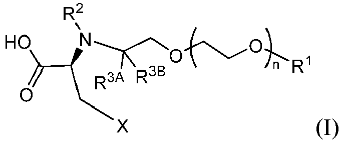
ou d'un sel pharmaceutiquement acceptable de celui-ci,
dans lequel : 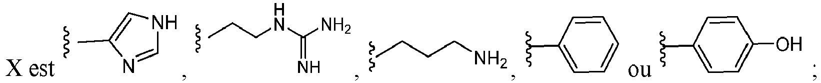
R¹ est H ou un méthyle ;
R² est H ou 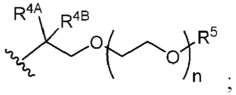
R^{3A} et R^{3B} sont chacun H ou forment ensemble un oxo ;
R^{4A} et R^{4B} sont chacun H ou forment ensemble un oxo ;
R⁵ est H ou un méthyle ; et
chaque occurrence de n est indépendamment 1 à 5 ;
et dans lequel 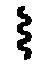 indique le point de fixation au reste du composé,
permettant d'abaisser la viscosité d'une composition pharmaceutique dans une solution aqueuse, dans laquelle la composition pharmaceutique comprend un ingrédient biologique actif (ABI) qui est présent en une concentration d'environ 50-250 mg/ml.
